(19) **Europäisches Patentamt**

**European Patent Office**

**Office européen des brevets**

(11) **EP 1 676 914 A1**

(12) **EUROPEAN PATENT APPLICATION**
published in accordance with Art. 158(3) EPC

(43) Date of publication:
**05.07.2006 Bulletin 2006/27**

(51) Int Cl.:
*C12N 15/11* (1990.01)    *C07K 16/18* (1995.01)
*C12Q 1/68* (1980.01)

(21) Application number: **04773479.3**

(22) Date of filing: **22.09.2004**

(86) International application number:
**PCT/JP2004/014305**

(87) International publication number:
**WO 2005/028648 (31.03.2005 Gazette 2005/13)**

(84) Designated Contracting States:
**DE FR GB**

(30) Priority: **22.09.2003  US 504208 P**
**30.03.2004  US 557390 P**

(71) Applicants:
• **Aichi Prefecture**
  **Nagoya-shi,**
  **Aichi 460-0001 (JP)**
• **NGK INSULATORS, LTD.**
  **Nagoya-City, Aichi Pref.  467-8530 (JP)**

(72) Inventors:
• **SETO, Masao**
  **Nagoya-shi, Aichi 465-0034 (JP)**

• **TAGAWA, Hiroyuki**
  **Chikusa-ku,**
  **Nagoya-shi,**
  **Aichi 464-0006 (JP)**
• **YOSHIDA, Yasuko**
  **Nagoya-shi,**
  **Aichi 467-8530 (JP)**
• **KIRA, Shigeki**
  **Nagoya-shi,**
  **Aichi 467-8530 (JP)**

(74) Representative: **Bufton, Karen A.J. et al**
  **Mewburn Ellis LLP**
  **York House**
  **23 Kingsway**
  **GB-London WC2B 6HP (GB)**

(54) **DISEASE TYPE OF LYMPHOMA AND METHOD OF ASSESSING PROGNOSIS**

(57)    A method for determining the prognosis of a CD5+DLBCL patient and a CD5-DLBCL patient is provided. It is determined that, in the chromosomal DNA from a patient with lymphoma, (1) the prognosis of the CD5+DLBCL patient with amplification of 13q21.1-q31.3 region is poor; (2) the prognosis of the CD5+DLBCL patient with deletion of 1p36.21-p36.13 region is poor; and (3) the prognosis of the CD5-DLBCL patient with amplification of 5p15.33-p14.2 region is good.

EP 1 676 914 A1

**EP 1 676 914 A1**

**Description**

**Technical Field**

The invention of this application relates to types of lymphoma and a method for prognosis thereof. More particularly, the invention of this application relates to a method for molecular biologically diagnosing the prognosis condition of malignant lymphoma and a material used for this method.

**Background Art**

Diffuse large B-cell lymphoma (DLBCL) comprises some 30% of non-Hodgkin lymphoma cases and is clinically heterogeneous (Harris, N.L. et al. Blood, 84: 1361-1392, 1994 ; Gatter, K. C. and Warnke, R. A. Pathology & Genetics of tumours of haematopoietic and lymphoid tissues, pp171-174. Washington: IARC press, Lyon, 2001). Recently, microarray analyses of transcripts of DLBCL sample have clearly shown biologically distinct subtypes in DLBCL that are also clinically relevant (Alizadeh, A. A. et al. Nature (Lond.), 403: 503-511, 2000;Shipp, M. A. et al. Nat. Med., 8: 68-74, 2002). Several genetic alterations have been identified as etiologically associated with DLBCL, but genome-wide screening has been lacking (Offit, K. et al. N. Engl. J. Med., 331: 74-80, 1994;Kramer, M. H. H. et al. Blood, 92: 3152-3162, 1998). The recently developed array CGH techniques allow high throughput analysis of copy number changes of genome at high resolution throughout the whole genome. The quantitative measurement of DNA copy number thus obtained may facilitate identifications of tumor-related genes (Weiss, M. M. et al. J. Pathol., 200: 320-326, 2003), and could also be used to classify tumors (O'Hagan, R. C. et al. Cancer Res., 63: 5352-5356, 2003).

CD5 is a cell surface molecule physiologically expressed on T cells, and subsets of B cells residing in mantle zone of lymphoid organs and peritoneal cavity. Clinically, CD5 expression is often associated with chronic lymphocytic leukemia (Muller-Hermelink, H. K. et al. Pathology & Genetics of tumours of haematopoietic and lymphoid tissues, pp 127-130. Washington: IARC press, Lyon, 2001) and mantle cell lymphoma (Swerdlow, S. H. et al. Pathology & Genetics of tumours of haematopoietic and lymphoid tissues, pp 168-170. Washington: IARC press, Lyon, 2001). Among DLBCL cases, the inventors have identified CD5 expression as a marker of poor prognosis; CD5-positive (CD5+) DLBCL is also associated with elderly onset, female predominance, frequently involvement of extra-nodal sites (Yamaguchi, M. et al. Blood, 99: 815-821, 2002). Microarray analyses of transcripts differently expressed between CD5+ and CD5-negative (CD5-) DLBCL cases are also indicative of distinct disease entity of CD5+ DLBCL (Kobayashi, T. et al. Cancer Res., 63: 60-66, 2003; Gascoyne, R. D. et al. Blood, 102: 178a-179a, 2003).

Chromosomal amplification is a common mechanism by which genes achieve over-expression in tumors. Identification and characterization of oncogenes present in amplified regions can thus provide important insights into the pathogenesis of cancer (Schwab, M. Cancer. Biol., 9: 319-325, 19991). Activated oncogenes, such as *MYCN* in neuroblastomas or *HER2* in breast cancer, also have prognostic relevance (Schwab, M. Cancer. Biol., 4: 13-18, 1993; Brodeur, G. M. et al. Science (Wash. DC), 224: 1121-1124, 1984 ; Slamon, D. J. et al. Science, 235: 177-182, 1987) .

The high-level amplification seen at 13q21-qter has been observed in hematologic and other solid neoplasms. Amplification at 13q21-qter has been reported in diffuse large B cell lymphoma (DLBCL) (Rao, P. H. et al. Blood, 92: 234-240, 1998), in mantle cell lymphoma (MCL) (Monni, O. et al. Genes Chromosomes Cancer, 21: 298-307, 1998), follicular lymphoma (Neat, M. J. et al. Genes Chromosomes Cancer, 32: 236-243, 2001), primary cutaneous B-cell lymphoma (Mao, X. et al. Genes Chromosomes Cancer, 35: 144-155, 2002), and nasal-type NK/T-cell lymphoma (Ko, Y. H. et al. Cytometry, 46: 85-91, 2001). Further cases of amplification at 13q21-qter have also been reported in solid tumors: glioma (Knuutila, S. et al. Am J Pathol., 152: 1107-1123, 1998), non-small cell lung cancer (Knuutila, S. et al. Am J Pathol., 152: 1107-1123, 1998), bladder cancer (Knuutila, S. et al. Am J Pathol., 152: 1107-1123, 1998), squamous-cell carcinoma of the head and neck (Knuutila, S. et al. Am J Pathol., 152: 1107-1123, 1998), peripheral nerve sheath tumor (Schmidt, H. et al. Genes Chromosomes Cancer, 25: 205-211, 1999), malignant fibrous histiocytoma (Lallamendy, M. L. et al. Am J Pathol., 151: 1153-61, 1997), and alveolar rhabdomyosarcoma (Gordon, A. T. et al. Genes Chromosomes Cancer, 28: 220-226, 2000).

**Disclosure of Invention**

The inventors of this application established their own DNA array-based CGH in which 2,088 types of BAC/PAC clone (bacterial artificial chromosome / P1-derived artificial chromosome) were spotted in duplicate, and applied it to 26 cases of CD5+ and 44 cases of CD5-DLBCL. As a result, they identified a genomic aberration possessed by both groups and an aberration specific to CD5+DLBCL, and found out that such a genomic aberration is useful in the prognosis of lymphoma.

Further, the inventors found out that amplification of 13q including 13q31-q32 was observed in the 18 cases out of 70 cases of the foregoing DLBCL patients, and identified a novel gene in this amplified region. In addition, they found out that the expression level of this novel gene is effective for the prognosis of lymphoma.

The invention of this application is based on the foregoing novel findings. That is, this application provides the following inventions.

A first invention is a method for determining the prognosis of a patient with CD5-positive diffuse large B-cell lymphoma (CD5+DLBCL) and a patient with CD5-negative diffuse large B-cell lymphoma (CD5-DLBCL), which comprises isolating a chromosomal DNA from the respective patients with lymphoma, and determining that, in the chromosomal DNA,

(1) the prognosis of the CD5+DLBCL patient with amplification of chromosome 13 q21.1-q31.3 (13q21.1-q31.3) region is poor;
(2) the prognosis of the CD5+DLBCL patient with deletion of chromosome 1 p36.21-p36.13 (1p36.21-p36.13) region is poor; and
(3) the prognosis of the CD5-DLBCL patient with amplification of chromosome 5 p 15.33-p 14.2 (5p 15.33-p 14.2) region is good.

One aspect of the first invention is a method in which the amplification or the deletion of the chromosomal region is measured by hybridization of a plurality of DNA probes containing the chromosomal region with a chromosomal DNA from the patient.

Another aspect of the first invention is a method in which the DNA probes are BAC/PAC DNA clones.

A preferred embodiment in the foregoing aspect of the first invention is a method for carrying out the hybridization on a solid phase carrier.

A second invention is a DNA array used for the foregoing method for carrying out the hybridization on a solid phase carrier, in which the plurality of DNA probes containing the chromosomal region are immobilized on the solid phase carrier.

One aspect of the second invention is a DNA array in which the DNA probes are BAC/PAC DNA clones.

A third invention is a method for determining the prognosis of a CD5+DLBCL patient and a CD5-DLBCL patient, which comprises isolating a biological sample from the patient with lymphoma, and determining that, in the biological sample,

(1) the prognosis of the CD5+DLBCL patient with an increased gene expression in 13q21.1-q31.3 region is poor;
(2) the prognosis of the CD5+DLBCL patient with a decreased gene expression in 1p36.21-p36.13 region is poor; and
(3) the prognosis of the CD5-DLBCL patient with an increased gene expression in 5p15.33-p14.2 region is good.

One aspect of the third invention is a method in which the gene in 13q21.1-q31.3 region is C13orf25 gene having the following characteristics of:

(a) encoding a protein containing the amino acid sequences of SEQ ID NO: 4 and SEQ ID NO: 5; and/or
(b) transcribing the following precursor micro RNAs:

miR91-precursor-13 micro RNA, miR18-precursor-1, 3 micro RNA,
miR19a-precursor-13 micro RNA, miR19b-precursor-13 micro RNA
and miR92-precursor-13 micro RNA, and

the following mature micro RNAs:

miR-17, miR-91, miR-18, miR-19a, miR-20, miR-19b and miR-92.

Another aspect of the third invention is a method in which the increased gene expression or the decreased gene expression is determined by measuring a gene transcript.

A preferred embodiment in the foregoing aspect of the third invention is a method in which the gene transcript is an mRNA.

In the third invention, a more preferred embodiment in the foregoing embodiment, in which the gene transcript is an mRNA, is a method in which the increased gene expression or the decreased gene expression is measured by hybridization of a DNA probe which is a full length or a part of the gene with a gene mRNA or cDNA.

In the third invention, a more preferred embodiment in the embodiment for carrying out the hybridization of the probe with an mRNA or a cDNA, is a method for carrying out the hybridization on a solid phase carrier.

A fourth invention is a DNA array used for the method for carrying out the hybridization on a solid phase carrier, in which the DNA probes are immobilized on the solid phase carrier.

Still another aspect in the method of the third invention is a method in which the gene transcript is a protein.

One embodiment in the foregoing method is that the increase or decrease of the gene transcript is determined by using an antibody which specifically binds to the protein.

A fifth invention is an antibody used for the foregoing method which uses an antibody specifically binding to the foregoing protein.

One aspect of the fifth invention is an antibody which recognizes the amino acid sequences of SEQ ID NO: 4 and SEQ ID NO: 5.

A sixth invention is a purified polynucleotide of C13 or f25 gene, which has the following characteristics of:

(a) encoding a protein containing the amino acid sequences of SEQ ID NO: 4 and SEQ ID NO: 5; and/or
(b) transcribing the following precursor micro RNAs:

miR91-precursor-13 micro RNA, miR18-precursor-1, 3 micro RNA,
miR19a-precursor-13 micro RNA, miR19b-precursor-13 micro RNA
and miR92-precursor-13 micro RNA, and

the following mature micro RNAs:

miR-17, miR-91, miR-18, miR-19a, miR-20, miR-19b and miR-92.

A seventh invention is an oligonucleotide probe, which comprises a partial continuous sequence of the polynucleotide of the sixth invention and is hybridized to C13orf25 gene under a stringent condition.

An eighth invention is a DNA array, which comprises the oligonucleotide probe of the seventh invention.

A ninth invention is an oligonucleotide primer for PCR amplification of C13orf25 gene.

Incidentally, the term "good prognosis" of a DLBCL patient in this invention indicates the status of a group of patients whose survival rate is favorable as determined by the p-value of the log rank test showing a significant difference of 0.05 or less when comparing the Kaplan-Meier curves between 2 DLBCL patient groups having differences (including any of amplification, deletion and normal) in a certain genomic region. The term "poor prognosis" indicates the status of a group of patients whose survival rate is unfavorable as determined by the p-value of the log rank test showing a significant difference of 0.05 or less when comparing the Kaplan-Meier curves.

The terms and concepts in this invention will be defined in detail in the description of the embodiments or Examples of the invention. The terms are basically in accordance with IUPAC-IUB Commission on Biochemical Nomenclature or the meanings of terms used commonly in the art. In addition, various techniques used for implementing the invention can be easily and surely carried out by those skilled in the art based on a known literature or the like except for the techniques whose sources are particularly specified. For example, techniques of genetic engineering and molecular biology can be carried out according to the methods described in J. Sambrook, E. F. Fritsch & T. Maniatis, "Molecular Cloning: A Laboratory Manual (2nd edition)", Cold Spring Harbor Laboratory Press, Cold. Spring Harbor, New York (1989); D. M. Glover et al. (ed.), "DNA Cloning", 2nd ed., Vol. 1 to 4, (The Practical Approach Series), IRL Press, Oxford University Press (1995); Ausubel, F. M. et al., Current Protocols in Molecular Biology, John Wiley & Sons, New York, N.Y, 1995; Japanese Biochemical Society (ed.), "Zoku Seikagaku Jikken Koza 1, Idenshi Kenkyuho II" Tokyo Kagaku Dozin (1986); Japanese Biochemical Society (ed.), " Shin Seikagaku Jikken Koza 2, Kakusan III (Kumikae DNA Gijutsu)" Tokyo Kagaku Dozin (1992); R. Wu (ed.), "Methods in Enzymology", Vol. 68 (Recombinant DNA), Academic Press, New York (1980); R. Wu et al. (ed.), "Methods in Enzymology", Vol. 100 (Recombinant DNA, Part B) & 101 (Recombinant DNA, Part C), Academic Press, New York (1983); R. Wu et al. (ed.), "Methods in Enzymology", Vol. 153 (Recombinant DNA, Part D), 154 (Recombinant DNA, Part E) & 155 (Recombinant DNA, Part F), Academic Press, New York (1987), etc. or the methods described in the references cited therein or substantially the same methods or the modifications thereof.

**Brief Description of Drawings**

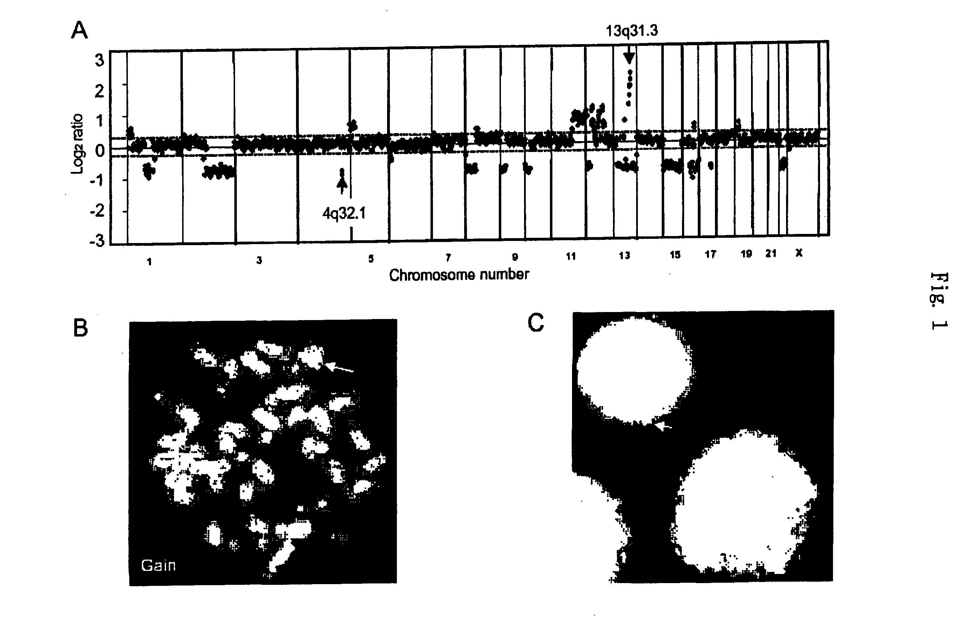

Fig. 1 is whole genome profile of REC1 cell line and FISH analyses for genomic amplification and loss. A, whole genomic profile of REC1 cell line. The log2 ratio for each of the 1,966 BAC/PAC clones was plotted as a function of its genome location. Horizontal dotted lines show threshold for gain and loss. B, FISH analysis of the cell line with BAC, RP11-430K10 (red signals) at 13q32. Arrow indicates 13q32 amplification. C, dual-color FISH analysis for detecting loss of 4q32.1. Each interphase chromosome has two pairs of red signal (BAC, RP11-154F14), whereas each of them has one pair of green signals (BAC, RP11- 312A15) beside the arrow. Physical Distance between these two BACs is 0.75Mb. A metaphase and chromosomes was counterstained by DAPI.

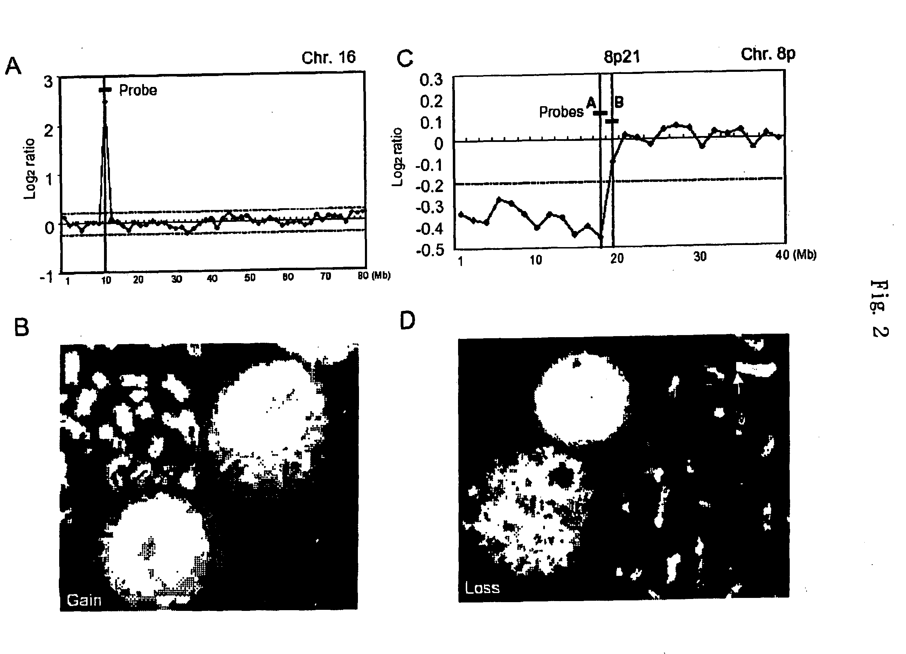

Fig.2. is individual genomic profiles of chromosome 16 and chromosome 8p in Karpas 1718 cell line (SLVL) and FISH analyses for genomic gain (16p13.3) and loss (8p21). *A*, individual genomic profile of chromosome 16, indicating high amplification (log2 ratio, +2.45) at BAC, RP11-27M24 locus (16p13.3). B, metaphase and interphase FISH analysis with BAC, RP11-27M24 shows genomic amplification at 16p13.13 (arrow). C, individual genomic profile of chromosome 8p, indicating loss at 8pte1-p21. *D*, dual-color FISH analysis with BAC, RP11-353K12 and BAC, RP11-369E15, indicating the breakpoint of 8p is between these BACs. BAC, RP11-369E15 is 500kb centromeric to BAC, RP11-353K12. Each interphase and metaphase chromosome has two pairs of red signals (BAC, RP11-369E15), whereas each of them has one pair of green signals (BAC, RP11- 353K12) beside the arrow.

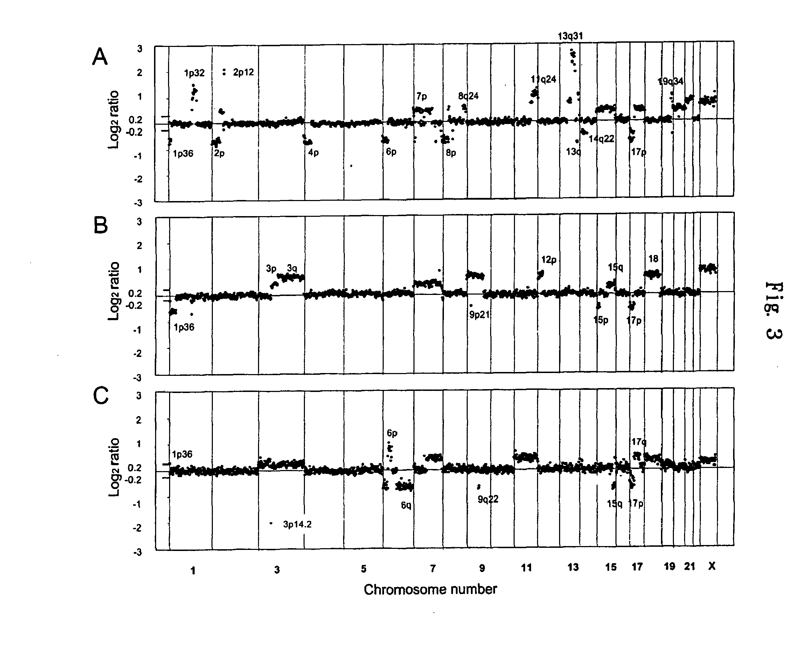

Fig. 3 is representative array CGH profiles for individual tumors. Whole genomic profiles are shown for three representative cases of DLBCL (A, CD5+ case 1, B, CD5+ case 2, and *C*, CD5- case 3). Log2 ratios were plotted for all clones based on chromosome position, with vertical dotted bar representing the separation of chromosomes. The BACs are ordered by position in the genome beginning at the 1p telomere and ending at the Xq telomere. A, copy number gains: 1q21.2-q24.3, 2p22.1-p16.1, 7pte1-p21.3, 7p21.1-p11.2, 7q21.11-q31.1, 8p11.23, 8q24.13-qtel, 11q22.3-qtel, 13q21.32-13q32.2, 13q34-qtel, 15, 17q, 19q13.43-qtel, 20, and 21. Copy number losses: 1p36.32-p36.21, 2ptel-p22.3, 4pte1-p15.1, 6p25.3-p22.3, 7p21.3, 7q31.33, 8ptel-p12, 8q12.1, 13q34-qtel, 14q22.2-q21.3, and 17p. B, copy number gains: 3p14.2-qtel, 7, 9ptel-q32.2, 12ptel-p11.2, 15q24.3-qtel, and 18. Copy number losses: 1ptel-p35.1, 9p21, 15q14-q21.1, and 17p. C, copy number gains: 1p36, 3, 6p, 7q21.11-qtel, 11, 16p13.3, 17cen-q23.2, 18. Copy number losses: 3p14.2, 6p25.1-p22.3, 6q14.1-qtel, 9q22.33, 15q26.2-qtel, and 17p. Log2 ratio, -2.01 of single BAC, RP11-48E21 suggests homozygous loss at 3p14.2 locus.

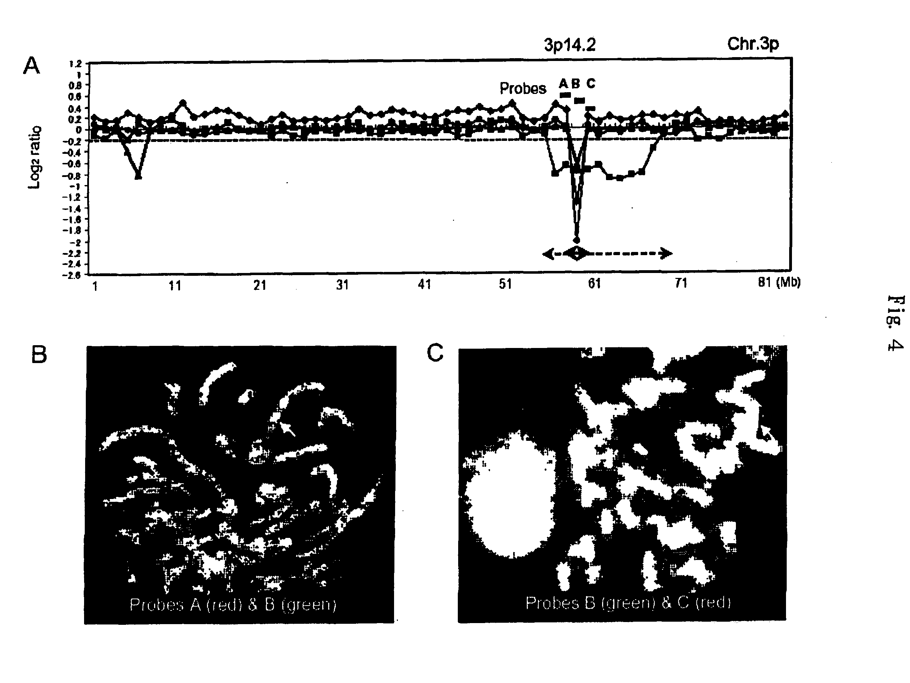

Fig. 4 is genomic profile of chromosome 3p and FISH analysis for minimum common loss. A, four representative individual genomic profiles of 3p are shown. Minimum common loss is shown at 3p14.2 with thick arrow (BAC, RP11-48E21 locus). Black three lines are individual profiles of each DLBCL case. Red line is individual profile of malignant lymphoma cell line, OCI-LY13.2. Physical distances of telomere are indicated under square line (Mb). Positions of probes used in FISH analyses in B and C are also presented as small bold horizontal lines. Probe A: BAC, RP11-391P4. Probe B: BAC, RP11-48E21. Probe C: BAC, RP11-611H10. Probe A is 1 Mb telomeric to probe B. Probe C is 1 Mb centromeric to probe B. Thin arrow indicates loss region of a case. BAC, RP11-48E21 contains FHIT tumor suppressor gene. B, dual-color FISH analysis with probes A and B for OCI-LY13.2 cell line. A metaphase chromosome has two pairs of red (probe A, red) signals, and one pair of green (probe B, green) signal, indicating heterozygous loss of probe B. C, dual-color FISH analysis with probes B and C for OCI-LY 13.2 cell line. A metaphase chromosome has two pairs of red (probe C, red) signals, and one pair of green signals (probe B, green), indicating heterozygous loss of probe B (green).

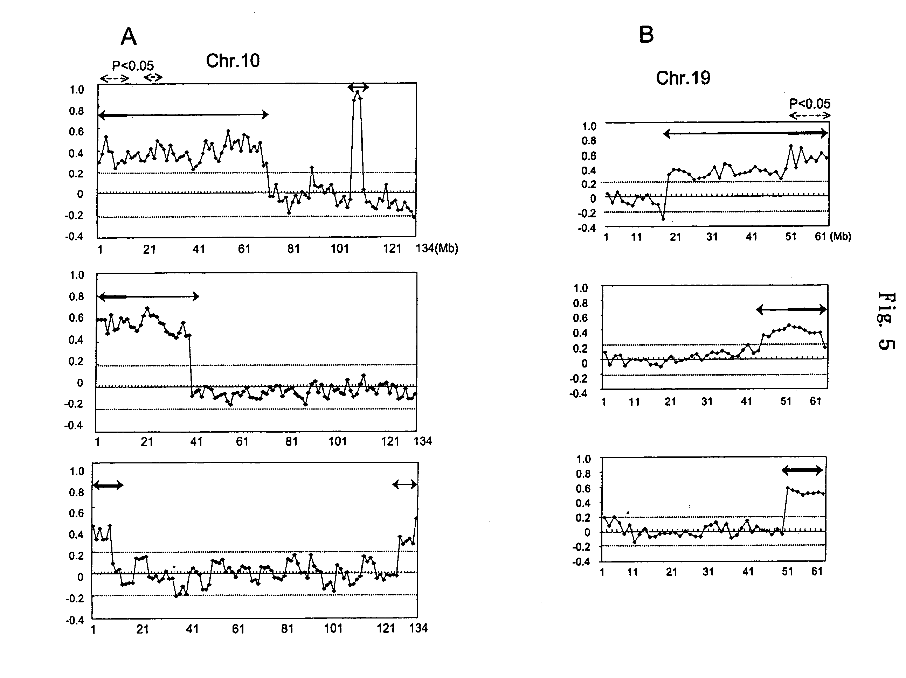

Fig. 5 is individual genomic profiles of characteristic gains of CD5+ DLBCL. Array CGH profiles of chromosome 10 and chromosome 19 with three representative cases are shown. Vertical lines indicate the log2 ratio. Horizontal lines indicate physical distance from p telomere to q telomere (Mb). A log2 ratio of over +0.2 represents genomic copy number gain, and a log2 ratio under -0.2 represents genomic copy number loss. Horizontal dotted lines show the threshold for gains and losses. Bold arrows indicate common gained regions among these three cases. Thin arrows indicate regions of gain in each case. Horizontal dotted arrow indicates significantly different gained regions in frequency between CD5+ and CD5-DLBCL. A, three representative individual genomic profiles of chromosome 10. B, three representative individual genomic profiles of chromosome 19.

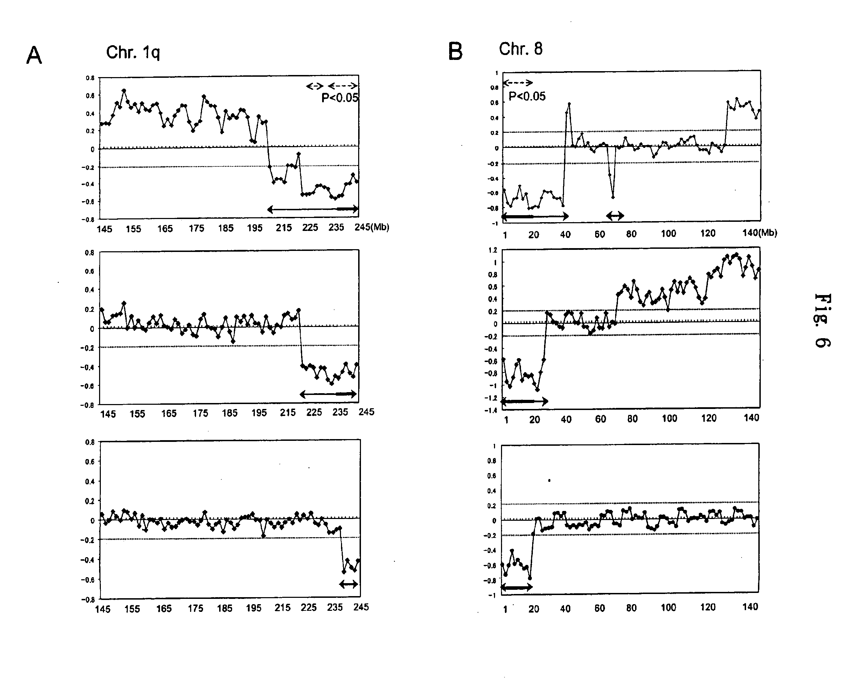

Fig. 6 is individual genomic profiles of characteristic losses of CD5+ DLBCL and minimal common region. Array CGH profiles of each chromosome with three representative cases are presented. Horizontal dotted lines show the threshold for gains and loss. Bold arrows indicate common lost regions among these three cases. Thin arrows indicate regions of loss of each case. Horizontal dotted arrow indicates significantly different gained regions in frequency between CD5+ and CD5-DLBCL. A, three representative individual genomic profiles of chromosome 1q. B, three representative individual genomic profiles of chromosome 8.

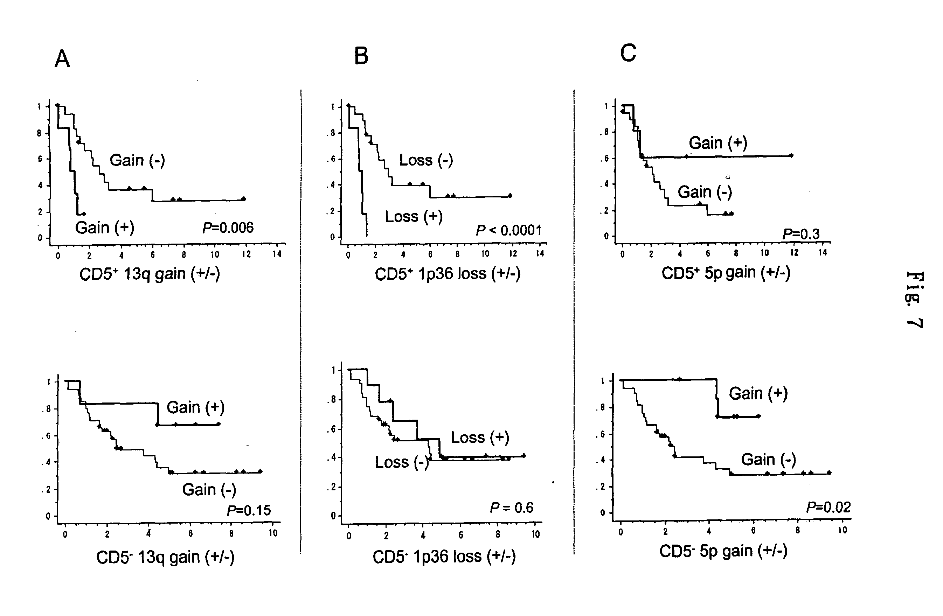

Fig. 7 is Kaplan-Meier survival curves for CD5+ and CD5- DLBCL cases in the presence or absence of 13q gain, 1p36 loss, and 5p gain. Overall survivals are shown for CD5+ and CD5- group. Horizontal lines: overall survival. Vertical lines: probability. A, survival curves for CD5+ cases according to the presence or absence of gain of 13q21.1-q31.3 (6 cases versus 19 cases) and survival curves for CD5- cases according to the presence or absence of gain of 13q21.1-q31.3 (6 cases versus 35 cases). B, survival curves for CD5+ cases according to the presence or absence of loss at 1p36.21-p36.13 (6 cases versus 19 cases) and survival curves for CD5-cases according to the presence or absence of loss at 1p36.21-p36.13 (9 cases versus 32 cases). C, survival curves for CD5+ cases according to the presence or absence of loss at 5p15.33-p14.2 (4 cases versus 21 cases) and survival curves for CD5- cases according to the presence or absence of loss at 5p15.33-p14.2 (7 cases versus 34 cases).

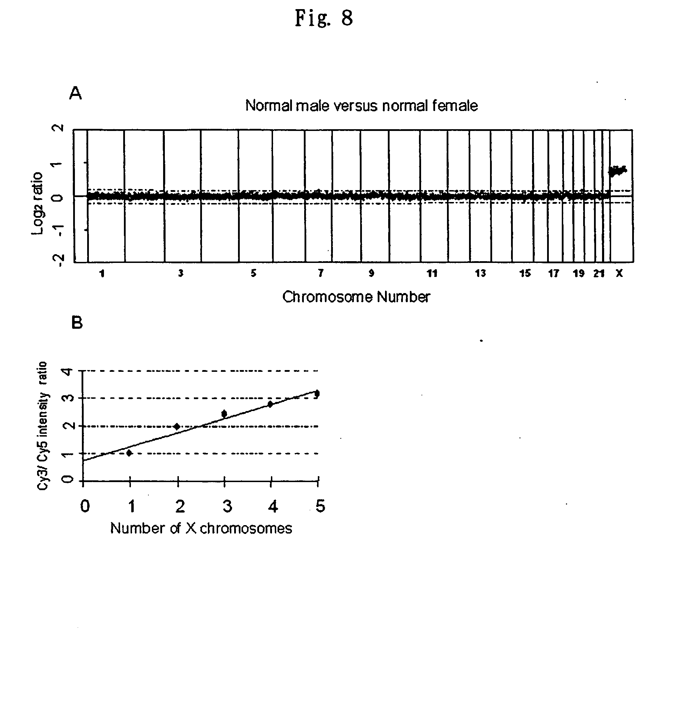

Fig. 8 is array CGH analysis of normal versus female. A, representative genomic profile of an array CGH using normal male versus normal female DNAs. Six simultaneous hybridizations of normal male versus normal male were performed to define the normal variation in $\log_2$ ratio ($\log_2$ cy3/cy5). In the control experiment, more than 95 % of the measured fluorescence $\log_2$ ratio values of each spot (2 x 1,966 clones) ranged from +0.2 to -0.2 (data not shown). The thresholds for the $\log_2$ratio of gains and loss were therefore set at $\log_2$ ratio of +0.2 and -0.2, respectively. Array data are plotted as the mean $\log_2$ratio of duplicate spots for each clone. Vertical lines show the threshold for the $\log_2$ratio of gains and loss. The $\log_2$ ratio for each of the BAC clone is plotted as a function of its genome location, with chromosome 1 to the left and X to the right; for each chromosome the order is short-arm telomeric to long-arm telomeric. B, normalized $\log_2$ratio for the changes in copy number of X chromosome. The normal male DNA was used as reference for all hybridizations. Array hybridizations were performed with the test genomic DNA from a normal male (1 X chromosome), a normal female (2 X chromosomes) and three cell lines containing three, four, and five copies of the X chromosome. Each plot stands for the mean value of all normalized fluorescence ratio of 57 clones from the X chromosome. The ratio on each of the X chromosome clone was normalized by the mean fluorescence intensity ratio of autosomal chromosome clones. The fluorescence intensity ratio of array-hybridization with normal male versus normal male was defined as 0. Each plot was then computed on the basis of the normalized value. The line represents the linear regression through all of the data with a slope of 0.51 and an intercept of 0.72.

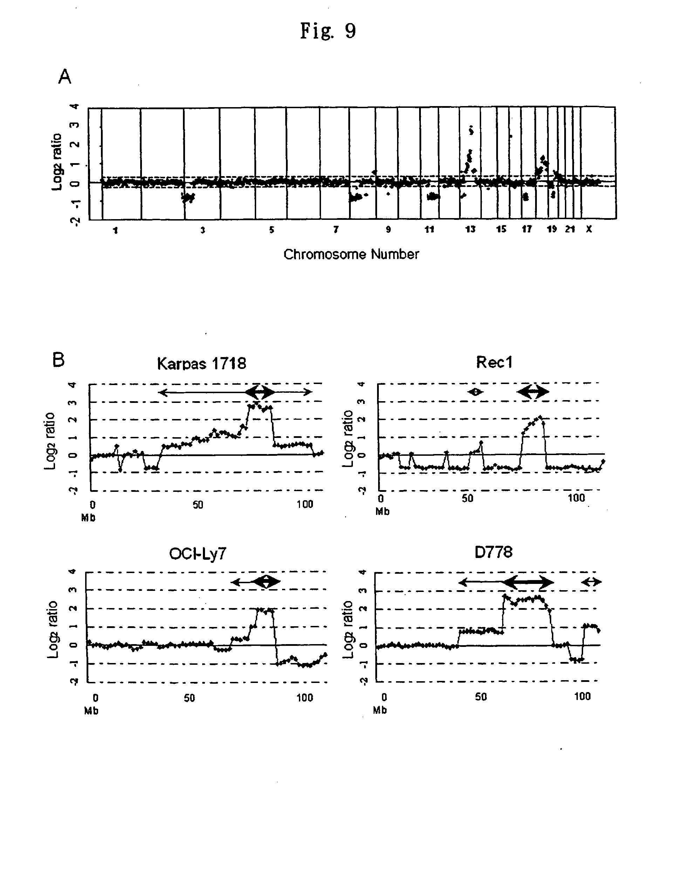

Fig. 9 is genomic profiles of array CGH. A, the representative genomic profile of array CGH with Karpas 1718. The BACs are ordered by position in the genome beginning at the 1p telomere and ending at the Xq telomere. The black arrow above the graph indicates high-level amplification (defined as $\log_2$ ratio > 1). B, detailed genomic profiles of chromosome 13 in the three cell lines (Karpas 1718, Rec1 and OCI-Ly7) and one DLBCL patient (D778). The $\log_2$ ratio for each of the 68 BAC and PAC clones is plotted as a function of its genome location, with chromosome 13q-centromere to the left and 13q-telomere to the right. Horizontal dotted lines show the threshold for gains and loss. Bold arrows indicate high- level amplification (defined as $\log_2$ ratio > 1) and thin arrows moderate-level amplification (0.2 > $\log_2$ ratio > 1). Karpas 1718 shows a wide region of amplification extending over more than 50-Mb of chromosome 13q. Furthermore, high-level amplification in Karpas 1718 is observed from 13q22.2 to 13q31.3, with 13q31.3 in particular showing the highest amplification ($\log_2$ ratio > 2). In the same manner, high-level amplification of OCI-Ly7 and Rec1 are shown at 13q31.3. Rec1 also shows wide loss in the vicinity of 13q31.3. The patient sample (D778) shows a wide region of amplification at 13q21.2-13q31.3 and 13q33.3-qter, with 13q31.1-q31.3 in particular indicating high-level amplification.

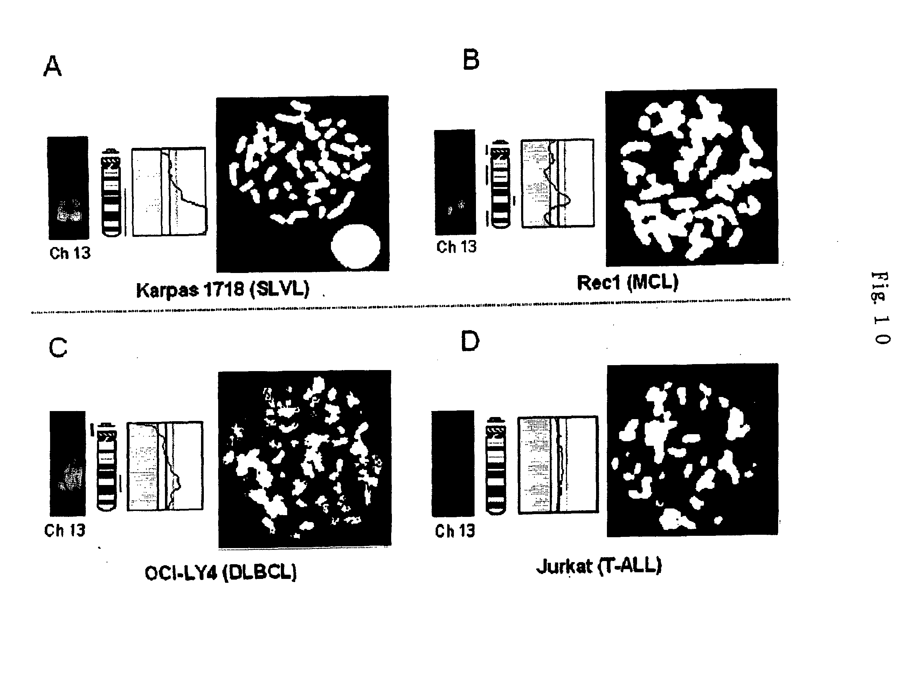

Fig. 10 is FISH and CGH analysis of cell lines with or without amplification at 13q31-q32. CGH results for four cell lines (Karpas 1718, Rec1, OCI-Ly4 and Jurkat) are shown. The lines to the right (green) and to the left (red) of each chromosome indicate the region gained or lost, respectively. Representative results of metaphase FISH with BAC, RP11-487A2 are shown on the right side of each panel. Chromosome 13 examined by means of CGH is also shown beside each ideogarm. Three B-cell lymphoma cell lines, Karpas 1718 (A), Rec1 (B) and OCI-Ly4 (C) show amplification at 13q31-q32, but Jurkat (D) does not show one. FISH analysis shows amplification in more than 15 copies in the three B-cell lines, but no amplification in Jurkat. Each metaphase chromosome was counterstained by DAPI.

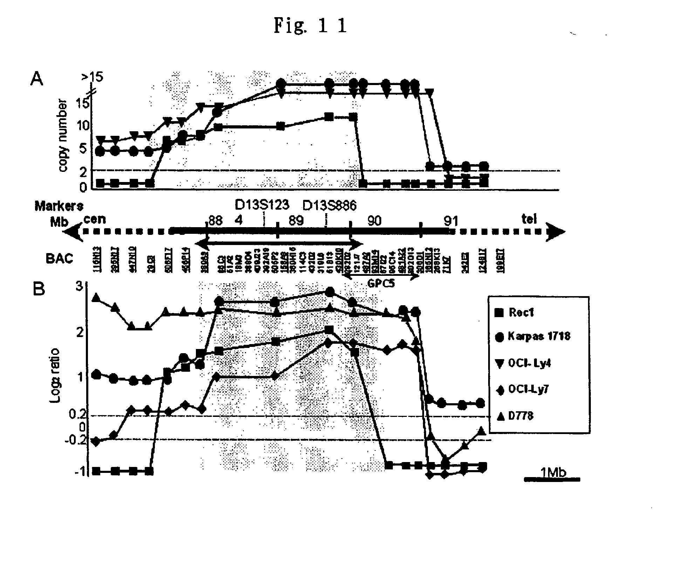

Fig. 11 is analysis of 13q31-q32 by a combination of array CGH and interphase FISH. A, summarized data of array CGH analysis of three cell lines (Rec1 Karpas 1718, and OCI-Ly7) and one DLBCL patient (D778). The vertical line shows $\log_2$ ratio. Horizontal dotted lines show the threshold for gain and loss set at $\log_2$ ratios of +0.2 and -0.2, respectively. The gray box shows the common region of high-level amplification ($\log_2$ ratio > 1) in the three cell lines, which is extended from RP11-360A9 to RP11-481A22. B, summarized data of DNA sequence copy numbers in three cell lines (Karpas 1718, OCI-Ly4, and Rec1) determined by interphase FISH using 19 BAC clones of 13q31.3, including a new BAC; RP11-93M14 that was not used for array CGH. Ten interphase cells were analyzed and the average copy numbers of the BAC clone signals were counted for each cell line. The vertical line indicates the copy number and the horizontal dotted line indicates normal two copies. The gray box shows the common region of gain in copy number, which extended from RP11-29C8 to RP11-93M14. The positions of STS markers and all BAC clones were confirmed from information archived by Ensembl Genome Data Resource (http://www.ensembl.org/). The underlined BAC clones were used for FISH and array CGH. The thin arrow indicates the *GPC5* gene loci.

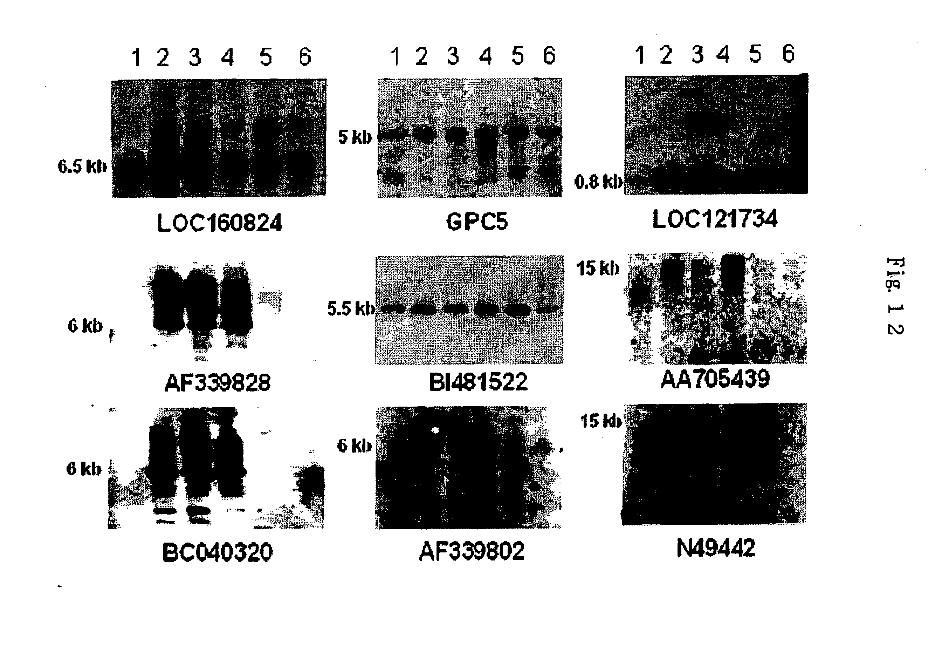

Fig. 12 is northern blot analysis of the candidate gene for 13q31-q32 amplification. Northern hybridization was performed against six kinds of RNAs comprising human placenta (lane 1), three B-cell lymphoma cell lines (lane 2:

Rec1, lane 3: Karpas 1718, lane 4: OCI-Ly4) with amplification at 13q31-q32 and two T-cell lymphoma cell lines (lane 5: Jurkat and lane 6: ATN-1) without amplification. Representative and characteristic expression patterns of eight of 30 ESTs and *GPC5* are shown. Expression of *GPC5* and BI481522 was not significantly different, while LOC160824, AF339828, BC040320, AF339802, LOC121734, AA705439 and N49442 showed clearly different patterns of expression. In particular, the expression of AF339828 and BC040320, which showed similar patterns of hybridization, demonstrates concordance with the gain in copy number at 13q31-q32.

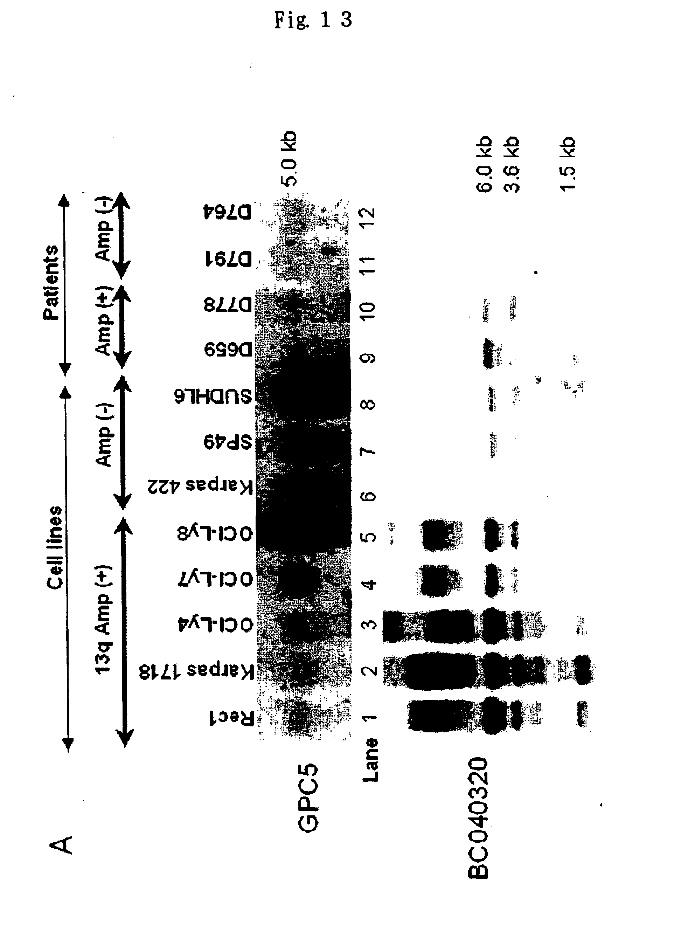

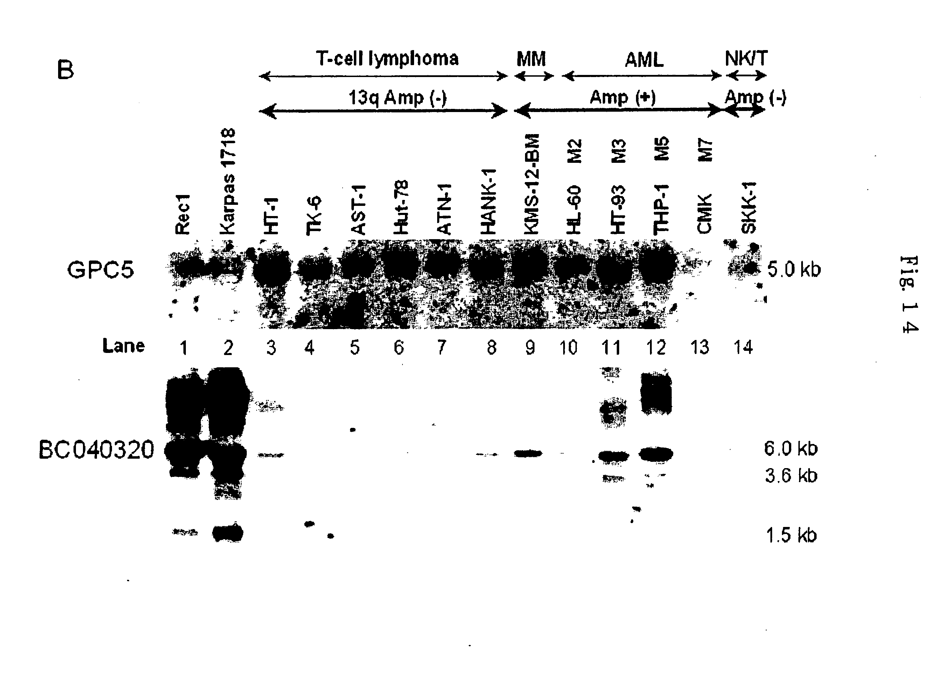

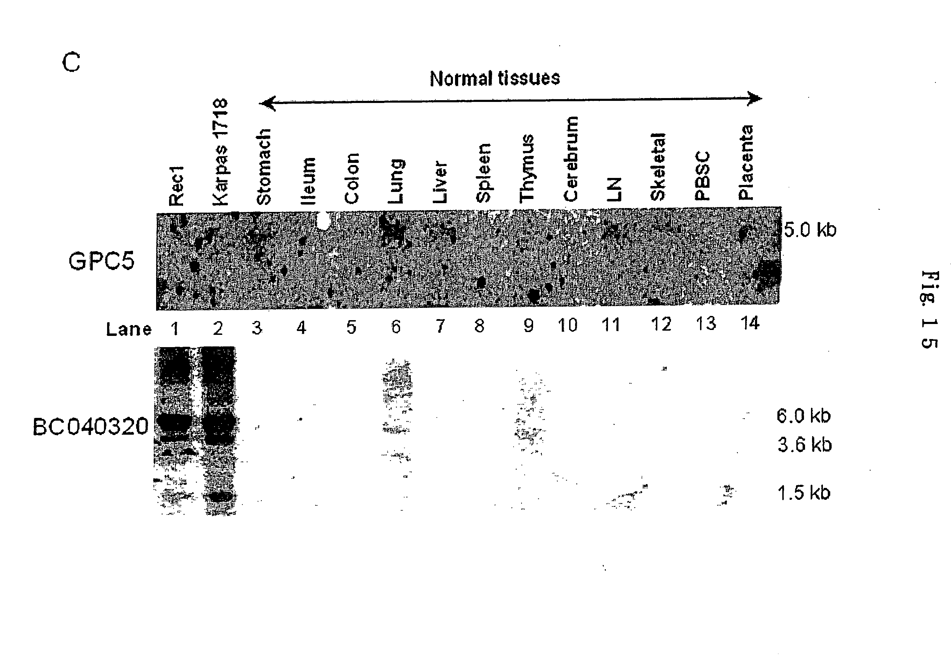

Figs. 13, 14 and 15 are xpression study of *GPC5* and BC040320. Amplification status at 13q31-q32 in each of the cell lines and DLBCL patients was examined by means of conventional-CGH is indicated above names of the samples. Fig. 13 is expression pattern of *GPC5* and BC040320 in cell lines and DLBCL patients with or without amplification at 13q31-q32. Expression of *GPC5* in five cell lines and two DLBCL patients with amplification at 13q31-q32 is not significantly different from that of the other cell lines and patients without amplification. BC040320 is expressed in cell lines with amplification at 13q31-q32 (lanes 1-5) and at much lower levels in cell lines without amplification (lanes 6-8). In the same manner, BC040320 is strongly expressed in DLBCL patients with amplification at 13q31-q32 (lanes 9 and 10), but very weakly in cell lines without amplification (lanes 11 and 12). Fig. 14 is expression pattern of *GPC5* and BC040320 in multiple cell lines with hematopoietic malignancies. Some cell lines (lanes 9, 11, and 12) with amplification at 13q31-q32 show weak signals when compared to the two cell lines (lanes 1 and 2) with high-level amplification. Expression of *GPC5* shows very weak signals with some variations but without significant differences. AML: acute myeloid leukemia cell line. MM: multiple myeloma cell line. NK/T: NK/T cell lymphoma/leukemia cell line. Fig. 15 is expression pattern of BC040320 and *GPC5* in multiple normal tissues. Expression of BCO40320 is hardly visible in normal tissues except for lung, thymus and lymph node when compared to that of the two cell lines (lanes 1 and 2) with high-level amplification at 13q31-q32.

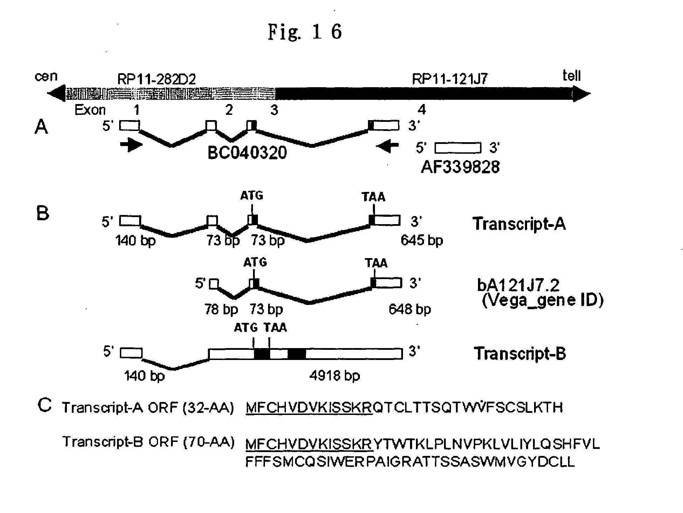

Fig. 16 is exon-intron structure of the *C13orf25* gene. A, two ESTs, BC040320 and AF339828, which are over-expressed in the cell lines with amplification at 13q31.3, are shown above the horizontal dotted line. BC040320 is split into four exons, encompassing two BAC clones, RP11-282D2 and RP11-121J7. AF339828 is located to the telomeric side of BC040320 and about 300-bp apart from BC040320. The primer set used for RT-PCR is shown below the exons. B, two transcripts obtained by RT-PCR. One (Transcript-A) is the same as the BC040320 sequence consisting of four exons containing 965-bp nucleotides. The other (Transcript-B) consists of two exons containing 5058-bp nucleotides. Computer analysis showed that 32-AA polypeptides of bA121J7.2 (Vega_gene ID) were encoded in the Transcript-A cDNA. Possible ORFs are shown as gray boxes. Five precursor microRNAs (miRNAs) (miR91-precursor-13 micro RNA, miR 18- precursor-13 micro RNA, miR19a- precursor-13 micro RNA, miR19b-precursor-13 micro RNA, and miR92- precursor-13 micro RNA), including seven mature microRNAs (microRNA miR-17, miR-91, miR-18, miR-19a, miR-20, miR-19b and miR-92) were obtained from the transcript-B sequence, and are shown by the black box in Transcript-B. C, polypeptides sequences are also shown below the structure. The polypeptides of 13-AA are shared by Transcript-A and Transcript-B, and are indicated by underlining.

**Best Mode for Carrying Out the Invention**

The first invention is a method for isolating a chromosomal DNA from a CD5+DLBCL patient and a CD5-DLBCL patient and for determining as follows with respect to amplification and deletion of a specific region of the chromosomal DNA.

(1) The prognosis of the CD5+DLBCL patient with amplification of chromosome 13 q21.1-q31.3 (13q21.1-q31.3) region is determined to be poor.
(2) The prognosis of the CD5+DLBCL patient with deletion of chromosome 1 p36.21-p36.13 (1p36.21-p36.13) region is determined to be poor.
(3) The prognosis of the CD5-DLBCL patient with amplification of chromosome 5 p15.33-p14.2 (5p15.33-p14.2) region is determined to be good.

One aspect of the first invention is a method for measuring the amplification or the deletion of the chromosomal region by hybridization of a plurality of DNA probes containing said chromosomal region with a chromosomal DNA from the patient. As the DNA probes, a known BAC/PAC DNA clones can be used. More specifically, among BAC/PAC DNA clones for respective chromosome shown in Tables 2 to 7, a clone which is hybridized to each of the chromosomal regions of the (1) to (3) described above can be selected for use. In addition, hybridization can be carried out in a liquid phase system, however, it can be carried out by using a solid phase system, particularly a DNA array in which the BAC/PAC DNA clone was immobilized on a solid phase carrier. A method using such a DNA array can be carried out by preparing a DNA array in accordance with the "array CGH" method shown in the Examples described later.

The third invention is a method for determining the prognosis of a DLBCL patient by using, as an index, an increasede gene expression or a descreased gene expression in the foregoing 3 chromosomal regions (13q21.1-q31.3 region, 1p36.21-p36.13 region and 5p15.33-p14.2 region). Examples of the gene include the genes contained in the respective chromosomal regions shown in Tables 2 to 7, and they can be used as a target. In addition, the invention of this application provides C13orf25 gene contained in 13q21.1-131.3 region as the gene (with respect to a specific configuration or the like, see Example 2 described later).

The method for prognosis of the third invention is a method for measuring the expression level of each gene (e.g., C13orf25) in the biological sample of the patient and determining the prognosis of the DLBCL patient by using the expression level of this gene as an index. More specifically, if the expression level of the gene in each chromosomal region is significantly high or low in comparison with that in the biological sample of a healthy subject, the patient is determined to be a poor prognosis patient with DLBCL or a patient with high risk of poor prognosis. The term that the expression level of the gene is "significantly high" means the case where the expression level of the gene of a patient is higher by 10% or more, preferably 30% or more, more preferably 70% or more, most preferably 100% or more in comparison with that of the same gene measured in the biological sample of a healthy subject. In addition, the term "significantly high" also includes the case, for example, when the mean value of the expression level of the gene in a plurality of samples of the same subject and the mean value obtained in the same manner for samples of a plurality of healthy subjects are statistically tested, where the former is significantly higher than the latter.

Each gene to be tested can be easily obtained by a known method, respectively. For example, in the case of a cDNA, it can be obtained by synthesizing a cDNA library by using a known method (Mol. Cell Biol. 2, 161-170, 1982; J. Gene 25, 263_269, 1983; Gene, 150, 243-250, 1994), and by a method of isolating the respective cDNAs with the use of a probe DNA prepared based on the known nucleotide sequences, respectively. The obtained cDNA can be amplified by a commonly performed gene amplification method such as the PCR (Polymerase Chain Reaction) method, the NASBN (Nucleic acid sequence based amplification) method, the TMA (Transcription-mediated amplification) method or the SDA (Strand Displacement Amplification) method. In addition, by using the primer set provided by this invention, a necessary amount of each cDNA can be obtained by also the RT-PCR method using a mRNA isolated from a human cell as a template.

The method for prognosis using the expression level of gene as an index as described above can be carried out in accordance with the known techniques of genetic engineering and molecular biology by detecting and measuring the expression level of HRF polynucleotide by a method known for detecting and measuring the expression of a specific gene in the art such as in situ hybridization, northern blotting, dot blot, RNase protection assay, RT-PCR, Real-Time PCR (Journal of Molecular Endocrinology, 25, 169-193 (2000) and the literatures cited therein), DNA array analysis (Mark Shena (ed.), "Microarray Biochip Technology", Eaton Publishing, March 2000). A measuring system for gene expression, a detection system for DLBCL disease and a risk detection system for DLBCL disease, in which such a technique is used, and a reagent, a method, a process and an analysis program, which are used for the systems, and the like are all included in the techniques of this invention and the systems used therefor.

This application particularly provides the following inventions as a material used in the foregoing method of the third invention.

That is, an oligonucleotide probe is characterized by being hybridized to a gene to be tested under a stringent condition.

Such an oligonucleotide probe can be also obtained by, for example, digesting a purified polynucleotide of a gene to be tested or its cDNA with an appropriate restriction enzyme. Alternatively, it can be synthesized in vitro by a known chemical synthesis technique as described in Carruthers (1982) Cold Spring Harbor Symp. Quant. Biol. 47: 411-418; Adams (1983) J. Am. Chem. Soc. 105: 661; Belousov (1997) Nucleic Acid Res. 25: 3440-3444; Frenkel (1995) Free Radic. Biol. Med. 19: 373-380; Blommers (1994) Biochemistry 33: 7886-7896; Narang (1979) Meth. Enzymol. 68: 90; Brown (1979) Meth. Enzymol. 68: 109; Beaucage (1981) Tetra. Lett. 22: 1859; or U.S. Patent No. 4,458,066.

The stringent condition is a condition capable of selective and detectable specific binding of the foregoing polynucleotide to the oligonucleotide probe. The stringent condition is defined by the concentration of a salt, an organic solvent (e.g., formamide), the temperature and other known conditions. More specifically, stringency is increased by decreasing the concentration of a salt, increasing the concentration of an organic solvent or increasing the hybridization temperature. For example, the stringent concentration of a salt is commonly about 750 mM or less of NaCl and about 75 mM or less of trisodium citrate, more preferably about 500 mM or less of NaCl and about 50 mM or less of trisodium citrate, most preferably about 250 mM or less of NaCl and about 25 mM or less of trisodium citrate. The stringent concentration of an organic solvent is about 35% or more, more preferably about 50% or more of formamide. The stringent temperature condition is about 30°C or higher, more preferably about 37°C or higher, most preferably about 42°C or higher. Other conditions include hybridization time, the concentration of a washing agent (e.g., SDS), presence or absence of a carrier DNA and the like, and various stringent conditions can be specified by combining these conditions. As one preferred embodiment, hybridization is carried out under the condition of 750 mM NaCl, 75 mM trisodium citrate and 1% SDS at 30°C. As a more preferred embodiment, hybridization is carried out under the condition of 500 mM NaCl, 50 mM trisodium citrate, 1% SDS, 35% formamide and 100 μg/ml of denatured salmon sperm DNA at 37°C. As a most preferred embodiment, hybridization is carried out under the condition of 250 mM NaCl, 25 mM trisodium citrate, 1% SDS, 50% formamide and 200 μg/ml of denatured salmon sperm DNA at 42°C. In addition, a washing condition after the hybridization will affect the stringency. The washing condition is also defined by the concentration of a salt and the temperature, and the stringency of washing is increased by decreasing the concentration of a salt and increasing the temperature. For example, the stringent salt condition for washing is preferably about 30 mM or less of NaCl and about 3 mM or less of trisodium citrate, most preferably about 15 mM or less of NaCl and about 1.5 mM or less of trisodium citrate. The stringent temperature condition for washing is about 25°C or higher, more preferably about 42°C or higher, most preferably about 68°C or higher. As one preferred embodiment, washing is carried out under the condition of 30 mM NaCl, 3 mM trisodium citrate and 0.1% SDS at 25°C. As a more preferred embodiment, washing is carried out under the condition of 15 mM NaCl, 1.5 mM trisodium citrate and 0.1% SDS at 42°C. As a most preferred embodiment, washing is carried out under the condition of 15 mM NaCl, 1.5 mM trisodium citrate and 0.1% SDS at 68°C.

In addition, the oligonucleotide probe can be labeled with a label used in the technical field. Labeling can be carried out by the radioisotope (RI) method or the non-RI method, however, it is preferred that the non-RI method be used. Examples of the non-RI method include the fluorescence labeling method, the biotin-labeling method, the chemiluminescence method and the like, however, it is preferred that the fluorescence labeling method be used. As the fluorescent substance, the one that can be bound to the base region of the oligonucleotide can be selected as appropriately for use, however, a cyanine dye (e.g., Cy Dye™ series such as Cy3 or Cy5), rhodamine 6G reagent, N-acetoxy-$N^2$-acetylaminofluorene (AAF), AAIF (an iodide derivative of AAF) or the like can be used. As the labeling method, a method known in the art (e.g., the random prime method, the nick translation method, DNA amplification by PCR, the labeling/tailing method, the in vitro transcription method or the like) can be selected as appropriately for use. For example, a labeled oligonucleotide probe can be prepared by introducing a functional group (e.g., a primary aliphatic amine group, a SH group or the like) into HRF oligonucleotide, and attaching the foregoing label to the functional group.

With regard to the DNA array of this invention, the foregoing oligonucleotide or a full length or a part of a gene cDNA is used as a target capture probe. As the method of preparing the DNA array, a method of synthesizing an oligonucleotide directly on the surface of a solid phase carrier (on-chip method) and a method of immobilizing an oligonucleotide or a polynucleotide prepared in advance on the surface of a solid phase carrier are known. The DNA array of this invention can be prepared by either of the methods. The on-chip method can be carried out by a method of performing a selective synthesis in a predetermined region of a small matrix (a masking technique: e.g., Fodor, S.P.A. Science 251: 767, 1991) or the like by combining a use of a protecting group that is selectively removed by exposure to light with a photolithography technique that is used for semiconductor production and a solid phase synthesis technique. On the other hand, in the case where an oligonucleotide or a polynucleotide prepared in advance is immobilized on the surface of a solid phase carrier, an oligonucleotide into which a functional group was introduced is synthesized, the oligonucleotide is spotted on the surface of the solid phase carrier subjected to a surface treatment, and have it covalently bound thereto (e.g., Lamture, J.B. et al. Nucl. Acids Res. 22: 2121-2125, 1994; Guo, Z. et al. Nucl. Acids Res. 22: 5456-5465, 1994). In general, the oligonucleotide or polynucleotide is covalently bound to the solid phase carrier subjected to a surface treatment via a spacer or a crosslinker. A method of aligning small pieces of polyacrylamide gel on the surface of glass and having the synthesized oligonucleotide covalently bound thereto (Yershov, G. et al. Proc. Natl. Acad. Sci. USA 94: 4913, 1996) is also known. In addition, a method of preparing an array of microelectrode on a silica microarray, in which a permeation layer of agarose containing streptavidin is provided on the electrode to make it a reactive region, immobilizing a biotinylated oligonucleotide by positively charging the region and controlling the electric charge of this region, thereby enabling high-speed and stringent hybridization is also known (Sosnowski, R.G. et al. Proc. Natl. Acad. Sci. USA 94: 1119-1123, 1997). In the case where the probe is dropped on the surface of the solid phase substrate to perform spotting, it can be performed by a pin system (e.g., U.S. Patent No. 5,807,5223), however, it is preferred that an inkjet system disclosed in JP 2001-116750A or JP 2001-186881A be adopted because uniform spots in a given shape are formed. In addition, this inkjet system can make the number of probes contained in the respective probe spots equal, therefore, the difference in hybridization due to the difference in the probe length can be accurately measured. Further, it is recommended for forming preferred spots that duplicate spotting be performed as disclosed in JP 2001-186880A, or a probe solution (a solution containing a moisturizing substance) comprising the composition disclosed in WO 03 / 038089 A be used.

After the spotting, each spot is immobilized on the solid phase substrate by cooling, adding moisture to the spots (maintaining a humidity of up to about 80% for a given period of time) and performing an immobilization treatment or the like by calcination and drying, whereby the microarray can be completed. As the solid phase substrate for the microarray, other than glass (slide glass) used for a common microarray, plastic, silicone, ceramic or the like can be also used.

In the case where diagnosis is performed by using this microarray, for example, a cDNA is synthesized by using a mRNA isolated from a cell of a patient as a template and PCR amplification is performed. During this time, the cDNA is labeled by incorporating a labeled dNTP. The labeled cDNA is brought into contact with the microarray and the cDNA hybridized to the capture probe (oligonucleotide or polynucleotide) on the microarray is detected. Hybridization can be carried out by spotting an aqueous solution of the labeled cDNA dispensed on a 96-well or 384-well plastic plate on the microarray. The amount to be spotted can be about 1 to 100 nl. It is preferred that hybridization be carried out at a temperature from room temperature up to 70°C for 6 to 20 hours. After finishing the hybridization, washing is carried out by using a mixed solution of a surfactant and a buffer solution to remove unreacted labeled cDNAs. As the surfactant, it is preferred that sodium dodecyl sulfate (SDS) be used.. As the buffer solution, citrate buffer solution, phosphate buffer solution, borate buffer solution, Tris buffer solution, Good's buffer solution or the like can be used, however, it is preferred that citrate buffer solution be used.

The oligonucleotide primer of this invention is designed based on the known nucleotide sequence of each gene and can be prepared through each step of synthesis and purification. Incidentally, the points of designing the primer to be kept in mind may include, for example, as follows: The size (the number of bases) of the primer should be 15 to 40 bases, desirably 15 to 30 bases considering satisfying a specific annealing with a template DNA. Note that in the case where LA (long accurate) PCR is carried out, at least 30 bases are effective. In order not to anneal one set or one pair (2 strands) of primers comprising a sense strand (5' terminal side) and an antisense strand (3' terminal side) each other, complementary sequences of both primers should be avoided and in order to prevent the formation of a hairpin structure in the primers, self-complementary sequences should be also avoided. Further, in order to ensure the stable bond with the template DNA, the GC content should be about 50%, and GC-rich or AT-rich should not occur in the primer. Since the annealing temperature depends on Tm (melting temperature), in order to obtain a PCR product with high specificity, primers whose Tm values are proximate each other within 55 to 65°C should be selected. In addition, it is necessary that the final concentration of the primer usage in PCR should be about 0.1 to about 1 μM. In addition, commercially available software for designing primer, for example, OligoTM (National Bioscience Inc., made in the US), GENETYX (Software Development Co., Ltd., made in Japan) or the like can be also used.

By using the materials as above, the prognosis of a CD5+DLBCL patient can be determined for example as follows.

One embodiment is a method (Northern blot analysis) for detecting the expression level (mRNA level) of a gene to be tested (e.g., C13orf25) by using the oligonucleotide probe. This diagnostic method is characterized by comprising at least the following steps:

(a) a step of preparing RNAs from a biological sample of a patient;
(b) a step of separating the RNAs prepared in the step (a) by electrophoresis;
(c) a step of hybridizing the RNAs separated in the step (b) to the oligonucleotide probe under a stringent condition;
(d) a step of comparing the level of the labeled oligonucleotide probes hybridized to the RNAs in the step (e), which is assigned to be the index of the expression level of gene, with the results obtained from a normal biological sample; and
(e) a step of using the expression level of gene which is significantly high or low in comparison with that in a normal biological sample as an index for indicating the degree of the prognosis of a DLBCL patient.

Another embodiment is a method of using the DNA array. This method is characterized by comprising at least the following steps:

(a) a step of preparing RNAs from a biological sample of a patient;
(b) a step of preparing labeled cDNAs from the RNAs prepared in the step (a);
(c) a step of bringing the labeled cDNAs prepared in the step (b) into contact with the DNA array;
(d) a step of comparing the level of the labeled cDNAs hybridized to the capture probe on the DNA array in the step (c), which is assigned to be the index of the expression level of gene, with the results obtained from a normal biological sample; and
(e) a step of using the expression level of gene which is significantly high or low in comparison with that in a normal biological sample as an index for indicating the degree of the prognosis of a DLBCL patient.

Still another embodiment is a method (RT-PCT method) for measuring the expression level of gene mRNA by using a primer set. This method is characterized by comprising at least the following steps:

(a) a step of preparing RNAs from a biological sample of a patient;
(b) a step of synthesizing cDNAs by using the RNAs prepared in the step (a) as a template with the use of a primer set;
(c) a step of comparing the level of the cDNAs synthesized in the step (b), which is assigned to be the index of the expression level of gene, with the results obtained from a normal biological sample; and
(d) a step of using the expression level of gene which is significantly high or low in comparison with that in a normal biological sample as an index for indicating the degree of the prognosis of a DLBCL patient.

Still further, the diagnostic method of the third invention can be carried out by combining the foregoing northern blot method, DNA array method and RT-PCR method as needed.

With regard to the respective diagnostic methods as above, for observing the label or measuring the labeled amount, a method known in the art can be selected for use as needed depending on the type of the label. For example, methods such as dark field microscopy, phase contrast microscopy, reflection contrast microscopy, fluorescence microscopy, digital imaging microscopy and electron microscopy can be also used.

Another aspect of the third invention is a method targeting a protein as a transcript of gene to be tested. The method for prognosis of using the protein level as an index can be carried out in accordance with the known techniques of genetic engineering and molecular biology by detecting and measuring the level of protein to be tested by a method known for detecting and measuring the level of a specific protein in the art such as in situ hybridization, western blotting and various immunohistological methods. A measuring system for protein level, a detection system for the prognosis of DLBCL and a risk detection system for DLBCL, in which such a technique is used, and a reagent, a method, a process and an analysis program, which are used for the systems, and the like are all included in the techniques of this invention and the systems used therefor.

In the prognosis according to the third invention, as one preferred embodiment, an antibody which specifically binds to a protein to be tested is used. Note that the term "antibody" in this specification may be the one used in the extensive meaning, a single monoclonal antibody to a desired polypeptide or a peptide fragment related thereto, or an antibody composition having specificity to a variety of epitopes. In addition, it includes a monovalent antibody, a polyvalent antibody, a polyclonal antibody and a monoclonal antibody, and also represents an intact molecule, a fragment thereof and a derivative thereof, and includes fragments such as F(ab'), Fab' and Fab. Further, it may include a chimera antibody or a hybrid antibody having binding sites for at least two antigens or epitopes, a recombinant antibody with dual specificity such as quadrome or triome, an interspecies hybrid antibody, an antiidio type antibody, the one which was chemically modified or processed and is considered to be a derivative thereof, an antibody obtained by applying a known cell fusion, hybridoma technique or antibody engineering, or using a synthetic or semisynthetic technique, an antibody prepared by applying a known conventional technique in view of the production of antibody or using a DNA recombinant technique, an antibody having neutralizing property related to a target antigenic substance or a target epitope described and defined in this specification, and an antibody having binding property. A particularly preferred antibody is the one which can specifically distinguish an intact protein (polypeptide).

In other words, the antibody is an antibody prepared by using a partial peptide of a protein to be tested as an antigen, and it is preferably used as a set of antibodies which recognize different sites of one protein. The peptide for preparing such an antibody is, for example in the case of using a C13orf25 gene product as a target protein, a peptide comprising the amino acid sequences of SEQ ID NO: 4 and 5, and it is synthesized by, for example, the Fmoc-bop method with a peptide synthesizer. A cysteine may be introduced into the N-terminal of HRF peptide. The synthesized peptide is purified by high performance liquid chromatography using a μBondasphere, a C18 column (Waters) and the like and used as an immunizing antigen.

Such an antibody can be obtained, for example in the case of a polyclonal antibody, from serum after immunizing an animal with a protein or a partial fragment (oligopeptide) thereof as an immunogen. Alternatively, it can be prepared by introducing a recombinant vector of a protein polynucleotide into the muscle or the skin of an animal with an injector or a gene gun, and collecting the serum. As the animal, mouse, rat, hamster, rabbit, goat, sheep, cow, horse, pig, dog, cat, monkey, chicken or the like is used. Further, there is a case where it is preferred that the animal should be selected considering the compatibility with a parent cell to be used for cell fusion.

Immunization of an animal with an sensitizing antigen is carried out in accordance with a known method, for example, it can be carried out in accordance with the method described in Shigeru Muramatsu et al. (ed.), Jikken Seibutsugaku Koza 14, Immunobiology, Maruzen, 1985; Japanese Biochemical Society (ed.), Zoku Seikagaku Jikken Koza 5, Meneki Seikagaku Kenkyuho, Tokyo Kagaku Dozin, 1986; Japanese Biochemical Society (ed.), Shin Seikagaku Jikken Koza 12, Molecular Immunology III, Antigens, Antibodies, and Complements, Tokyo Kagaku Dozin, 1992; or the like. For example, as a general method, immunization can be carried out by injecting a sensitizing antigen intraperitoneally or subcutaneously into a mammal. In addition, during the immunization with the sensitizing antigen, an appropriate carrier can be also used. Immunization is attained by injecting an immunizing agent (if necessary together with an adjuvant) once or more times into a mammal. Typically, the immunizing agent is subcutaneously or intraperitoneally injected, alone or together with an adjuvant, into a mammal a plurality of times. Examples of the immunizing agent include the foregoing antigen peptide or a peptide fragment related thereto. The immunizing agent may be used in the form of a conjugate with a protein (e.g., one of the foregoing carrier proteins) known to be antigenic in the mammal to be treated for immunization. Examples of the adjuvant include, for example, Freund's complete adjuvant, Ribi adjuvant, pertussis vaccine, BCG, lipid A, liposomes, aluminum hydroxide, and silica and the like.

An antiserum containing the polyclonal antibody can be prepared from the blood collected from the animal after feeding of the immunized animal for a predetermined period. After confirming that the obtained antiserum recognizes HRF, it is submitted to use as a predetermined active ingredient of this invention.

As the antibody of this invention, the one obtained as a monoclonal antibody derived from a mammal can be also used. The monoclonal antibody produced against the antigenic substance can be produced by any of the methods capable of causing the production of antibody molecules in a series of cell lines under cultivation. The modifier "monoclonal" indicates the characteristic of an antibody that it is obtained from a substantially homogeneous antibody population. It is not to be construed that the antibody should be produced by a certain specific method. Individual monoclonal antibodies each include a population of the same antibodies except that a slight amount of a mutant possibly formed spontaneously may be present therein. Monoclonal antibodies each has high specificity and is directed to one single antigenic site. As compared with an ordinary (polyclonal) antibody preparation typically containing various antibodies directed to different antigenic determinants (epitopes), each monoclonal antibody is directed to one single antigenic determinant on the antigen. In addition to their specificity, monoclonal antibodies are synthesized by hybridoma culture and are superior in that they are not or only a little contaminated with other immunoglobulins. The monoclonal antibodies include hybrid antibodies and recombinant antibodies. So long as they show a desired biological activity, a constant region domain may be substituted for a variable region domain thereof, or a heavy chain may be substituted for a light chain thereof, a chain derived from a certain species may be replaced with a chain derived from another species, or they may be fused with a heterogeneous protein, irrespective of their origin or immunoglobulin class or subclass (e.g. U.S. Patent No. 4,816,567; Monoclonal Antibody Production Techniques and Applications, pp. 79-97, Marcel Dekker, Inc., New York, 1987, etc).

The monoclonal antibody can be prepared in accordance with a known method for preparing monoclonal antibodies ("Monoclonal Antibody", co-authored by Komei Nagamune and Hiroshi Terada, Hirokawa Shoten, 1990; "Monoclonal Antibody", James W. Goding, third edition, Academic Press, 1996).

With regard to the antibody of this invention, the one in the form being further purified if necessary is used. As the method for purifying and isolating the antibody, a conventionally known method, for example, salting out by the ammonium sulfate precipitation, gel filtration using Sephadex, ion exchange chromatography, electrophoresis, dialysis, ultrafiltration, affinity chromatography, high-performance liquid chromatography or the like can be used for purification. Preferably, ascitic fluid containing the antiserum or the monoclonal antibody can be purified and isolated by ammonium sulfate fractionation, followed by treatment with an anion exchange gel such as DEAE-Sepharose, and an affinity column such as a protein A column. Especially preferred are affinity chromatography with an immobilized antigen or antigen fragment (e.g., synthetic peptide, recombinant antigen protein or peptide, site specifically recognized by the antibody), affinity chromatography with an immobilized protein A, hydroxyapatite chromatography and the like.

By treatment of those antibodies with an enzyme such as trypsin, papain or pepsin, antibody fragments such as Fab, Fab' and F(ab') 2, if necessary followed by reduction may also be used. The antibodies can be used in any of the known assay methods, for example, competitive binding assay, direct and indirect sandwich assay, and immunoprecipitation (Zola, Monoclonal Antibodies: A Manual of Techniques, pp. 147-158 (CRC Press, Inc. 1987)).

In order to conjugate the antibody to a detectable atomic group, any of the methods known in the field can be used and, examples include, for example, the methods described in David et al., Biochemistry, Vol. 13, pp. 1014-1021 (1974); Pain et al., J. Immunol. Meth., 40: pp. 219-231 (1981); and "Methods in Enzymology", Vol. 184, pp. 138-163 (1990). As the antibody to be labeled, an IgG fraction and, further, the specific binding portion Fab' obtainable by reduction following pepsin digestion can be used.

A large number of carriers capable of immobilizing antigen or antibody are known, and an appropriate one may be selected from among them for use in this invention. Various carriers are known to be useful in an antigen-antibody reaction or the like and, of course, an appropriate one can be selected from such known ones for use in this invention. Especially preferred for use are glass, for example activated glass such as aminoalkylsilylated glass, porous glass, silica gel, silica-alumina, alumina, magnetized iron, magnetized alloys and other inorganic materials, polyethylene, polypropylene, polyvinyl chloride, polyvinylidene fluoride, polyvinyl, polyvinyl acetate, polycarbonates, polymethacrylates, polystyrene, styrene-butadiene copolymers, polyacrylamide, crosslinked polyacrylamide, styrene-methacrylate copolymers, polyglycidyl methacrylate, acrolein-ethylene glycol dimethacrylate copolymers and the like, crosslinked albumin, collagen, gelatin, dextran, agarose, crosslinked agarose, cellulose, microcrystalline cellulose, carboxylmethylcellulose, cellulose acetate and other natural or modified cellulose, crosslinked dextran, nylons and other polyamides, polyurethanes, polyepoxy resins and other organic polymers, polymers obtained by emulsion polymerization, silicone rubbers and the like, cells, erythrocytes and the like. If necessary, they may have a functional group introduced therein using a silane coupling agent.

Examples of the carrier include particles, minute particles, microparticles, membrane, filter paper, beads, tubes, cuvettes, inside walls of test vessels such as test tubes, titer plates, titer wells, microplates, glass cells, synthetic resin cells and cells made of some other synthetic materials, and the surfaces of solid substances (bodies) such as glass rods, rods made of a synthetic material, rods having a thickened or tapered end, rods having a round projection or flat projection at an end, and thin plate-like rods.

The binding of the antibody with such a carrier can be realized by physical means such as adsorption, by chemical means using a condensing agent or an activated form, by means utilizing a mutual chemical binding reaction or the like.

The antibodies of this invention include antibodies labeled with a labeling substance, respectively. Examples of the label include enzymes, enzyme substrates, enzyme inhibitors, prosthetic groups, coenzymes, enzyme precursors, apoenzymes, fluorescent substances, dye substances, chemoluminescent compounds, luminescent substances, chromophores, magnetic substances, metal particles such as gold colloid, nonmetallic element particles such as selenium colloid, radioactive substances, and the like. As a preferred labeling substance, an enzyme, a chemical substance such as a radioisotope or a fluorescent dye can be used. There is no particular restriction on the enzyme as long as it fulfills the requirement such as a large turnover number, stability even upon binding to an antibody and an ability of staining a substrate specifically, and an enzyme used for common EIA can be used. Examples of the enzyme may include dehydrogenases, reductases, oxidases and other oxidation-reduction enzymes, transferases catalyzing the transfer of, for example, an amino, carboxyl, methyl, acyl or phosphoryl group, for example, hydrolases hydrolyzing the ester, glycoside, ether or peptide bond, such as lyases, isomerases, ligases and the like. A plurality of enzymes may be used in combination for detection purposes. For example, enzymatic cycling may also be used. A biotin label and an enzyme-labeled avidin (streptavidin) may be substituted for the enzyme label. Thus, it is possible to suitably employ a sensitivity increasing method known in the art, for example the use of such a biotin-avidin system or the use of a secondary antibody such as an antibody to anti-HRF antibody. It is also possible to use a plurality of different types of labels. In such a case, it is also possible to carry out a plurality of measurements continuously or discontinuously, and simultaneously or separately.

Typical examples of the enzyme label include peroxidases such as horseradish peroxidase, galactosidases such as Escherichia coli-derived β-D-galactosidase, malate dehydrogenase, glucose-6-phosphate dehydrogenase, glucose oxidase, glucoamylase, acetylcholine esterase, catalase, bovine small intestine-derived alkaline phosphatase, alkaline phosphatases such as Escherichia coli-derived alkaline phosphatase and the like.

The conjugation of such an enzyme with the antibody can be carried out by a known method using a crosslinking agent such as a maleimide compound. As the substrate, a known substance can be used according to the type of an enzyme to be used, and examples include umbelliferone derivatives such as 4-methylumbelliferyl phosphate, phosphorylated phenol derivatives such as nitrophenyl phosphate and the like. For example, in the case where peroxidase is used as an enzyme, 3,3',5,5'- tetramethylbenzidine can be used, and in the case where alkaline phosphatase is used as an enzyme, p-nitrophenol or the like can be used. In this invention, the combination of enzyme-reagents may also be used for the formation of signals, for example the combination of 4-hydroxyphenylacetic acid, o-phenylenediamine (OPD), tetramethylbenzidine (TMB), 5-aminosalicylic acid, 3,3-diaminobenzidine tetrahydrochloride (DAB), 3-amino-9-ethylcarbazole (AEC), tyramine, luminol, lucigenin luciferin or a derivative thereof, Pholad luciferin or the like with peroxidase such as horseradish peroxidase, the combination of Lumigen PPD, (4-methyl)umbelliferyl phosphate, p-nitrophenol phosphate, phenol phosphate, bromochloroindolyl phosphate (BCIP), AMPAK™ (DAKO), AmpliQ™ (DAKO) or the like with alkaline phosphatase, the combination of an umbelliferyl galactoside such as 4-methylumbelliferyl-β-D-galactoside, a nitrophenyl galactoside such as o-nitrophenyl-β-D-galactoside or the like with β-D-galactosidase or glucose-6-phosphate dehydrogenase, and the combination of ABTS with glucose oxidase, and a compound which can form a quinol compound such as hydroquinone, hydroxybenzoquinone, hydroxyanthraquinone, a thiol compound such as lipoic acid, glutathione, a phenol derivative, a ferrocene derivative or the like under the action of an enzyme or the like can be used.

As the radioisotope, the one used in a common RIA such as $^{32}$P, $^{125}$I, $^{14}$C, $^{35}$S or 3H can be used. Examples of the fluorescent substance or chemiluminescent compound include fluorescein isothiocyanate (FITC), rhodamine derivatives such as rhodamine B isothiocyanate, tetramethylrhodamine isothiocyanate (RITC) and tetramethylrhodamine isothiocyanate isomer R (TRITC), 7-amino-4-coumarin-3-acetic acid, dansyl chloride, dansyl fluoride, fluorescamine, phycobilin protein, acridinium salts, luciferin, luciferase, aequorin and other luminols, imidazole, oxalate esters, rare earth chelate compounds, coumarin derivatives and the like. As the fluorescent dye, the one used for a common fluorescent antibody method can be used. For detecting the resulting signal including coloring, fluorescence and the like, visual observation may be employed, or a known apparatus may also be used, thus, for example, a fluorophotometer or a plate reader may be used. For detecting the signal emitted by a radioisotope (isotope) or the like, a known apparatus may be used, for example, a gamma counter or scintillation counter or the like may also be used.

The labeling of antibody can be carried out by utilizing the reaction between a thiol group and a maleimide group, the reaction between a pyridyl disulfide group and a thiol group, the reaction between an amino group and an aldehyde group or the like, and an appropriate method can be selected for use from among known methods, methods that can be easily carried out by those skilled in the art and, further, the modifications thereof. Further, a condensing agent which can be used in preparing the immunogenic conjugate, a condensing agent which can be used in binding to the carrier or the like can be used. Examples of the condensing agent include, for example, formaldehyde, glutaraldehyde, hexamethylene diisocyanate, hexamethylene diisothiocyanate, N,N'-polymethylenebis-iodoacetamide, N,N'-ethylenebismaleimide, ethylene glycol bissuccinimidyl succinate, bisdiazobenzidine, 1-ethyl-3-(3-dimethylaminopropyl) carbodiimide, succinimidyl 3-(2-pyridyldithio) propionate (SPDP), N-succinimidyl 4-(N-maleimidomethyl)cyclohexane-1-carboxylate (SMCC), N-sulfosuccinimidyl 4-(N-maleimidomethyl) cyclohexane-1-carboxylate, N-succinimidyl (4-iodoacetyl)aminobenzoate, N-succinimidyl 4-(1-maleimidophenyl)butyrate, N-($\varepsilon$-maleimidocaproyloxy)succinimide (EMCS), iminothiolane, S-acetyl-mercaptosuccinic acid anhydride, methyl 3-(4'-dithiopyridyl) propionimidate, methyl 4-mercaptobutyrylimidate, methyl 3-mercapto-propionimidate, N-succinimidyl S-acetylmercaptoacetate and the like.

One aspect in the diagnostic method using such an antibody is a method for detecting the binding of the antibody to the protein to be tested in a liquid phase system. For example, a labeled antibody obtained by labeling an antibody is brought into contact with a biological sample to bind the labeled antibody to the protein, and this conjugate is separated. The separation can be carried out by a method of separating the conjugate of the protein and the labeled antibody by a known separation method (chromatography, solid phase method or the like). In addition, a method in accordance with the known western blot method can be adopted. With regard to the measurement of the labeled signal, in the case of using an enzyme as the label, a substrate which develops color by being decomposed due to an enzymatic action is added, the enzyme activity is achieved by optically measuring the amount of decomposed substrates, which is converted into the amount of bound antibodies, and the amount of antibody is calculated in comparison with the standard value. In the case of using a radioisotope, the amount of radiation emitted by the radioisotope is measured with a scintillation counter or the like. In addition, in the case of using a fluorescent dye, the fluorescent amount may be measured with a measuring apparatus combined with a fluorescence microscope.

In another method for diagnosis in the liquid phase system, an antibody (primary antibody) is brought into contact with a biological sample to bind the primary antibody to the protein to be tested, a labeled secondary antibody is bound to the conjugate, and the labeled signal in the third party of the conjugate is detected. In order to further enhance the signal, first a non-labeled secondary antibody is bound to the conjugate of an antibody and an antigen peptide, and a labeling substance may be conjugated to the secondary antibody. Such conjugation of the labeling substance to the secondary antibody can be carried out by, for example, biotinylating the secondary antibody and avidinylating the labeling substance. In addition, an antibody (tertiary antibody) that recognizes a partial region of the secondary antibody (e.g., Fc region) is labeled, and the tertiary antibody may be bound to the secondary antibody. Note that for both of the primary antibody and the secondary antibody, monoclonal antibodies can be used, or for either of the primary antibody or the secondary antibody, a polyclonal antibody can be used. The separation of the conjugate from the liquid phase or the detection of the signal can be carried out in the same manner as described above.

Another diagnostic method using the antibody is a method of testing the binding of the antibody to the protein in a solid phase system. The method in the solid phase system is a preferred method for the detection of a very few amount of the protein to be tested and the convenience of the operation. More specifically, the method in the solid phase system is a method in which an antibody is immobilized on a resin plate, membrane or the like, a protein to be tested is bound to the immobilized antibody, a non-bound protein is washed out, a labeled secondary antibody is bound to the conjugate of the antibody and the protein remaining on the plate, then the signal in the labeled antibody is detected. This method is what is called the "sandwich method", and in the case of using an enzyme as a marker, it is a widely used method as "ELISA (enzyme linked immunosorbent assay)". With regard to the two types of antibodies, monoclonal antibodies can be used for both antibodies, or a polyclonal antibody can be used for either of them.

The prognosis in this invention can be carried out by immunostaining, for example tissue or cell staining, immune electron microscopy, or immunoassay, for example competitive immunoassay or noncompetitive immunoassay, and radioimmunoassay (RIA), fluoroimmunoassay (FIA), luminescent immunoassay (LIA), enzyme immunoassay (EIA), ELISA or the like may also be used. B-F separation may be performed, or the assay can be performed without such separation. Preferred are RIA, EIA, FIA, LIA and, further, sandwich assay. The sandwich assay may include simultaneous sandwich assay, forward sandwich assay, reversed sandwich assay and the like.

As the assaying system for protein level in the invention of this application, for example, a protein assaying system such as immunostaining or immune electron microscopy for a tissue sample, a protein assaying system such as EIA, RIA, FIA, LIA or western blotting for blood, body fluid or the like can be carried out.

In the assaying system of EIA, in the case of the competitive method, for example, the antibody is used as an immobilized antibody and a labeled antigen and an unlabeled antigen (a protein or a fragment peptide thereof may be mentioned as the antigen) are used and, in the case of the noncompetitive method, for example the sandwich method, an immobilized antibody or a labeled antibody can be used or the antibody may be directly labeled or an antibody to the antibody may be labeled without immobilization or with immobilization. Examples of the sensitivity increasing method include in the combination with a non-enzyme-labeled primary antibody, a method of using a macromolecular polymer and an enzyme and the primary antibody (application of Envision reagent: Enhanced polymer one-step staining (EPOS)) and, in the combination with a non-enzyme-labeled secondary antibody, for example, the combination of an enzyme and an anti-enzyme antibody complex as in the PAP (peroxidase-antiperoxidase) method or the like, the combination of a biotin-labeled secondary antibody and a biotin-labeled enzyme-avidin complex as in the SABC (avidin-biotinylated peroxidase complex) method or the like, the combination of a biotin-labeled secondary antibody and a biotin-labeled enzyme-streptavidin complex as in the ABC (streptavidin-biotin complex) method, the LSAB (labeled streptavidin-biotin) method or the like, the combination of SABC, a biotin-labeled tyramide and an enzyme-labeled streptavidin as in the CSA (catalyzed signal amplification) method, and a method of using a secondary antibody and an enzyme labeled with a macromolecular polymer and the like.

For the details of such a general technical means, reference may be made to reviews, reference books and the like (e.g., the description in Hiroshi Irie (ed.), "Radioimmunoassay" (published by Kodansha, 1974); Hiroshi Irie (ed.), "Radioimmunoassay; Second Series" (published by Kodansha, 1979); Eiji Ishikawa, et al. (ed.), "Enzyme Immunoassay" (published by Igaku Shoin, 1978); Eiji Ishikawa, et al. (ed.), "Enzyme Immunoassay" (Second Edition) (published by Igaku Shoin, 1982); Eiji Ishikawa, et al. (ed.), "Enzyme Immunoassay" (Third Edition) (published by Igaku Shoin, 1987); H. V. Vunakis et al. (ed.), "Methods in Enzymology", Vol. 70 (Immunochemical Techniques, Part A), Academic Press, New York (1980); J. J. Langone et al. (ed.), "Methods in Enzymology", Vol. 73 (Immunochemical Techniques, Part B), Academic Press, New York (1981); J. J. Langone et al. (ed.), "Methods in Enzymology", Vol. 74 (Immunochemical Techniques, Part C), Academic Press, New York (1981); J. J. Langone et al. (ed.), "Methods in Enzymology", Vol. 84 (Immunochemical Techniques, Part D: Selected Immunoassays), Academic Press, New York (1982); J. J. Langone et al. (ed.), "Methods in Enzymology", Vol. 92 (Immunochemical Techniques, Part E: Monoclonal Antibodies and General Immunoassay Methods), Academic Press, New York (1983); J. J. Langone et al. (ed.), "Methods in Enzymology", Vol. 121 (Immunochemical Techniques, Part I: Hybridoma Technology and Monoclonal Antibodies), Academic Press, New York (1986); J. J. Langone et al. (ed.), "Methods in Enzymology", Vol. 178 (Antibodies, Antigens, and Molecular Mimicry), Academic Press, New York (1989); M. Wilchek et al. (ed.), "Methods in Enzymology", Vol. 184 (Avidin-Biotin Technology), Academic Press, New York (1990); J. J. Langone et al. (ed.), "Methods in Enzymology", Vol. 203 (Molecular Design and Modeling: Concepts and Applications, Part B: Anibodies and Antigens, Nucleic Acids, Polysaccharides, and Drugs), Academic Press, New York (1991) and the like, or the description in the references cited therein).

Incidentally, the method for prognosis provided by this application can be carried out in combination with a method of measuring the protein level and a method of measuring the expression level of genes (e.g., northern blotting method, RT-PCR method, DNA array method or the like) as needed.

Hereunder, the invention of this application will be explained further specifically and in detail by showing Examples, however, this invention is not intended to be limited to the following examples.

**Example 1**

Genomic aberrations in CD5+ and CD5- DLBCL cases were analyzed by DNA array CGH.

1. MATERIALS AND METHORDS

1-1. Patients

DNA samples of 26 cases of *de novo* CD5+ DLBCL and 44 cases of CD5- DLBCL were analyzed. These samples were obtained with informed consent from patients at Aichi Cancer Center and collaborating institutions under the approval of the institutional review boards. All patients had been reported on previously (Yamaguchi, M. et al. Blood, 99: 815-821, 2002;Harada, S. et al. Leukemia, 13: 1441-1447, 1999;Karnan, S. et al. Genes Chromosomes Cancer, 39: 77-81, 2004). The median age was 61 years and 56 years for the CD5+ and CD5- cases, respectively. Among the CD5+ cases, 68% were female, 80% were at an advanced stage (III-IV), 72% had elevated LDH, 24% had a poor performance status and 28% had extranodal site(s) of involvement. In the CD5- cases, 41% were female, 52% had advanced stage (III-IV), 45% had elevated LDH, 10% had a poor performance status and 38% had extranodal site(s) of involvement. All the samples were obtained from tumors at diagnosis before any treatment was given.

1-2. DNA Samples

DNA was extracted using a standard phenol chloroform method from lymphoma samples from a total of 70 DLBCL cases: 26 cases of CD5+ DLBCL and 44 cases of CD5- DLBCL. Normal DNA was prepared from peripheral-blood lymphocytes of healthy male donors.

1-3. Malignant Lymphoma Cell Lines

Cell lines used in this study were Karpas 1718 (splenic lymphoma with villous lymphocytes: SLVL, kindly provided by Dr. A. Karpass, Cambridge University, UK), OCI-LY13.2 (DLBCL, kindly provided by Dr. R.Dalla-Favera, Columbia University) (Tweeddale, M. E. et al. Blood, 69: 1307-1314, 1987) and REC1 (Mantle cell lymphoma cell line, kindly provided by Dr. M. Dyer, Leicester University, UK) (Martinez-Climent, J. A. et al. Blood, 98: 3479-3482, 2001). Cell lines were maintained in RPMI1640 medium supplemented with 10% fetal bovine serum at 37°C in 5% $CO_2$ -95% air.

1-4. Assessment of linearity of Copy Numbers

[0086]    In order to test linearity of array CGH signals with copy number changes, we performed array-hybridization of normal male DNA versus either one of the following cell lines: GM04626; 47XXX, GM01415D; 48XXXX, and GM05009C; 49XXXXX (Kallioniemi, A. et al. Science (Wash. DC), 258: 818-821, 1992;Pinkel, D. et al. Nat. Genet., 23: 41-46, 1998). These cell lines were obtained from the NIGMS Human Genetics Cell Repository Coriell Institute for Medical Research, and were maintained in MEM Eagle-Earle medium supplemented with 2 x essential amino acids, vitamins, and 20% fetal bovine serum at 37°C in 5% $CO_2$ -95% air.

1-5. Selection of BAC/PAC clones for Array CGH

The array consisted of 2,088 BAC and PAC clones (BAC/PACs), covering whole human genome with roughly 1.5-Mb of resolution. BAC clones (BACs) were derived from RP11 and RP13 libraries, and, PAC clones (PACs) were derived from RP1, RP3, RP4 and RP5 libraries. BAC/PAC clones used were selected based on information from NIBC (http://www.ncbi.nlm.nih.gov/) and Ensembl Genome Data Resources (http://www.ensembl.org/). These clones were obtained from the BACPAC Resource Center at the Children's Hospital (Oakland Research Institute, Oakland, CA; http: //bacpac.chori.org/). Clones were sequenced from chromosomes 1 to 22 and X. Within each chromosome, clones were sequenced on the basis of Ensembl Genome Data Resources of Sanger Center Institute, January 2004 version. All the clones used for array CGH were confirmed for their location on chromosomes by FISH analyses. Clone names and their locations on chromosomes are available on request.

1-6. DNA Amplification for Spotting on Slides

10 ng of BAC (or PAC) DNA was used as the template for degenerate oligonucleotide primed PCR (DOP-PCR) (Hakan, T. et al. Genomics, 13: 718-725, 1992) with the 5' amine-modified DOP primer:

5'-CCGACTCGAGNNNNNNATGTGG-3' : SEQ ID NO:1,

and amplified on a TaKaRa PCR Thermal Cycler MP (TaKaRa, Tokyo, Japan) using Ex Taq polymerase (TaKaRa). A 3 min, 94°C denaturation step was followed by 25 cycles of 94°C for 30 s, a 37-72°C linear ramp for 10 min, and 72°C for 1 min, with a final 7 min extension at 72°C.

1-7. DNA Spotting and Quality Control of Glass Slides

DOP-PCR products were ethanol-precipitated and dissolved in distilled water, and then an equal volume of DNA spotting solution DSP0050 (Matsunami, Osaka, Japan) was added (~1 μg of DNA/μl). The resulting DNA samples were robotically spotted by an inkjet technique (NGK, Nagoya, Japan) in duplicate onto CodeLinkTM activated slides (Amersham Biosciences, Piscataway, NJ). In this study, we used only glass slides on which it had been confirmed that all 2,088 clones had been spotted completely and uniformly in duplicate.

1-8. Array Hybridization

The array fabrication and hybridization was performed according to the method described by Pinkel *et al* (Nature (Lond.), 20: 207-211, 1998) and Hodgson *et al.* (Nat Genet., 29: 459-464, 2001). The detailed protocol was kindly provided by Dr. Joe Gray at the University of California San Francisco, CA. One μg of tested (tumor) and referenced (normal) DNAs were digested with *Dpn*II and labeled with Bio prime DNA labeling system (Invitrogen Life Technologies, Inc., Tokyo, Japan) with cyanine3-dUTP and cyanine5-dUTP (Amersham Pharmacia Biotech, Piscataway, NJ), respectively. Unincorporated fluorescent nucleotides were removed by means of Sephadex G- 50 spin columns (Amersham Biosciences). Tested and referenced DNA was mixed together with 50 μg of human Cot-1 DNA (Invitrogen Life Technologies), precipitated, and resuspended in 45 μl of hybridization mixture which consisted of 50% formamide, 10% dextran sulfate, 2 x SSC, 4% SDS and 10 μg/μl yeast tRNA (Invitrogen Life Technologies). The hybridization solution was heated to 73°C for 5 min to denature the DNA, and then incubated for 45 min at 37°C to block repetitive sequences. The glass slides spotted with DNAs were denatured in 70% formamide/2 x SSC at 73°C for 4 min, then dehydrated in cold 70%, 85% and 100% ethanol for 5 min each and air-dried. Hybridization was performed for 66 hours in a container on a slowly rocking table with 200 μl of 50% formamide/2 x SSC, followed by post-hybridization washings in 50% formamide/2 x SSC for 15 min at 50°C, 2 x SSC/0.1% SDS for 30 min at 50°C, and PN buffer (0.1M NaH2PO4, 0.1M Na2HPO4 to attain pH 8, and 0.1% NP-40) for 15 min at room temperature. The glass slides were then rinsed in 2 x SSC at room temperature, and finally dehydrated in 70%, 85%, and 100% ethanol at room temperature for 2 min each and air-dried. Scanning of slides was carried out with an Agilent Micro Array Scanner (Agilent Technologies, Palo Alto, CA) and the acquired array images were analyzed using Genepix Pro 4.1 (Axon Instruments, Inc., Foster City, CA). DNA spots were automatically segmented, and the local background was subtracted, and intensities of the signals were obtained. Subsequently, ratios of the signal intensity of two dyes (Cy3 intensity/Cy5 intensity) were calculated for each spot, converted into log2 ratios on an Excel sheet in the order of chromosomal positions, and then normalized.

1-9. Normalization of Data Set

Normalization of the 'log2 ratio' of each sample was performed by computing medium log2 ratio value for all the clones. In this study, 'log2 ratio' represents the mean log2 ratio of duplicate spots for each clone. We then selected clones with a log2 ratio more than "the median + SD x A" or less than "the median - SD x A". "A" was visually defined as the normal region by referring to the log2 ratio plots of all clones in each of the experiments. "A" ranged approximately from 0.5 to 1.0. Next, the mean log2 ratio value of the selected clones was computed, and was defined as "X". Finally, we obtained the "Y" values by subtracting the corresponding "X" values from log2 ratio of each clone. In this study, each log2 ratio was analyzed on the basis of the "Y" values. We visually selected the clones, computed the SD for each experiment and confirmed that the SD did not exceed 0.15.

1-10. FISH Analysis

Metaphase chromosomes were prepared from normal male lymphocytes and cell lines. Approximately 200 ng of BAC/PACs were labeled using a nick translation kit (Vysis Inc., Downers Grove, IL) with Spectrum Green-dUTP or Spectrum Red-dUTP (Vysis). Labeled DNA and 10 $\mu$g of human Cot-1 DNA were co-precipitated in ethanol, dissolved in 3 $\mu$l of distilled water and 7 $\mu$l of hybridization buffer consisting of 50% formamide, 20% dextran sulfate, and 2 x SSC. Interphase chromosome slides of cell lines were prepared according to the standard method. Hybridization was performed for 12-24 hr at 37°C on the slides that had been denatured at 73°C in 70 % formamide/2 x SSC. The slides were then washed in 0.4 x SSC/0.3 % NP-40 at 75°C and 2 x SSC/0.1 % NP-40 at room temperature. After the slides had been dried, chromosomes were counterstained with DAPI (4,6-diamidino-2-phenylindole)-II (Vysis). Chromosomes were identified on the basis of the banding pattern of the DAPI-staining. Digital image analysis was performed with a BX-60-RF microscope (Olympus) and IP Lab Scientific Imaging Software (Scanalytics Inc., Fairfax, VA).

1-11. Statistical Analysis

To analyze genomic regions for statistical difference between the two patient groups, the data-set was constructed as follows. Genomic alterations were defined by log2 ratio thresholds of +0.2 for copy number gain, and -0.2 for copy number loss. Gained clones (log2 ratio>+0.2) were input as "1" versus no-gained clones (log2 ratio <0.2) as "0" in an Excel template for each case. Similarly, lost clones were input as "1" versus no-lost clones as "0" in another Excel template for each case. Cases showing genomic gain or loss were counted with Excel for each single clone (1,966 clones in total) in the CD5+ group or CD5- group. Data analyses were then carried out for the following purposes: 1) comparison of frequencies of gain or loss of each single clone between the CD5+ and CD5-groups (1,966 tests each for gain and loss, 3,932 tests in total), 2) comparison of overall survival between cases showing gain or loss of a single clone and cases without respective gain or loss (1,966 tests for each gain and loss with or without CD5 expression, in total 7,864 tests maximum). A Fisher's exact test for probability was applied to the former analysis and a logrank test comparing survival curves between the two groups was applied to the latter. *P*-value for screening of candidate clones for each analysis was p < 0.05. When a candidate clone was identified, the clone's continuity with the following clones was evaluated. In the case where the nth clone and succeeding *k* clones (K$\geqq$0) were revealed to be candidate clones, the p-value for continual association was calculated as:

$$\prod_{i=n}^{n+k} P_i \qquad (1)$$

under the assumption that each clone is independent throughout whole genome. As we applied multiple tests (11,796 tests maximum), the conventional Bonferroni procedure was applied to define the alfa-error for the final conclusion. Therefore, we defined a p-value less than 0.05/12,000 (= 4.2 x $10^{-6}$) as statistically significant (Wright, SP. Biometrics, 48: 1005-1013, 1992). All the statistical analyses were conducted with a statistical package STATA ver.8 (College Station, TX).

2. Results

2-1. Quality of Array CGH

To validate our CGH array, we first performed hybridizations of normal male DNA versus normal male DNA on six different occasions. The average of the SE per clone, representing the average clone variability in six normal samples, was 3% in this set of controls. Forty two spotted clones were found to show less than 10% of the mean fluorescence intensity of all the clones. Sixty-two clones were with the most extreme average test over reference ratio deviations from 1.0, and 18 clones were with the largest SD in this set of normal controls. Thus, 122 clones were excluded from further analyses. Log2 ratios of fluorescence of each spot (1,966 clones in duplicate) were within the range of +0.2 to -0.2. We therefore considered log2 ratio beyond this range as significant. Linearity between copy number and values of log2 ratio was confirmed by the use of human fibroblast cell lines that had different copy number of X chromosomes (see Example 2). Next, we performed array CGH for several malignant lymphoma cell lines to see if our system could detect gains and losses. Fig. 1A shows a whole genomic profile of REC1 cell line that revealed genomic aberrations at multiple loci including gain of 13q31.3 (BAC, RP11-430K10) and loss of 4q32.1 (BAC, RP11-154F14). In accordance with array CGH data, these gains and losses were subsequently also detected by FISH analysis using the corresponding BACs (Fig. 1*B*). Fig. 2 shows individual array CGH profiles of chromosome 16 (Fig. 2*A*) and chromosome 8p (Fig. 2*C*) of Karpass 1718 cell line. The profile showed a single peak of gain of 16p13.13 (BAC, RP11-24M12) (Fig. 2*A*) and the breakpoint of the 8p21 between BAC, RP11-353K12 and BAC, RP11-369E15. These results were also subsequently confirmed by FISH analyses (Fig. 2*B* and 2*D*). These findings demonstrated the accuracy and high-resolution of our array CGH.

2-2. Genomic Profiles and Data Analysis for DLBCL cases

We next performed array CGH analysis to compare genomic alterations between CD5+ and CD5- DLBCL cases. All the clones on chromosome X (57 clones) were separately analyzed because of sex mismatching. A total of 70 DLBCL cases were enrolled. Four cases (one case of CD5+ and three cases of CD5- DLBCL) did not show any genomic aberrations. Thus, 25 cases of CD5+ DLBCL and 41 cases of CD5- DLBCL were subjected to the data analysis. The average sizes of genomic gains and losses of CD5+ and CD5- DLBCL are shown in Table 1. Their percent coverage in total genome is also summarized in the table. CD5+ group contained the larger fraction of copy number gain than CD5- group on an average, while the former contained smaller fraction of copy number loss than the latter.
Fig. 3 shows whole genomic profiles of two representative CD5+ (Fig. 3*A* and 3*B*) samples and one CD5- (Fig. 3*C*) sample. Copy number changes were easily detectable at a high resolution genome-wide. Regions showing low-level amplifications (defined as log2 ratio +0.2 to +1.0) as well as regions suggestive of a heterozygous deletion (defined as log2 ratio -1.0 to -0.2) were also discernible.
In this invention, the term '*common regions* of gain or loss' was defined as i) the continuously ordered at least continuous three clones (more than 3-5Mb of resolution level) that showed gain or loss in more than 20% of cases, or, ii) if less than three, clone(s) showing high copy number gains (defined as log2 ratio>+1.0) or homozygous loss (defined as log2 ratio<-1.0) (gain of 2p16.1, loss of 3p14 and loss of 9p21). *'Minimum common region'* was defined, in the '*common region',* as the region that was shared by the highest number of cases. Most frequent BAC/PAC clones in the *minimum common regions,* and representative genes (known candidate oncogenes for malignant lymphoma and tumor suppressor genes) (Monni, O. et al. Blood, 87: 5269-5278, 1996 ; Rao, P. H. et al. Blood, 92: 234-240, 1998;Wright, G. et al. Proc. Natl. Acad. Sci. USA, 100: 9991-9996. 2003) contained therein are listed in Tables 2 and 3.
*Common regions of gain in CD5+ group* (25 cases) were 1q21.2-q32.3, 1q42.2-q42.3, 2p16.1, 3, 5p13.2-p13.1, 6p25.3-p22.3, 7p22.2-q31.1, 8q24.13-q24.22, 11q22.1-q25, 12, 13q21.1-q34, 16p13.3-q21, 17q23.2-q24.3, 18, 19p13.13-q13.43, and X, and, *common region of loss in CD5+ group* were 1p36.32-p36.23, 1p36.21-p36.13, 1p35.1-p34.3, 1q43-q44, 3p14.2, 6q14.1-q27, 8p23.3-p21.2, 9p21, 15q13.1-q14, and 17p13.3-p11.2.
*Common regions of gain in CDS- group* (41 cases) were 1q21.2-q31.1, 1q32.1-q32.2, 3p25.2-q29, 5p13.2-p13.1, 5p14.1-p13.2, 6p25.3-p12.3, 7, 8q24.13-q24.21, *9p24.2-p13.2,* 11q23.2-q24.2, 12q13.2-q21.2, 16p13.3, 18, and X, and, *common region of loss in CD5- group* were 1p36.32-p36.23, 3p14.2, 6q12-q25.2, 6q27, 9p21, 15q15.2-q21.1, and 17p13.3-p11.2.
Regions of gain observed in more than 20% of cases of *both* the CD5+ and CD5- groups were 1q21.2-q31.1, 1q32.1-q32.2, 3p25.2-q29, 5p13.2-p13.1, 6p25.3-p21.1, 7p22.2-q31.1, 8q24.13-q24.21, 11q23.2-q24.3, 12q13.2-q21.2, 16p13.3, 18, and X, and, regions of loss observed in more than 20% of cases of *both* the CD5+ and CD5- groups were 1p36.32-p36.31, 3p14.2, 6q14.1-q25.2, 6q27, 9p21, and 17p 13.3-p 11.2.
Among above *common region* of either CD5+ or CD5- groups, some cases with gain of 2p16.1 *(REL* gene locus, BAC, RP11-17D23), loss of 3p14.2 *(FHIT* gene locus), and loss of 9p21.1 *(p16INK4a* gene locus) showed genomic aberrations by single clone or continuous two clones. Three cases out of 13 cases of 2p 16.1 gain showed such the gain. Two cases out of three cases of 2p 16.1 gain in DLBCL showed extremely high-level amplification (log2 ratio>+1.5) with only continuous two clones (BACs, RP11-17D23 and RP11-511I11). Loss of 3p14.2 in DLBCL had not been reported previously.

Eighteen out of a total of 66 cases (28%) showed loss at 3p14.2. Among clones contained in 3p14, BAC, RP11-48E21 *(FHIT* locus) was singly lost in 13 out of 18 cases (Seven cases of CD5+ and 11 cases of CD5- DLBCL), with no surrounding BACs showing obvious copy number losses (Fig. 4A). Among singly lost 13 clones, two cases out of 13 cases were suggesting homozygous loss (log2 ratio<-1.0). The *FHIT* locus was therefore considered to be a minimum common region, and was also deleted in a cell line OCI-LY13.2 established from a patient of aggressive malignant lymphoma (Karnan, S. et al. Genes Chromosomes Cancer, 39: 77-81, 2004). FISH analyses were consistent with the array CGH data (Fig. 4B and C). Similarly, a BAC clone RP11-14912 (including *p161NK4a* locus) was singly lost in 9 out of 32 cases exhibiting loss of 9p21 in a total of 66 DLBCL cases (2 cases out of 9 cases were suggesting homozygous loss), thus considered as a *minimum common region.*

Finally, X chromosomes for male patients only (CD5+ group: 10 cases, CD5- group: 25 cases) were analyzed. Two out of 10 cases of CD5+ group and 3 out of 15 cases of CD5- group showed low-grade copy number gains (+0.2<log2 ratio<+1.0) throughout whole X chromosome without any high-grade amplification. Low-grade losses (-1<log2 ratio<-0.2) were found at Xq21 in two cases in CD5+ group.

2-3. Genomic Copy Number Changes Characteristic of CD5+ DLBCL

Next, frequency of gains and losses of clones between the CD5+ and CD5- groups was compared. Screening on a single-clone basis for candidate clones revealed that forty eight clones were more frequently (p<0.05) gained or lost in the CD5+ group than the CD5- group. Among these 48 clones, six out of six (6/6) clones at 10p15.3-p14, three out of three (3/3) clones at 12p12, three out of five (3/5) clones at 16p12, and nine out of nine (9/9) clones at 19q13.33-q13.4 were continuously ordered (according to whole genome mapping position by Ensembl Genome Data Resources of Sanger Center Institute, January 2004 version). Two out of five (2 / 5) clones at 16p 12 and remaining 25 out of 48 clones showed P<0.05 singly, i.e. with no neighboring clones showing difference between the CD5+ and the CD5- groups.

Twenty clones were found lost significantly more frequently in the CD5+ group than the CD5- group. Among these, three out of nine clones (3/9) at 1q43, six out of nine clones (6/9) at 1q43-q44 two out of seven clones (2/7) at 8p23, and five out of seven clones (5/7) at 8p23 were continuous, and remaining four clones of 20 showed P<0.05 singly with no neighboring clones showing difference between CD5+ and CD5- groups.

Since multiple comparisons were made, clones screened by a single-clone basis as above were subsequently subjected to multiple comparison corrections, so as to find clones statistically relevant in terms of difference of frequency between the CD5+ and the CD5- groups. In the case where the nth clone and succeeding k clones (k≧0) were revealed to be candidate clones through screening, the p-value for continual association was calculated by the formula (1) in 1-11, under the assumption that each clone is independent. As we applied multiple tests (maximum 11,796 tests), the conventional Bonferroni procedure was applied to define the alfa-error for the final conclusion. Therefore, we defined p-value less than 0.05/12,000 (= 4.2 x $10^{-6}$) as statistically significant. Since value from the formula (1) of 10p15.3-10p14 gain, 19q13.33-q13.43 gain, 1q43-q44 loss, and 8p23.2-p23.1 loss of CD5+ DLBCL were <4.2 x $10^{-6}$, these regions were considered as characteristic of the CD5+ group. But value from the formula (1) of other clones in CD5+ DLBCL did not show significance in this criterion.

Ten gained clones and six lost clones showed a difference (p<0.05) in frequency of cases between the CD5+ and CD5- groups, but these clones were found of no significance after multiple comparison corrections being performed.

Thus, gain of 10p15.3-p14 and 19q13.33-q13.43, and, losses of 1q43-q44 and 8p23.2-p23.1 are characteristic of CD5+ DLBCL. No gains and losses were found characteristic of CD5- DLBCL. Regions characteristic of CD5+ DLBCL and BAC clones contained therein are listed in Table 4 (gains) and Table 5 (losses). Three representative examples of individual genomic profilings of chromosome 10, chromosome 19, chromosome 1q, and chromosome 8 of CD5+ DLBCL cases are shown in Fig. 5A, Fig. 5B, Fig.6, and Fig. 6*B*, respectively.

2-4. Genomic Copy Number Changes Affecting Prognosis of DLBCL cases

The inventors next analyzed probabilities of survival of the cases that were stratified according to the presence or absence of one of the specific genomic gains or losses, by the use of Kaplan-Meier method and logrank test. All the clones that showed aberrations of copy number changes (gain/loss) in either the CD5+ or the CD5- group were subjected to screening. Subsequently, multiple comparison corrections were performed for the clones that showed p<0.05 by the screening logrank test (see materials and methods).

In CD5+ group, 252 clones (98 gains and 154 losses) showed p<0.05 in regard to prognosis by logrank test on a single-clone basis. Of these 252 clones, 18 clones were continuously ordered at 1p36.21-p34.3 and were found, by multiple comparison corrections, to have deleterious effects on survival (*p*<4.2 x $10^{-6}$). Forty four out of 252 clones fell in 65 clones analyzed for 13q. 29 out of 44 clones (29/44) at 13q21.1-q31.3 and 15 out of 44 clones (15/44) at 13q31.3-q34 were, respectively, continuously ordered clones and were found by multiple comparison corrections to be linked to poor prognosis (p< 4.2 x 10$^{-6}$). The remaining 190 clones fell short of statistical significance (p>4.2 x 10$^{-6}$).

In CD5- group, 252 clones (197 gains and 55 losses) showed p<0.05 on a single-clone basis in regard to prognosis. Of these 252 clones, 34 clones fell in 38 clones analyzed for 5p. Among the 34 clones, 19 out of 34 clones at 5p15.33-p14.2 and five out of 34 clones at 5p13.2-p12 were, respectively, continuously ordered clones and were found, by multiple comparison corrections, to have favorable impacts on survival ($p$<4.2 x 10$^{-6}$). The remaining 218 clones were not statistically significant ($p$>4.2 x 10$^{-6}$).

Survival curves for CD5+ and CD5- cases according to the presence or absence of gain of 13q21.33-q31.1, loss of 1p36.21-p36.13, and gain of 5p 15.33-p 14.2, respectively, are presented in Fig. 7.

As shown in Table 6, CD5+ DLBCL cases with gain of 13q21.1-q34 showed significantly inferior survival than CD5+ cases without such respective gain. Similarly, CD5+ DLBCL cases with loss of 1p36.21-p34.3 showed significantly inferior survival than CD5+ cases without such respective loss (Table 7). In contrast, loss at the corresponding region did not affect survival of CD5- DLBCL cases. Conversely, CD5- DLBCL cases with a gain of 5p showed superior overall survival to those without such gain, but a gain of 5p had no impact on survival of CD5+ DLBCL cases (data not shown).

Table 1

Table 1 *Copy number alteration of DLBCL cases*

|  |  | All cases n=66 | CD5+DLBCL n=25 | CD5- DLBCL n=41 |
|---|---|---|---|---|
| Copy number gain |  |  |  |  |
|  | Average genome size (Mb) *a* | 335.6 | 370.9 | 311.1 |
|  | Average % of the genome | 11.8 | 13.4 | 10.9 |
| Copy number loss |  |  |  |  |
|  | Average genome size (Mb) | 148.2 | 110.5 | 174.4 |
|  | Average % of the genome | 5.2 | 3.8 | 6.8 |
| Total genome covered (Mb, +X chromosome) |  | 2,988 |  |  |
| Total genome covered (Mb, -X chromosome) |  | 2,834 |  |  |
|  | Number of clones (+X chromosome) . | 1,966 |  |  |
|  | Number of clones (-X chromosome) | 1.907 |  |  |
|  | Average distance between clones (Mb) | 1.5 |  |  |
|  | Maximun distance between clones (Mb) | 25.1 |  |  |

Size of a genomic alteration was defined as the sum of the affected clones, each representing one -half of the distance between its own center and that of its two neighbouring clones (see ref 29).

Table 2

Table 2 *Most frequency gained clones of each common region in each group*

|  |  |  |  | CDS+ (n=25) | CD5- (n=41) | All (n=66) |
|---|---|---|---|---|---|---|
| Chromosome | Clone name | Cytogenetic position | Genes*c* | %*d* | %*d* | %*d* |
| Chr.1 | RP11-367J7*a,b* | 1q23.1 | *HDGF* | 36 | 29.3 | 31.8 |

Table continued

| Chromosome | Clone name | Cytogenetic position | Genes[c] | CDS+ (n=25) %[d] | CD5- (n=41) %[d] | All (n=66) %[d] |
|---|---|---|---|---|---|---|
| | RPII-263K19 | 1q22 | *MUC1* | 20 | 26.8 | 24.2 |
| | RP11-2317[b] | 1q31.1 | *MDM4* | 24 | 24.4 | 25.8 |
| | RPS-956018[a] | 1q42.2 | *PGBD5* | 20 | 22.7 | 21.2 |
| Chr.2 | RP11-17D23[a] | 2p16.1 | *REL* | 24 | 17.1 | 19.7 |
| Chr.3 | RP11-33905 | 3p22.3 | *CCR4* | 40 | 24.4 | 30.3 |
| | RP11-56K23 | 3p13 | *FOXP1* | 36 | 24.4 | 28.7 |
| | RP11-861A13[b] | 3q12.1 | *CD47* | 48 | 24.4 | 33.3 |
| | RP11-362K14 | 3q26.2 | *EVI-1* | 44 | 31.7 | 36.3 |
| | RP11-211G3[a] | 3q27.3 | *BCL6* | 56 | 36.6 | 43.9 |
| Chr.5 | RP11-317123[a,b] | 5p13.2 | - | 20 | 22.0 | 21.2 |
| Chr.6 | RP11-233K4[b] | 6p25.3 | *MUM1* | 24 | 29.3 | 27.3 |
| | RP3-431A14[a] | 6p21.31 | *p21* | 29 | 26.6 | 33.3 |
| Chr.7 | RJ11-498D18 | 7p22.2 | *CARD18* | 24 | 39.0 | 33.3 |
| | RP11-221B19 | 7p21.2 | *ETV1* | 20 | 24.4 | 22.7 |
| | RP11-240H8 | 7p15.3 | *IL6* | 24 | 26.8 | 25.8 |
| | RP4-777023 | 7p14.3 | *CARD4* | 20 | 34.1 | 28.8 |
| | RP11-911H5[a,b] | 7q21.2 | *CDK6* | 24 | 41.5 | 34.8 |
| Chr.8 | RPI-80K22[a,b] | 8q24.2 | *MYC* | 20 | 24.4 | 22.7 |
| Chr.9 | RP11-39K24[b] | 9p24.1 | *JAK2* | 12 | 26.6 | 21.2 |
| | RP11-8N6 | 9p13.2 | *PAX5* | 8 | 24.4 | 18.2 |
| Chr.11 | RP11-144G7 | 11q22.3 | *DDX10* | 36 | 12.2 | 21.2 |
| | RPII-770K18 | 11q23.3 | *DDX6* | 32 | 22.7 | 25.8 |
| | RP11-758H14[b] | 11q23.3 | *MLL* | 40 | 31.7 | 34.8 |
| | RP11-1007G5[a] | 11q24.3 | *ETSI* | 56 | 29.3 | 39.3 |
| Chr.12 | RP11-100C20[a] | 12p12.1 | *BCATI* | 44 | 17.1 | 25.8 |
| | RP11-181L23[b] | 12q13.3 | *GLI* | 32 | 24.4 | 21.2 |
| | RP11-571M6[b] | 12q14.1 | *CDK4* | 32 | 24.4 | 21.2 |
| | RP11-450G15[b] | 12q 15 | *MDM2* | 32 | 24.4 | 27.2 |
| Chr.13 | RP11-335P18[a] | 13q21.32 | - | 32 | 12.2 | 19.7 |
| | RPII-121J7 | 13q31.3 | *C13orf25* | 24 | 9.7 | 16.7 |
| | RP11461N23° | 13q32.3 | *EB12* | 32 | 17.1 | 21.2 |
| Chr.16 | RPII-473M20b | 16p13.3 | *NK4* | 36 | 65.9 | 56.1 |
| | RP11-548B6[a] | 16p12.1 | *PKC beta* | 28 | 7.3 | 15.1 |
| Chr.17 | RPS-371B4[a] | 17q23.2 | - | 20 | 4.9 | 10.6 |
| Chr.18 | RP11-380M21 | 18q21.1 | *SMAD2* | 26 | 39.4 | 39.4 |
| | RP11-4G6 | 18q21.1 | *MALT1* | 36 | 39.0 | 37.9 |
| | RP11-28F1[b] | 18q21.1 | *BCL2* | 36 | 41.5 | 39.4 |
| | RP11-676J15[a] | 18q22.3 | *NET01* | 44 | 38.8 | 47.0 |
| Chr.19 | RP11-50I11[a] | 19q13.41 | *BAX* | 56 | 14.6 | 30.3 |
| | RP11-45K21[a] | 19q13.43 | *PEG3* | 56 | 26.8 | 37.9 |

[a] Most frequently gained clones in CDS+ DLBCL.

[b] Most fequently gained clones in CD5' DLBCL.

[c] Genes contained in clones.

[d] % of cases with copy number gain.

Table 3

Table 3 *Most frequently lost clones of each common region in each group*

| Chromosome | Clone name | Cytogenetic position | Genes[c] | CD5[+] (n=25) %[d] | CD5- (n=41) %[d] | All (n=66) %[d] |
|---|---|---|---|---|---|---|
| Chr.1 | RP11-37J18[a,b] | 1p36.32 | - | 40 | 27 | 31.8 |
| | RP11-473A10[a] | 1p36.13 | - | 24 | 22 | 22.7 |
| | RPS-1125N11[a] | 1p35.2 | - | 28 | 22 | 24.2 |
| | RP11-439E19[a] | 1q44 | NM 016009 | 24 | 0 | 9.1 |
| Chr.3 | RP11-48E21[a,b] | 3p14.2 | FHIT | 28 | 26.8 | 28.7 |
| Chr.6 | RP11-329G3[a,b] | 6q21 | BLIMPI | 44 | 36.6 | 39.4 |
| | RP11-421D16[b] | 6q27 | AF6 | 24 | 19.5 | 21.2 |
| Chr.8 | RP11-240A17[a] | 8p23.3 | - | 36 | 7.3 | 18.1 |
| Chr.9 | RP11-14912[a,b] | 9p21.3 | $p16^{INK4a}$ | 40 | 31.7 | 34.8 |
| Chr.15 | RP11-2C7[a] | 15q13.2 | - | 24 | 17.1 | 19.7 |
| | RP11-164J23[b] | 15q21.1 | - | 16 | 29.3 | 24.2 |
| Chr.17 | RP11-199F11[a] | 17p13.1 | p53 | 36 | 26.8 | 30.3 |
| | RP11-187D20[b] | 17p12 | MAP2K4 | 20 | 39 | 30.3 |

[a] Most frequently gained clones in CD5[+] DLBCL.
[b] Most fequently gained clones in CD5[-] DLBCL.
[c] Genes contained in clones.
[d] % of cases with copy number gain.

Table 4

Table 4 *List of BAC clones showing chracteristic gains of CD5[+] DLBCL*

| Clone name[a] | Chromsome band | Mega base[b] | Gene | CD5[+] (n=25) %[c] | CD5[-] (n=41 ) %[c] | All (n=66) %[c] | Fisher's P[d] |
|---|---|---|---|---|---|---|---|
| RP11-362D13 | 10p15.3 | 2.2 | | 16 | 0 | 6.1 | 0.0176 |
| RP11-453F1 | 10p15.2 | 3.3 | | 16 | 0 | 6.1 | 0.0176 |
| RP11-154P11 | 10p15.1 | 4.3 | | 16 | 0 | 6.1 | 0.0176 |
| RP11-445P17 | 10p15.1 | 5.1 | AKRIC4 | 16 | 0 | 6.1 | 0.0176 |
| RP11-563J2 | 10p15.1 | 6.3 | | 16 | 0 | 6.1 | 0.0176 |
| RP11-379F12 | 10p14 | 8.1 | GATA3 | 16 | 0 | 6.1 | 0.0176 |
| RP11-124P12 | 19q13.33 | 52.2 | | 40 | 17.1 | 25.8 | 0.0476 |
| RP11-3N16 | 19q13.41 | 53.3 | LIGI | 48 | 29.3 | 28.8 | 0.0113 |
| RP11-50I11 | 19q13.41 | 54.5 | BAX. CD37 | 56 | 14.6 | 30.3 | 0.0007 |

Table continued

| Clone name[a] | Chromsome band | Mega base[b] | Gene | CD5+ (n=25) %[c] | CD5- (n=41 ) %[c] | All (n=66) %[c] | Fisher's P[d] |
|---|---|---|---|---|---|---|---|
| RP11-25A12 | 19q13.42 | 55.4 | IL411 | 48 | 19.5 | 31.8 | 0.0051 |
| RP11-256B9 | 19q13.43 | 57.5 | ZNF137 | 44 | 14.6 | 25.8 | 0.0046 |
| RP11-158G19 | 19q13.43 | 59.1 | MYDM | 44 | 14.6 | 25.8 | 0.0046 |
| RP11-705C4 | 19q13.43 | 60.7 | IL11 | 48 | 19.5 | 30.3 | 0.0259 |
| RPII-45K21 | 19q13.43 | 61.8 | PEG3 | 56 | 26.8 | 37.9 | 0.0216 |
| RP11-43B2 | 19q13.43 | 62.6 | | 56 | 17.1 | 31.8 | 0.0022 |
| RP11-420P11 | 19q13.43 | 64.1 | | 44 | 22.0 | 30.3 | NS (0.0964) |

[a] List of BAC/PAC clones from p telomere to q telomere for each chromosome number.

[b] Based on Ensembl genome mapping position (http://www. Ensembl.org).

[c] % of cases with copy number gain.

[d] P values of Fisher's exact test in frequency of cases between CD5+ and CD5- DLBCL.

[e] Not significant.

Table 5

Table 5 *List of BAC clones showing chracteristic loss of CD5+ DLBCL group and statistic analysis*

| Clone name[a] | Chromsome band | Mega base[b] | Gene | CD5+ (n=25) %[c] | CDS- (n=41) %[c] | All (n=66) %[c] | Fisher's P[d] |
|---|---|---|---|---|---|---|---|
| RP11-435F13 | 1q44 | 238.1 | RGS7 | 24 | 0 | 9.1 | 0.0019 |
| RP11-269F20 | 1q44 | 240.6 | AKT3 | 20 | 0 | 7.6 | 0.0059 |
| RP11-424N15 | 1q44 | 241.5 | ADSS | 20 | 0 | 7.6 | 0.0059 |
| RP11-74P14 | 1q44 | 242.9 | Q96QW3 | 20 | 0 | 7.6 | 0.0059 |
| RP11-439E19 | 1q44 | 243.7 | NM 016001 | 24 | 0 | 9.1 | 0.0019 |
| RP11-152M6 | 1q44 | 243.9 | | 20 | 0 | 7.6 | 0.0059 |
| RP11-18D5 | 8p23.3 | 0.3 | NM 181648 | 28 | 4.9 | 13.6 | 0.0214 |
| RP11-240A17 | 8p23.3 | 1.2 | | 36 | 7.3 | 18.1 | 0.0066 |
| RP11-82K8 | 8p23.3 | 2.1 | | 36 | 14.6 | 22.7 | NS[e] (0.0685) |
| RP11-29A2 | 8p23.2 | 5.1 | | 32 | 9.8 | 18.2 | 0.0449 |
| RP11-728L1 | 8p23.2 | 5.6 | | 32 | 9.8 | 18.2 | 0.0449 |
| RP11-18L2 | 8p23.1 | 8.6 | MASL1 | 24 | 2.4 | 10.6 | 0.0099 |
| RP11-241I4 | 8p23.1 | 10.0 | | 32 | 9.8 | 18.2 | 0.0449 |
| RP11-254E10 | 8p23.1 | 11.2 | | 32 | 7.3 | 16.7 | 0.0154 |

[a] List of BAC/PAC clones from p telomere to q telomere for each chromosome number.

[b] Based on Ensembl genome mapping position (http://www. Ensembl.org).

[c] % of cases with copy number loss..

[d] P values of Fisher's exact test in frequency of cases between CD5+ and CD5- DLBCL.

[e] Not significant.

Table 6

Table 6 *List of BAC clones associated with inferior prognosis of gains in the CD5 DLBCL group and statistic analysis*

| Clone name[a] | Chromsome band | Mega base[b] | Gene[c] | CD5+ (n=25) | | CD5- (n=41) | |
|---|---|---|---|---|---|---|---|
| | | | | %[d] | log-rank P[e] | %[d] | log-rank P[e] |
| RP11-174I10 | 13q14.2 | 46.8 | *RBI* | 16 | 0.01997 | 19.5 | NS |
| RP11-103J18 | 13q14.2 | 47.6 | GPR38 | 12 | 0.02032 | 22.0 | NS |
| RP11-364I19 | 13q14.2 | 49.5 | RFP2 | 12 | 0.02032 | 7.3 | NS |
| RP11-24B19 | 13q14.3 | 50.8 | DDX26 | 20 | 0.00035 | 14.6 | NS |
| RP11-456B18 | 13q21.1 | 53.3 | | 16 | 0.00097 | 7.3 | NS |
| RP11-505I19 | 13q21.1 | 54.8 | | 16 | 0.00097 | 7.3 | NS |
| RP11-334O13 | 13q21.1 | 57.2 | | 20 | 0.00009 | 9.6 | 0.0305 |
| RP11-430I3 | 13q21.2 | 58.5 | DIAPH3 | 24 | 0.00019 | 9.6 | 0.0305 |
| RP11-459E2 | 13q21.2 | 58.8 | TDRD3 | 24 | 0.00117 | 14.6 | NS |
| RP11-168G22 | 13q21.31 | 60.9 | bA539I23.1 | 24 | 0.00631 | 12.2 | NS |
| RP11-468L10 | 13q21.31 | 62.8 | | 24 | 0.00631 | 12.2 | NS |
| RP11-335P18 | 13q21.32 | 64.9 | | 32 | 0.02655 | 12.2 | NS |
| RP11-370A2 | 13q21.33 | 67.1 | | 28 | 0.00477 | 12.2 | NS |
| RP11-280J7 | 13q21.33 | 68.9 | | 24 | 0.00631 | 12.2 | NS |
| RP11-11C5 | 13q21.33 | 70.9 | | 24 | 0.00631 | 9.6 | 0.0305 |
| RP11-459P23 | 13q22.1 | 72.6 | bA459P23.1 | 24 | 0.00631 | 12.2 | NS |
| RP11-182M20 | 13q22.2 | 73.7 | 5A182M20.2 | 24 | 0.00631 | 9.6 | 0.0305 |
| RPII-388E20 | 13q22.3 | 76.6 | | 24 | 0.00631 | 9.6 | NS |
| RPII-263K14 | 13q3 1.1 | 78.5 | | 24 | 0.00631 | 9.6 | NS |
| RPII-193G17 7 | 13q31.1 | 80 | PTMA | 20 | 0.03369 | 14.6 | NS |
| RPII-115N13 | 13q31. | 80.9 | | 20 | 0.03369 | 12.2 | NS |
| RPII-39SN17 | 13q31.1 | 83.5 | | 20 | 0.03369 | 17.1 | NS |
| RP11-447N10 | 13q31.1 | 84.6 | | 20 | 0.03369 | 14.6 | NS |
| RP11-29C8 | 13q31.1 | 83.9 | | 24 | 0.03053 | 19.5 | NS |
| RPII-506FI7 | 13q31.1 | 84.9 | | 20 | 0.03369 | 14.6 | NS |
| RP11-456P14 | 13q31.2 | 86.1 | Y918 | 24 | 0.00631 | 12.2 | NS |
| RP11-360A9 | 13q31.2 | 86.8 | bA360A9.1 | 20 | 0.03369 | 12.2 | NS |
| RP11-86C3 | 13q31.3 | 87.9 | | 24 | 0.00631 | 7.3 | NS |
| RP11-158A8 | 13q31.3 | 88.7 | | 28 | NS | 9.8 | NS |
| RP11-430K10 | 13q31.3 | 89.4 | GPC5 | 28 | NS | 17.1 | NS |
| RP11-121J7 | 13q31.3 | 89.9 | C13orf25 | 24 | 0.00631 | 9.7 | NS |
| RPII-71K7 | 13q31.3 | 91.5 | GPC6 | 20 | 0.00009 | 9.7 | 0.0305 |
| RP11-342E2 | 13q31.3 | 92.5 | GPC6 | 20 | 0.00009 | 9.7 | NS |
| RP11-124B17 | 13q32.1 | 92.8 | DCT | 20 | 0.00009 | 12.2 | NS |
| RP11-199B17 | 13q32.1 | 95.4 | | 20 | 0.00009 | 17.1 | NS |
| RP11-461N23 | 13q32.3 | 97.5 | EBI2 | 32 | 0.00023 | 17.1 | NS |
| RP11-484I6 | 13q33.2 | 101.1 | ERCC5 | 20 | 0.00009 | 17.1 | NS |
| RP11-317H7 | 13q33.1 | 102.3 | | 16 | 0.00009 | 9.7 | NS |
| RP11-166E2 | 13q33.2 | 104.5 | G72 | 20 | 0.00009 | 7.3 | NS |
| RP11-272L14 | 13q33.2-3 | 104.7 | EFNB2 | 16 | 0.00147 | 9.7 | NS |
| RP11-346C4 | 13q33.3 | 106.1 | ba232k22.1 | 24 | 0.00631 | 9.7 | NS |
| RP11-313L9 | 13q34 | 108.1 | IRS2 | 28 | 0.00155 | 14.6 | 0.0378 |

Table continued

| | | | | CD5+ (n=25) | | CD5- (n=41) | |
|---|---|---|---|---|---|---|---|
| Clone name[a] | Chromsome band | Mega base[b] | Gene[c] | %[d] | log-rank P[e] | %[d] | log-rank P[e] |
| RP11-65D24 | 13q34 | 109.8 | | 24 | 0.00631 | 14.6 | NS |
| RP11-391H12 | 13q34 | 111.8 | *CULA4* | 24 | 0.00631 | 22.0 | NS |
| RP11-569D9 | 13q34 | 112.8 | *CDC16* | 24 | 0.01158 | 24.4 | NS |

[a] List of BAC/PAC clones from p telomere to q telomere for each chromosome number.

[b] Based on Ensembl genome mapping position (http://www. Ensembl.org).

[c] Genes contained in clones.

[d] % of cases with copy number gain.

[e] P values of logrank test between cases with copy number gain and those without such copy number gain.

[f] Not significant.

Table 7

Table 7 *List of BAC clones associated with inferior prognosis of losses in the CD5 + DLBCL group and statistic analysis*

| | | | | CD5+ (n=25) | | CDS- (n=41) | |
|---|---|---|---|---|---|---|---|
| Clone name[a] | Chromsome band | Mega base[b] | Gene[c] | %[d] | log-rank P[e] | %[d] | log-rank P[e] |
| RP5-888M10 | 1p36.21 | 12.2 | *Q9UIM0* | 16 | 0.00112 | 14.6 | NS[d] |
| RP5-1177E19 | 1p36.21 | 13.2 | *PRDM2* | 12 | 0.00007 | 17.1 | NS |
| RP3-410I8 | 1p36.21 | 14.4 | *Q9UIL2* | 20 | 0.00045 | 14.6 | NS |
| RP11-276H7 | 1p36.13 | 15.8 | *EPHA2* | 20 | 0.00011 | 12.2 | NS |
| RP11-473A10 | 1p36.13 | 16.8 | *NM 018125* | 24 | 0.00004 | 22.0 | NS |
| RP5-1056L3 | 1p36.13 | 19.4 | *NBLI* | 12 | 0.00019 | 12.2 | NS |
| RP11-401M16 | 1p36.12 | 20.2 | *NM 018584* | 24 | 0.00004 | 12.2 | NS |
| RP11-63N8 | 1p36.12 | 21.6 | *RAPIGAI* | 12 | <0.00001 | 14.6 | NS |
| RP1-74M1 | 1p36.12 | 22.8 | *EPHB2* | 20 | 0.004098 | 19.5 | NS |
| RP3-462O23 | 1p36.11 | 24.3 | *NM 178122* | 12 | 0.00019 | 17.1 | NS |
| RP11-111D20 | 1p36.11 | 25.8 | *PAFAH2* | 12 | 0.00019 | 12.2 | NS |
| RP1-159A19 | 1p36.11 | 27.5 | *NM 015699* | 16 | 0.0052 | 19.5 | NS |
| RP11-242024 | 1p35.3 | 29.1 | *EPB41* | 16 | 0.01959 | 22.0 | NS |
| RPS-1125N11 | 1p35.2 | 32.2 | *HDAC1* | 28 | 0.04845 | 19.5 | NS |
| RP4-811H24 | 1p35.1 | 32.6 | | 28 | 0.00529 | 14.6 | NS |
| RP5-1007G16 | 1p35.1 | 33.7 | *NM145205* | 24 | 0.00585 | 17.1 | NS |
| RPS-997D16 | 1p34.3 | 34.7 | *DLP3* | 24 | 0.00855 | 19.5 | NS |
| RP4-789D17 | 1p34.3 | 35.7 | *EIF2C1* | 16 | 0.01959 | 17.1 | NS |
| RP3-423B22 | 1p34.3 | 37.4 | *NGP1* | 20 | 0.00732 | 22.0 | NS |

[a] List of BAC/PAC clones from p telomere to q telomere for each chromosome number.

[b] Based on Ensembl genome mapping position (http://www. Ensembt.org).

[c] Genes contained in clones.

[d] % of cases with copy number loss.

[c] P values of logrank test between cases with copy number loss and those without such copy number loss.

[f] Not significant.

## 3. DISSCUSSION

Although DLBCL has been known as clinically heterogeneous, tools to elucidate underling molecular events for heterogeneity have been lacking. The advent of array technologies is now allowing such analysis. Microarray analysis of transcripts revealed DLBCL comprises at least three distinct subgroups that are also clinically relevant (Wright, G. et al. Proc. Natl. Acad. Sci. USA, 100: 9991-9996. 2003). Array CGH methods have been successfully used for the analysis of genomic alterations in a variety of tumors (Hackett, C. S. et al. Cancer Res., 63: 5266-5273, 2003 ; Veltman, J. A. et al. Cancer Res., 63: 2872-2880, 2003;Wilhelm, M. et al. Cancer Res., 62: 957-960, 2002), but not for DLBCL. The inventors established their own array CGH and analyzed 70 cases of DLBCL. This analysis for the first time revealed gains and losses of genome in DLBCL. The inventors were also able to identify regions of genomic gains and losses that were relevant to clinical subtypes and patient survival.

To confirm the accuracy of our array CGH, the inventors first excluded clones that did not show expected fluorescence in test-hybridization using normal male DNA. Secondly, the inventors confirmed the log2 ratio for concordance with the copy number. Finally, the inventors used lymphoma cell lines with known genomic aberrations and confirmed that their array was able to detect expected gains and losses. Having confirmed accuracy, the inventors next examined the resolution of the array. For this, the inventors compared the array CGH with a conventional CGH method for the detection of genomic aberrations of DLBCL samples (data by conventional CGH have been reported elsewhere) (Karnan, S. et al. Genes Chromosomes Cancer, 39: 77-81, 2004). Array CGH revealed aberrations of several loci that were undetectable by conventional CGH. Loss of 3p14.2 was such an example. This lost locus was detectable by array CGH in 18 out 66 cases of DLBCL whereas it was totally undetectable by conventional CGH. The responsible regions for 13 out of 18 cases were covered by a single BAC, RP11-48E21, which included the FHIT tumor suppressor gene. Thus array CGH was found to be more sensitive than conventional CGH. These findings suggest array CGH provides a useful tool in identifying and narrowing down aberrant genes. However, it should be noted that array CGH is limited to identification of copy number changes of genome, and is therefore unable to detect chromosomal translocations, mutations and epigenetic events that could affect gene expressions. Applications of array CGH method in combination with other newly developed technologies such as microarray analysis of transcripts and SKY analysis of chromosome may further facilitate our understanding of molecular events underlying DLBCL.

Conventional CGH analyses of our patient samples have revealed 6 frequent regions of gain (3q, 6p, 11q21-q24, 12q, 13q22-q32, 18q, X) and four frequent regions of loss (1p, 6q, 17p, 19p) (Karnan, S. et al. Genes Chromosomes Cancer, 39: 77-81, 2004). Array CGH analysis of the same set of patient samples revealed (since we defined continuous three clones level as commonly gained or loss regions) at least five novel regions as frequently gained in addition to regions identified by CGH. Regions with minimum common region of loss such as 3p14 and 9p21 were also found. However loss of 19p that had been found by CGH were not confirmed in array analysis. Reasons for this apparent discordance between conventional CGH and array CGH is not entirely clear at present. One possibility is that chromosome 19 contains blocks of heterochromatin that are difficult to evaluate by conventional CGH. Therefore, it is possible that the discordance is due to the unreliable results of conventional CGH in these regions. Other researchers reported, by the use of conventional CGH methods, 1q21-q23, 2p12-p16, 3q26-q27, 7q11, 8q24, 9q34, 11cen-11q2 12p, 12cen-q13, 13q32, 16p12, 16q21, 18q21-q22 and 22q12 as frequent regions of gain, and, 1ptel-1p34, 6q23-qtel, 8ptel-8p22, 17p 12, and 22q as frequent regions of loss (Monni, O. et al. Blood, 87: 5269-5278, 1996;Rao, P. H. et al. Blood, 92: 234-240, 1998;Berglund, M. et al. Mod. Pathol., 15: 807-816, 2002). Among these we were not able to identify 9q34 and 22q12 as frequent regions of gain by our array CGH; gains of 9p34 and 22q12 were found in 5 (7.6%) and 6 (9.1%), respectively, out of a total of 66 DLBCL cases. This discordance could be due to different patient samples analyzed and different sensitivity of the methods used.

Array CGH analysis revealed largely identical genomic aberration patterns both in the CD5+ and in CD5-groups. However, gains of 10p15.3-p14 and 19q13.33-13.43 and loss of 1q43-q44 and 8p23.2-p23.1 (Tables 4 and 5) were found characteristic of CD5+ DLBCL, although gain of 10p15.3-p14 in CD5+ DLBCL was low in frequency (16%). These findings, along with characteristic clinical behaviors, are indicative of distinct entity of CD5+ DLBCL. Genes included in the regions of 10p 15.3-p 14 and 1q43-q44 have not been yet demonstrated to be linked to malignancy, but 19q13.4 region includes tumor-related genes such as *BAX, PEG3* (Kohda, T., et al. Genes Cells, 6: 237-247, 2001), *CD37,* and *IL4R*1 that was discovered as a responsible gene for primary mediastinal diffuse large B-cell lymphoma (Copie-Bergman, C. et al. Blood, 101: 2756-2761, 2003). Genomic loss of 8p23 has frequently been found in leukemic mantle cell lymphoma (MCL); this deletion has a possible link with leukemic dissemination and poor prognosis of patients with MCL (Martinez-Climent, J. A. et al. Blood, 98: 3479-3482, 2001). Given that 8p23 is frequently lost in CD5+ DLBCL, in addition to MCL, one could speculate this locus may contain tumor suppressor genes accounting for poor prognosis of patients with both CD5+ DLBCL and leukemic MCL.

It was found that gain of 13q21.1-q34 and losses of 1p36.21-p34.3 had deleterious impacts on survival of CD5+ cases; these regions had no such impact on CD5- cases.

In contrast to CD5+ cases, we were not able to find genomic regions, either gained or lost, characteristic to CD5- DLBCL. However, among CD5- DLBCL cases, gain of 5p was found associated with a favorable rate of survival. Clearly, analysis of more cases is needed to elucidate its prognostic roles.

In summary, we have performed for the first time array CGH analysis for DLBCL cases and found genomic regions frequently altered in DLBCL. In comparing CD5+ and CD5- cases, we were able to identify genomic alterations specific to the CD5+ DLBCL group. Array CGH analysis should provide new insights into understanding genetic background of lymphomagenesis.

**Example 2**

1. MATERIALS AND METHODS

1-1. Cell Lines, Tumor Samples and CGH Method

The cell lines used were Karpas 1718 (splenic lymphoma with villous lymphocytes: SLVL) (Martinez-Climent, J. A. et al. Blood, 101: 3109-3117, 2003), OCI-Ly4, OCI-Ly7, and OCI-ly8 (DLBCL, kindly provided by Dr. R. Dalla-Favera of Columbia University, NY, NY), Ree1 (mantle cell lymphoma: MCL, kindly provided by Dr. M. Dyer of Leicester University, Leicester, UK) (Martinez-Climent, J. A. et al. Blood, 98: 3479-3482, 2001), Karpas 422 (B-cell lymphoma cell line) (Dyer, M. J. et al. Blood, 75: 709-714, 1990), ATN-1 (Adult T-cell lymphoma cell line, kindly provided by Dr. T. Naoe of Nagoya University School of Medicine, Nagoya, Japan) (Naoe, T. et al. Cancer Genet Cytogenet., 34: 77-88. 1988). SUDHL6 (Southwestern University Diffuse Histiocytic Lymphoma cell line, B-cell lymphoma), SP49 (MCL cell line), Jurkat (T-cell acute lymphocytic leukemia), and other cell lines were described elsewhere (Takizawa, J. et al. Jpn J cancer Res., 89: 712-718, 1998). Cell lines with variable copy numbers of the X chromosome were purchased from the NIGMS Human Genetics Cell Repository Coriell Institute for Medical Research (Camden, NJ). Patient samples were collected with informed consent and this experiment was approved by the IRB (Institutional Review Board) of Aichi Cancer Center. All cell lines were maintained in RPMI1640 supplemented with 10% fetal calf serum. Genomic DNA was extracted according to standard procedures using proteinase K digestion and phenol chloroform extraction. Normal DNA for use with conventional CGH and array CGH was prepared by using peripheral-blood lymphocytes from a normal male. 'Conventional' CGH was carried out according to manufacturer's protocol (Vysis, Downers Grove, IL).

1-2. Array CGH

The array fabrication and hybridization was performed according to the method described by Hodgson et al (Nat Genet., 29: 459-464, 2001) and Pinkel et al (Nat. Genet., 23: 41-46, 1998), respectively. The array consisted of 2,088 BAC and PAC clones, covering the human genome at roughly a 1.5-Mb resolution, from library RP11, 13 for BAC clones and RP1, 3, 4, 5 for PAC clones. Information of clone names and their location on chromosomes is available on request. These clones were obtained from the BACPAC Resource Center at the Children's Hospital Oakland Research Institute in Oakland, CA (http://bacpac.chori.org/). Each clone was cultured in Terrific Broth (TB) medium with the chloramphenicol (25 $\mu$g/ml) and BAC and PAC DNA was extracted with a plasmid Mini-kit (QIAGEN, Germantown, MD). The location of each clone was also confirmed by FISH analysis. Roughly 10% of these clones could not be assigned to their expected region and excluded from this study, while the confirmed clones were used for array CGH. We used 10 ng of BAC and PAC DNA as the template for degenerate oligonucleotide primed PCR (DOP-PCR) with the primer 5'-CCGACTCGAGNNNNNNATGTGG-3' (SEQ ID NO: 1) (Hakan, T. et al. Genomics, 13: 718-725, 1992). Amplifications were performed on a TaKaRa PCR Thermal Cycler MP (TaKaRa, Tokyo, Japan) using ExTaq polymerase (TaKaRa). DOP-PCR products were enriched by ethanol precipitation and dissolved in distilled water, and then equal. volume of DNA spotting solution DSP0050 (MATSUNAMI, Osaka, Japan) was added (~1 $\mu$g/$\mu$l) . DNA was robotically spotted (NGK Insulators, Ltd., Nagoya, Japan) in duplicate onto CodeLink™ activated slides (Amersham Biosciences, Piscataway, NJ). Tested and reference DNA (1 $\mu$g each) was digested with *Dpn*II and labeled with the Bio prime DNA labeling system (Invitrogen Life Technologies, Inc., Tokyo, Japan) using cyanine3-dUTP and cyanine5-dUTP (Amersham Pharmacia Biotech, Piscataway, NJ), respectively. Unincorporated fluorescent nucleotides were removed with the aid of Sephadex G-50 spin columns (Amersham Biosciences). Labeled 1 $\mu$g of tested and reference DNA samples was mixed with 100 $\mu$g of Human Cot-1 DNA (Invitrogen Life Technologies) and precipitated, after which the pellet was resuspended in the 45 $\mu$l hybridization mixture which consisted of 50% formamide, 10% dextran sulfate, 2 x SSC, 4% SDS and 10 $\mu$g/)$\mu$l yeast tRNA (Invitrogen Life Technologies). The hybridization solution was heated to 73°C for 5 min to denature the DNA, and then incubated for 45 min at 37°C to allow blocking of the repetitive sequences. The slides spotted with DNA were denatured in a solution consisting of 70% formamide/2 x SSC at 73°C for 4 min, then dehydrated in cold 70%, 85%, and 100% ethanol for 5 min each and air-dried. Hybridization was performed for 48 hours in a container placed on a slowly rocking table and containing 200 $\mu$l of 50% formamide and 2 x SSC to control moisture, and followed by 15 min post hybridization washing in 50% formamide/2 x SSC at 50°C, 30 min in 2 x SSC/0.1% SDS at 50°C, 15 min in PN buffer consisting of 0.1M NaH$_2$PO$_4$, 0.1M Na$_2$HPO$_4$ at pH8, and 0.1% NP-40 at room temperature, rinsing in 2 x SSC at room temperature, and finally dehydration in 70%, 85%, 100% ethanol at room temperature for 2 min each and air-drying. Scanning analysis was basically carried out with the Agilent Micro Array Scanner (Agilent Technologies, Palo Alto, CA). Thus acquired array images were analyzed using Genepix Pro 4.1 (Axon Instruments, Inc., Foster City, CA).

DNA spots were automatically segmented, the local background was subtracted, and the total intensities and fluorescence intensity ratio of the two dyes for each spot were calculated. Fluorescence intensity ratio of the two dyes (Cy3 intensity/Cy5 intensity) were converted into $\log_2$ intensity ratios ($\log_2$ ratio).

For the array used in this study, six simultaneous hybridizations of normal male versus normal male were performed to define the normal variation for $\log_2$ ratio. In this experiment, 122 clones showed less than one-tenth the fluorescence intensity of the mean value for all clones, and were excluded from array CGH analysis. The remaining 1,966 clones were used for the array CGH analysis. More than 95% of the measured fluorescence $\log_2$ ratio values of each spot (2 x 1,966 clones) ranged from +0.2 to -0.2. The thresholds for the $\log_2$ ratio of gains and losses were set at the $\log_2$ ratio of +0.2 and -0.2, respectively. We also normalized the $\log_2$ ratio of each sample according to the following method.

The medium $\log_2$ ratio value for all clones was computed and the clones were selected with a $\log_2$ ratio more than "the median + SD x A " or less than " the median - SD x A ". "A" was visually defined as the normal region by referring to the $\log_2$ ratio plots of all clones for each experiment. " A " was also assigned an approximate ranged from 0.3 to 0.7. We then computed the mean $\log_2$ ratio value for the selected clones, and designated this mean $\log_2$ ratio value as " X ". Finally, we obtained the " Y " value by subtracting " X " from the $\log_2$ ratio for each clone. In this study, each $\log_2$ ratio was analyzed on the basis of the " Y " value. We visually selected the clones, computed the SD for each experiment and confirmed that the SD did not exceed 0.15. If it did, the value was considered unreliable for CGH analyses.

Array-hybridization of normal male versus normal female was performed to check any change in one copy number of the X chromosome. In order to confirm linearity associated with a change in the copy number, we also performed array-hybridization of normal versus each cell line with different number of the X chromosome (GM04626: 47XXX; GM01415D: 48XXXX; and GM05009C: 49XXXXX). These cell lines were obtained from the NIGMS Human Genetics Cell Repository Coriell Institute for Medical Research. 57 BAC or PAC clones of the X chromosome were used for the analysis. We computed the mean $\log_2$ ratio value of those clones on each hybridization.

1-3. FISH Analysis

We confirmed the location of BAC clones on 13q31-q32 from information archived by the Ensembl Genome Data Resources (http://www.ensembl.org/). FISH analysis using 19 BAC clones located around the region of high-level amplification of 13q31-q32 demonstrated by array CGH, covering approximately 15-Mb, was used for three cell lines (Karpas 1718, OCI-Ly4, and Rec1). Each interphase chromosomes slide of cell lines was prepared according to a standard method. FISH was carried out according to the method described elsewhere (Tagawa, H. et al. Oncogene, in press, 2003).

1-4. Location of ESTs and Genes

ESTs and genes located on the region of chromosome 13q31.3 were referenced by the National Center for Biotechnology Information (NCBI; http://www.ncbi.nlm.nih.gov/), the Ensembl genome data resource (http://www.ensembl.org/) and the University of California at Santa Cruz (UCSC; http://genome.ucsc.edu/). Array CGH and FISH analysis demonstrated that the common region of amplification at 13q31-q32 extended from BAC, RP11-360A9 to BAC, RP11-93M14. This region contained 65 independent ESTs that do not overlap each other and *GPC5* (Table 8).

1-5. Reverse Transcription-Polymerase Chain Reaction (RT-PCR) Analysis

Three cell lines, Rec1, Karpas 1718, and OCI-Ly4, which showed high amplification on 13q31.3 by means of FISH and array CGH, were used for RT-PCR analysis. cDNA derived from fetal brain was also included. In order to avoid amplification from contaminated genomic DNA in the RNA samples, RNA was treated with amplification grade DNaseI (Invitrogen Life Technologies, Inc.) before cDNA syntheses of the samples, which were performed using SuperScriptII (GIBCO-BRL, Div. of Life Technologies, Inc., Gaithersburg, USA). Briefly, each 5 $\mu$l of total RNA was reverse-transcribed into cDNA dissolved in 40$\mu$l of distilled water. RT-PCR was performed for 65 ESTs and *GPC5* using the specific primers (Table 8). Each primer was also designed so that the Tm value would be between 55°C and 60°C. Amplifications were performed on a Thermal Cycler (Perkin-Elmer Corporation, Norwalk, CT). RT-PCR was conducted with the touchdown PCR method described elsewhere (Motegi, M. et al. Am J Pathol., 156: 807-812, 2000). Briefly, the reactions were comprised 10 cycles of denaturation (94°C, 0.5 min), annealing (63°C, 0.5 min, 1°C decrease per 2 cycles), and extension (72°C, 2.5 min), followed by 35 cycles of denaturation (94°C, 0.5 min), annealing (58°C, 0.5 min), and extension (72°C, 2.5 min), and a final extension of 5 min at 72°C. Basically, the annealing temperature of the reaction was from 63 to 58°C. Additionally, RT-PCR was also performed under different conditions by changing the annealing temperature from 65 to 60°C or 60 °C to 55°C. If no PCR products were obtained, we designed new primer sets to confirm their true negativity. All PCR products were separated by electrophoresis and purified using the QIA quick™ Gel Extraction Kit (QIAGEN). TA cloning to purified PCR products was performed by using pBluescriptII SK (-), and sequenced by using ABI PRISM™ 310 Genetic Analyzer (Applied Biosystems, Foster City, VA).

1-6. Northern Blot Analysis

Northern blotting was performed with 30 ESTs and *GPC5* cDNA against five cell lines (Rec1, Karpas 1718, OCI-Ly4, Jurkat, and ATN-1) and human placenta. Further analysis used the candidate genes BC040320 and *GPC5,* which was included because it has been reported to be a candidate gene for this region (Yu, W. et al. J Hum Genet., 48: 331-335, 2003). We analyzed and compared the expression of each of the ESTs and *GPC5* in the cell lines with high-level amplification at 13q31-q32 (Rec1, Karpas 1718, and OCI-Ly4) and in the cell lines without (Jurkat and ATN-1). In order to examine the expression of BC040320 and *GPC5* in detail, Northern blot analysis was also performed for several cell lines and patients. Northern blot hybridization was performed with a standard method (Naoe, T. et al. Cancer Genet Cytogenet., 34: 77-88. 1988). Each RT-PCR product was used as a specific probe labeled by means of PCR. Briefly, 10 ng of the RT-PCR products were labeled with [$\alpha$-$^{32}$P]-dCTP by means of PCR. The reactions were carried out with 25 cycles of denaturation (94°C, 0.5 min), annealing (55°C, 0.5 min), and extension (72°C, 2.5 min), and a final extension of 5 min at 72°C. Total cellular RNA (5 $\mu$g) was size-fractioned on 1% agarose/0.66 M formaldehyde gel, transferred onto a Hybond-N$^+$ nylon membrane (Amersham Pharmacia Biotech, Tokyo, Japan). The membranes were then hybridized overnight at 42°C with [$\alpha$-$^{32}$P]-dCTP-labeled probes, washed, and then exposed to BIOMAX™ MS films (EKC, Rochester, NY).

1-7. Candidate Gene Analysis

BC040320, a candidate gene in the 13q31-q32 amplification region was further analyzed. In order to confirm the sequence of the candidate gene, RT-PCR was performed between exon 1 and exon 4 of BC040320 using the primers 5'-TCCGGTCGTAGTAAAGCGCAGGCG-3' (SEQ ID NO: 2), designed on the side of exon 1 and 5-'CTGAAGTCTCAAGTGGGCAT-3' (SEQ ID NO: 3), designed on the side of exon4 of BC040320. The PCR reaction was the same as the one described in the RT-PCR section.

2. RESULTS

2-1. Array CGH Analysis

Array CGH consisting of 1,966 BAC and PAC clones were examined with normal male versus female, and demonstrated that most of the signals from autosomal chromosomes are within log$_2$ ratio of +0.2 to -0.2 (Fig. 8A). The linearity of copy number changes was studied with cell lines having different number of X chromosomes. As shown in Fig. 8*B*, the result of the plot of each calculated mean fluorescence ratio demonstrated that the fluorescence ratio was proportional to the change of one copy number. The array CGH used for four cell lines (Karpas 1718, Rec1 OCI-Ly4, and OCI-Ly7) and one DLBCL patient (D778) demonstrated high-level gains in copy number changes at 13q31-q32. Fig. 9A shows a representative result for Karpas 1718 of the array CGH. Detailed results of chromosome 13 from three cell lines (Karpas 1718, Rec1, and OCI-Ly7) and one DLBCL patient (D778) are shown in Fig. 8*B*. Conventional CGH and FISH analyses clearly confirmed these array CGH data (Fig. 9).

2-2. The Common Region of Amplification at 13q31-q32

In an attempt to narrow the amplicon at 13q31-q32, FISH analysis of three cell lines (Karpas 1718, Rec1, and OCI-Ly4), using 19 BAC/PAC probes located on 13q31-q32 (Fig. 10A) were conducted, and it was found that the common amplified region at 13q31-q32 was located between RP11-29C8 and RP11-93M14. The high-resolution array CGH data is shown in Fig. 10*B*. Karpas 1718 and D778 (DLBCL patient sample) showed a wide area of amplification extending over more than 50Mb of chromosome 13q. A small genomic region showing high-level amplification (defined as log$_2$ ratio > 1) extended from 13q22.2 to 13q31.3, with the region of 13q31.3 in particular showing a higher log$_2$ ratio of > 2. In the same manner, OCI-Ly7 and Rec1 also showed high-level amplification, which confined to 13q31.3. These results identified the common region of high-level amplification to extend from RP11-360A9 to RP11-481A22. On the basis of the FISH and array CGH results, we defined the genomic region between RP11-360A9 and RP11-93M 14 as the common and smallest region of amplification in four cell lines and one DLBCL patient.

2-3. RT-PCR Analysis for ESTs of Chromosome 13q31.3

Expression of 65 ESTs and *GPC5* located in the common region of amplification on 13q31.3 were examined by RT-PCR using cDNA derived from three cell lines (Karpas 1718, OCI-Ly4, and Rec1) and fetal brain. RT-PCR products were examined by gel electrophoresis. A positive signal was defined as detection of an expected size of band. The results are summarized in Table 8. Thirty ESTs and GPC5, which showed the expected size of band, were found to be positive in all of the three cell lines with amplification of 13q31.3, and they were confirmed by their nucleotide sequence. Fifteen ESTs also showed the expected size but RT-PCR analysis demonstrated only one or two cell lines so that they were excluded as candidate ESTs associated with amplification at 13q31.3. The remaining 21 ESTs did not show any bands in either OCI-Ly4 or fetal brain. They were also examined with another set of primers, but again no bands were detected (data not shown) so that they were also excluded as the candidate ESTs. A total of 35 out of 65 ESTs were thus excluded and not analyzed further.

2-4. Northern Blotting

To identify the expression patterns of 30 ESTs and *GPC5,* Northern blot was used for six kinds of RNAs, which were human placenta, three B-cell lymphoma cell lines (Rec1, Karpas 1718, and OCI-Ly4) with 13q31.3 amplification and two T-cell lymphoma cell lines (Jurkat and ATN-1) without 13q31.3 amplification. Twenty-two of the ESTs showed hardly any detectable bands in any of the cell lines (Table 9). Fig. 11 shows representative expression patterns of the ESTs. AF339828 and BC040320 showed the similar expression pattern of a transcript of about 6-kb and a smeary band bigger than 6-kb. The signals were observed in only three B cell lymphoma cell lines, but not in human placenta or the T cell lines. *GPC5,* a gene incompletely included in the common region of amplification at 13q31-q32, showed weak expression of about 5-kb transcript in all cell lines and the human placenta at similar intensity. The signals for the other ESTs did not reflect any difference in copy number at 13q31.3 between cell lines and human placenta. We therefore regarded AF339828 and BC040320 as the most possible target gene for amplification at 13q31.3. Further study against various samples including patient samples and normal tissues was performed with these two ESTs, and *GPC5,* reportedly a target gene for 13q31.3 amplification, was also examined.

Northern blot results with the BC040320 probe are shown in Fig. 12. High-level expression of BC040320 was seen in five cell lines with amplification at 13q31-q32 (Rec1, Karpas 1718, OCI-Ly4, OCI-Ly7, and OCI-Ly8). Lower level of expression than that of the five cell lines was seen in three cell lines without amplification (Karpas 422, SP49, and SUDHL6). Furthermore, two patients with amplification showed higher expression than the other two patients without amplification. These results indicated that the expression of BC040320 paralleled the gain in copy number shown by both conventional and array CGH. On the same membrane, expression of *GPC5* in five cell lines with amplification at 13q31-q32 was not significantly different from that of the other cell lines without amplification, suggesting that *GPC5* is not a likely candidate gene. The expression pattern of BC040320 was examined against various hematopoietic cell lines (T cell Lymphoma, multiple myeloma, myeloid leukemia, and NK/T cell lymphoma) (Fig. 14). Some cell lines showed weak signals when compared with the two cell lines with high-level amplification. *GPC5* cDNA again yielded very weak signals without significant differences but with some variation. When normal tissues were examined, the BC040320 signal was hardly observable except for lung, thymus and lymph node (Fig. 15).

In conclusion, the result of Northern blot using each of the probes revealed that the expression of BC040320 paralleled the gain in copy number at 13q31-q32 and that BC040320 was thus most likely to be the candidate gene.

2-5. Full Length, Genomic Location and Characterization of The Candidate Gene

We focused on AF339828 and BC040320 as the most likely candidate gene on the basis of the results of FISH, array CGH and Northern blot analysis. We named this candidate gene *C13orf25 (Chromosome 13 open reading frame 25)* according to the recommendation of HUGO Gene Nomenclature Committee (http://www.gene.ucl.ac.uk/nomenclature/).

In order to further characterize this gene and cDNA structures, we performed RT-PCR on exon 1-exon 4 of BC040320. Two transcripts were obtained and sequence analysis found the shorter one to be transcript-A and the longer one transcript-B (Fig. 14). Data base search with the Vega Genome Browser showed that bA121J7.2 (Vega_gene ID) is also located in this region (http://vega.sanger.ac.uk/). The peptides of 32-amino acids (AA) were predicted as bA121J7.2. The peptides of 32-AA predicted in bA121J7.2 were also predicted transcript-A. Furthermore, the same initiation codon predicted the 70-AA polypeptide in the transcript-B. It should be noted that five kinds of precursor microRNAs (miRNAs) (miR91-precursor-13 micro RNA, miR18- precursor-13 micro RNA, miR19a-precursor-13 micro RNA, miR19b-precursor-13 micro RNA and miR92-precursor-13 micro RNA), including seven kinds of miRNA (microRNA miR-17, miR-91, miR-18, miR-19a, miR-20, miR-19b and miR-92) were also recognized in the sequence of transcript-B (Fig. 14).

Table 8

Table 8 *RTPCRanalysis* BAC clones, EST and STS Marker was refered to the informaton obtained from Ensembl genome data resource. All BAC clones are included in RPCI-11 human male BAC library .

| BAC | EST/GENE | STS Marker | Forward primer | Reverae primer | FB | Rec1 | Karpas | OCI-Ly4 |
|---|---|---|---|---|---|---|---|---|
| 360A9 | AW664738 | D13SI457 | 5'-gccacatggtcaggttaaac | 5'-cttcactacettgcgaetct | - ND[b] | | ND | |
| 27D9 | AA309162 | | 5'-ggctctcatttagctaaatg | b'-sagaagtggttgagacaaca | + | + | + | + |
| 15N8 | no EST | | | | | | | |
| 321E13 | AW236754 | D13S1175 | 5'-gegatggcacagcegttgaa | 5'-tagttcaaactctacctgca | - | ND | ND | - |
| 447M23 | no EST | | | | | | | |
| 370B1 | LOC121723 | | 5'-aactactggtgaggactgca | 5'-caagttocccttctgcagaa | - | ND | ND | - |
| | AW059867 | | 5'-aaggcttagctattatgctg | 5'-aceatgaggaaaatctccca | ++[d] | ++ | ++ | ++ |
| 275J18 | BC042969 | D13S1818 | 5'-ctggtcacacatccacaatg | 5'-cagtggaatctgagtcctag | ++ | + | + | ++ |
| | AA628299 | | b'-cagaaggagtgttaagtctg | 5'-eaagaagaegctgccagtat | ++ | ++ | ++ | ++ |
| 143010 | no EST | | | | | | | |
| 309H8 | BG183515 | D1351239 | 5'-ctcatgactgtaatcccagc | 5'-gtgatgccgtgaaatgagtc | + | + | + | + |
| | BG191981 | | 5'-ttcagtgacctcactgactg | 5'-gaggattttgcagtcatcgc | - | ND | ND | - |
| 114G1 | LOC160824 | D135767 | 5'-aggttttgtcagccacacct | 5'-gaactatccgtaocttgtcc | - | ++ | + | + |
| 75N6 | AA398228 | | 5'-ctgtaccettgtgcccagaa | 6'-etgectcagtccttctggct | - | ND | ND | - |
| 86C3 | no EST | D13S265 | | | | | | |
| 51A2 | no EST | | | | | | | |
| 18M3 | no EST | | | | | | | |
| 388D4 | LOC144774 | | 5'-cactgtggcagttatacggt | 5'-caatggttttccgcaccagt | - | ND | ND | - |
| | LOC121727 | | 5'-cctgggaaggatggtttctt | 5'-ttacaacacaaggggcacac | + | + | + | ++ |
| 409J23 | no EST | | | | | | | |
| 392A19 | LOC121729 | | 5'-ggagccattacttcaagacc | 5'-agaccagtacttgtceagct | + | ++ | ++ | ++ |
| | BG776186 | | 5'-tggacacgtgagtgtgtttc | 5'-atgagctcaggcagctctat | + | ++ | - | + |
| | LOC121728 | | 5'-tgataggagacagacctgac | 5'-tocagtcatcctgaggtaga | - | ND | ND | - |
| | BG186078 | | 5'-tcacacagtgtgactcacag | 5'-tgatctcctctctgtagtgc | ND | - | - | + |
| | AA487882 | | 5'-acccaagggaatattgacac | 5'-gaagctagggaagragtatt | ND | - | - | + |
| | BM542991 | | 5'-tgcagtaggtggcaatctca | 5'-tcgacttggactccatgaga | - | ND | ND | - |
| 505P2 | BM695971 | D13S1234 | 5'-ccttggagtgcttaaggtag | 5'-actagggctctttgtagacc | ND | - | - | ++ |
| 168A8 | AI027278 | | 5'-gctgtgattgcgaagaagtg | 5'-tocatatgtgagtgtggcag | ND | - | - | + |
| | BG927281 | | 5'-acgccaagctcteatacgac | 5'ctcgggctatggtatatgac | - | ND | ND | - |
| | BI82b411 | | 5'-aceetseaeaggtatggaat | 5'-accatgagcacagtgctcaa | - | ND | ND | - |
| | AA888411 | | 5'-ctcocattgcagttactatg | 5'-gtcgacetgttgttgaggtt | + | + | + | + |

Table continued

| BAC | EST/GENE | STS Marker | Forward primer | Reverae primer | FB | Rec1 | Karpas | OCl-Ly4 |
|---|---|---|---|---|---|---|---|---|
| 360H15 | AW515966 | | 5'-atgttcaccaggctggtctt | 5'-actaactctgtgggcttgca | - | ND | ND | - |
| | BF818219 | | 5'-tctgccactaacatctggt | 5'-cttgagtaBcigagaccaca | - | ND | ND | - |
| 114C3 | no EST | | | | | | | |
| 432D3 | LOC144776 | D13S1190 | 5'-cacttccttgaaggggtttc | 5'-ctctgactcttgggcacaat | ND | + | + | + |
| | AI126313 | | 5'-ttgagacaragtcctgctct | 5'-gcagggcacaatgttttcag | - | ND | ND | - |
| 319L6 | AV731092 | | 5'-attggtgaagccacctcaaa | 5'-caggctaacatggatctagt | ND | - | - | + |
| | BG941714 | | 5'-agtgcctacttcetaagacc | 5'-tcaggaatcagtgcccaaac | ND | - | - | + |
| | AI262947 | | 5'-ttgtgttcctggtccaccta | 5'-acattgggecggtcacttat | ND | - | - | + |
| | AI493127 | | 5'-tcaaatcaactgcacctcag | 5'-cagttcgagacttcttccat | - | ND | ND | - |
| 51B13 | BX097335 | | 5'-gaagtaggatggtgacacct | 5'-ctcegcaagtcatcctttgc | - | ND | ND | - |
| 430K10 | AL701000 | D13S886 | 5'-tggcctggagtaattagctg | 5'-atttaggggtaggagagea | ND | - | - | + |
| | LOC160827 | | 5'-ctgtgcactatcaettggga | 5'-taggctctaagccgttggtt | - | ND | ND | - |
| | LOC160826 | | 5'-atggaatraggttccctcca | 5'-ctgttcccttcatctgaatg | - | ND | ND | - |
| | BQ477330 | | 5'-caaagtgctgggattacagg | 5'-gccagctttgctgcacatta | - | ND | ND | - |
| | BQ477741 | | 5'-caacagaagatcggcccttt | 5'-actcectEaagcacagcatt | ND | + | + | + |
| | AV731847 | | 5'-caggcacttgcttaagggat | 5'-aactagcctgcttcagcttc | - | ND | ND | - |
| | B1481522 | | 5'-atgtgaagagaggtctcagc | 5'-agcaaeccacctagaggctt | ND | + | + | + |
| | BU729287 | | 5'-cttagcctaatctccctaggcgga | 5'-tatcaggtaggtggtccagtctca | ND | ++ | ++ | ++ |
| | BM703078 | | 5'-caggatcttgccctgttegt | 5'-tatcgggtggcgaacaagat | ND | + | + | + |
| | AA719672 | | 5'-tgtecttaggtagacattgt | 5'-ttcaacctctgagaaaccca | ND | ND | ND | - |
| | LOC121734 | | 5'-acttcactgtcaacagcgag | 5'-agagaccacatgcttgccat | ND | ++ | ++ | ++ |
| | BE466687 | | 5'-agttctggaggctaaasgtccagt | 5'-gccaaaatcacatggagagactac | ND | + | + | + |
| 282D2 | BF362993 | | 5'-gagaacagtaatttetttcc | 5'-tgcaattattggggtaaagc | ND | ND | ND | ND |
| | BC040320 | | 5'-gtcatacacgtggacctaac | 5'-ctgaagtctcaagtgggcat | ND | ++ | ++ | ++ |
| 121J7 | AF339828 | | 5'-ctgacaagttctcagatcac | 5'-actctgcatgagcctagatt | ND | ++ | ++ | ++ |
| | AA701926 | | 5'-agaccctgatggtctcttta | 5'-ggctcaatgttttcctacgg | ND | ++ | ++ | ++ |
| | BF908089 | | 5'-tggaagaaaggacatgaggt | 5'-tctcatgaatccatgcccaa | ND | + | + | + |
| | AW866481 | | 5'-aagtaaatgtgagaagtagc | 5'-tgctcatcctcattgtata | ND | - | - | + |
| | BX107378 | | 5'-taacctgagcagaatccagccttg | 5'-atggacccaaatgctgagaggaac | ND | ++ | ++ | ++ |
| | AA599001 | | 5'-aagagggacttgctgtgttg | 5'-tgcacagacggtacagaagt | ND | ++ | ++ | + |
| | GPC5 | | 5'-cactggcgggtaaaggggac | 5'-agtattcagggaactgtcagtcacacc | ND | + | + | + |
| | AL043638 | | 5'-ccagtctatcattgatggac | 5'-gaagtgcctctgtaattgga | ND | ++ | ++ | ++ |

Table continued

| BAC | EST/GENE | STS Marker | Forward primer | Reverae primer | FB | Rec1 | Karpas | OCI-Ly4 |
|---|---|---|---|---|---|---|---|---|
| | AL708734 | | 5'-gtaatcccagcactttggga | 5'-tcttgttcttgtcccccagt | ND | + | + | + |
| 487A2 | AF339802 | D13S1490 | 5'-tacctgggtaaccaagactc | 5'-ctctgttcactgcattgaag | ND | ++ | ++ | ++ |
| | H56919 | | 5'-tgttgacegactgagtgaac | 5-ttatggtgaagtccttcccc | ND | + | + | ++ |
| | AA705439 | | 5'-cgtactctagagttaaccaa | 5'-atgattgtaagttccctgag | ND | ++ | ++ | ++ |
| | W86832 | | 5'-atcctcatttctcaggggct | 5'-cctgtctgctctatgaagct | ND | - | - | - |
| | N49442 | | 5'-tggctgggcagaaatctgaa | 5'-tacaggtctgttcgccacat | ND | ++ | ++ | ++ |
| | N33596 | | 5'-tccctageaatgtgatgtac | 5'-ctaaggtattcctaggctca | ND | - | - | + |
| | T84913 | | 5'-gtagtaggtagaactgtcct | 5'-atctaccctcggcaattttc | ND | ++ | ++ | ++ |
| | BU656134 | | 5'-tgctagggctggagtacaat | 5'-cattttctcttggctcaccc | ND | ++ | ++ | ++ |
| 93M14 | AW105449 | | 5'-ccagcaactgtaatacatgc | 5'-tcttcaaatccttgcctctg | ND | - | + | ++ |
| | AV754681 | | 5'-acagccttctttggagagtg | 5'-tccaagggcacagtggaatt | ND | - | ++ | ++ |

Fetal Brain. [b]Not Done. Detection of thin band or [d]a thick band from the result of electrophoresis.

Table 9

Table 9 *Northern blot analysis* Each signal of those ESTs and GPC-5 was visually evaluated after one week expose.

| BAC | EST/gene | probe size (bp) | size (kb) | Northern Blot | | | | | |
|---|---|---|---|---|---|---|---|---|---|
| | | | | placenta | Rec1 | Karpas 1718 | OCI-Ly4 | Jurkat | ATN-1 |
| RP11-27D9 | AA309162 | 130 | - | - | - | - | - | - | - |
| RP11-370B1 | AW059867 | 160 | - | - | - | - | - | - | - |
| RP11-275J18 | BC042969 | 440 | - | - | - | - | - | - | - |
| | AA628299 | 220 | - | - | - | - | - | - | - |
| RPM-309H8 | BG183515 | 440 | - | - | - | - | - | - | - |
| RPII-11401 1 | LOC160824 | 550 | 6.5 | +++ | +++++ | ++++ | +++ | +++ | +++ |
| RPII-38SD4 | LOC121727 | 420 | - | - | - | - | - | - | - |
| RP11-392A19 | LOC121729 | 350 | 1.5 | + | - | - | - | - | - |
| RP11-158A8 | AA888411 | 300 | - | - | - | - | - | - | - |
| RP11-432D3 | LOC144776 | 500 | - | - | - | - | - | - | - |
| RP11-430K10 | BQ477741 | 240 | - | - | - | - | - | - | - |
| | B1481522 | 210 | 5.5 | + | ++ | ++ | + | ++ | + |
| | BU729287 | 460 | | + | - | - | - | - | - |
| | BM703078 | 470 | - | - | - | - | - | - | - |
| | LOC121734 | 240 | 0.8 | + | +++ | ++ | ++ ++ | | +++ |
| | BE466687 | 390 | - | - | - | - | - | - | - |
| RP11-282D2 | BC040320 | 400 | 6 | - | +++++ | +++++ | +++++ | - | - |
| RP11-121J7 | AF339828 | 410 | 6 | - | +++++ | +++++ | +++++ | +/- | - |
| | AA701926 | 240 | - | - | - | - | - | - | - |
| | BF908089 | 100 | - | - | - | - | - | - | - |
| | BX10T378 | 420 | - | - | - | - | - | - | - |
| | AA599001 | 200 | - | - | - | - | - | - | - |
| | GPC5 | 600 | 5 | + | + | + | + | + | + |
| | AL043638 | 200 | - | - | - | - | - | - | - |
| | AL708734 | 250 | - | - | - | - | - | - | - |
| RP11-487A2 | AF339802 | 320 | 6 | +/- | ++ | ++ | ++ | + | + |
| | H56919 | 160 | - | - | - | - | - | - | - |
| | AA705439 | 290 | 15 | + | ++ | + | +++ | +/- | +/- |
| | N49442 | 320 | 15 | + | ++ | + | +++ | +/- | +/- |
| | T84913 | 200 | - | - | - | - | - | - | - |
| | BU656134 | 390 | - | - | - | - | - | - | - |

3. DISCUSSION

Genetic alteration in 13q has been reported in a wide range of human cancers, including hematopoietic malignancies. Recent molecular genetic studies using FISH and CGH have demonstrated that amplification at 13q31-q32 has been frequently detected in hematopoietic malignancies. Amplification at 13q21-qter was frequently demonstrated in B-cell malignancies (Rao, P. H. et al. Blood, 92: 234-240, 1998;Monni, O. et al. Genes Chromosomes Cancer, 21: 298-307, 1998;Neat, M. J. et al. Genes Chromosomes Cancer, 32: 236-243, 2001 ; Mao, X. et al. Genes Chromosomes Cancer, 35: 144-155, 2002). Recently, *GPC5* has been proposed as the candidate gene for 13q31-q32 amplification region in B cell lymphoma cell lines (Yu, W. et al. J Hum Genet., 48: 331-335, 2003). In the study reported here, we examined genomic alteration at the *GPC5 loci* using array CGH and the expression of *GPC5* using northern blotting. The *GPC5* sequence in the 2-Mb genomic region at 13q31.3 approximately ranges from BAC, RP11-121J7 to BAC, RP11-268K13. Our array CGH data for Rec1 showed that the $\log_2$ ratio of BAC, RP11-481A22, which located the intron of *GPC5* between exon 6 and exon 7, showed a loss in copy number ($\log_2$ ratio = -0.76). The array data indicated that other BACs located on the telomeric side of this BAC also showed a loss. Our FISH data with Rec1 using the new BAC clone, RP11-93M14, containing exon 3, exon 4, and exon 5 of *GPC5,* also showed a loss in copy number. These results demonstrated that the *GPC5* locus was not fully included in the common region of amplification at 13q31-q32 in the cell lines, suggesting that *GPC5* in this allele might not be not functional.

Northern blotting also showed that expression of *GPC5* in cell lines with amplification at 13q31-q32 was not significantly different from that of the other cell lines without amplification. On the other hand, both BC040320 and AF339828 were expressed only in B-cell lymphoma cell lines with 13q31-q32 amplification but not in T cell lymphoma or human placenta without 13q31-q32 amplification. Their ESTs were fully included in the common region of amplification at 13q31-q32. Detailed analysis using Northern blot showed that the expression of BC040320 almost paralleled the gains in copy number shown by both conventional and array CGH. Northern blot analysis showed that BC040320 was especially over-expressed in B-cell lymphoma cell lines with amplification at 13q31-q32 and hardly expressed in normal tissues, including lymphoid tissues. Although we also found minor mRNA expression of BC040320 in SP49 (MCL cell line) and SUDHL6 (B-cell lymphoma cell line) without amplification at 13q31-q32 (Fig. 6*B*), this expression may well have been caused not by the gain in copy number but other reasons that are not yet fully understood. These results suggested that BC040320 was the most likely a candidate gene for the amplification at 13q31-q32. The inventors named this candidate gene *C13orf25 (Chromosome 13 open reading frame* 25).

In order to confirm the validity of *C13orf25* cDNA, RT-PCR was performed and two transcripts (Transcript-A and -B) were obtained. The Vega genome browser (http://vega.sanger.ac.uk/) predicted the presence of a gene, bA121J7.2, encoding 32-AA polypeptides in the Transcript-A cDNA. A possible ORF in Transcript-B was also predicted, encoding 70-AA polypeptides starting from the same ATG (Fig. 14). The genomic structure of *C13orf25* might be incomplete on the 3' side because AF339828, which showed the same pattern of hybridization as BC040320, was observed near *C13orf25* and was located at 300-bp downstream of *C13orf25.* Because of the presence of multiple bands in Northern blot analysis with BC040320, various transcripts might also be produced, but RT-PCR demonstrated major transcripts. The result of computer analysis using NCBI BLAST (http://www.ncbi.nlm.nih.gov/BLAST/) showed that the predicted proteins of *C13orf25* contained no putative domains in those transcripts. Further study is needed however, to characterize the proteins.

Five precursor microRNAs (miRNAs) (miR91-precursor-13 micro RNA, miR18- precursor-13 micro RNA, miR 19a- precursor-13 micro RNA, miR19b- precursor-13 micro RNA and miR92- precursor-13 micro RNA), including seven mature miRNAs (microRNA miR-17, miR-91, miR-18, miR-19a, miR-20, miR-19b and miR-92) were obtained from the transcript-B sequence. The function of microRNA reportedly is to regulate the expression of target genes in both human (Kawasaki, H., and Taira, K. Nature (Lond.), 423: 838-842, 2003) and C. elegans (Lee, R. C. et al. Cell, 75: 843-854, 1993;Reinhart, B. J. et al. Nature (Lond.), 403: 901-906, 2000;Pasquinelli, A. E. et al. Nature (Lond.), 408: 86-89, 2000 ; Slack, F. J. et al. Mol Cell, 5: 659-669, 2000). miRNAs also mediate a cleavage of mRNA in A. thaliana (Llave, C. et al. Science (Wash. DC), 297: 2053-2056, 2002; Tang, G. et al. Genes Dev., 17: 49-63, 2003). Recently, Calin et al. reported an association between chronic lymphocytic leukemia and deletion of a section of chromosome 13 that contains the genes for miR-15 and miR-16 (Proc. Natl. Acad. Sci. USA, 99: 15534-15529, 2002). The presence of these miRNAs on the *C13orf25* gene may provide an insight into the processes of tumorigenesis.

In this Example 2, we were able to demonstrate that *C13orf25* but not *GPC5* is the most likely candidate gene for amplification. *C13orf25* gene was expressed in association with genomic amplification, and may play an important role in tumorigenesis and resulting poor prognosis.

Further investigation into the function of *C13orf25,* including the seven miRNAs, can be expected to provide an insight into the role of the *C13orf25* in tumorigenesis.

**Industrial Applicability**

As explained in detail above, according to the invention of this application, it becomes possible to accurately determine the prognosis of a DLBCL patient. The invention of this application can be used in medical fields and industrial fields such as a field of manufacturing a variety of reagents.

**Claims**

1.  A method for determining the prognosis of a patient with CD5-positive diffuse large B-cell lymphoma (CD5+DLBCL) and a patient with CD5-negative diffuse large B-cell lymphoma (CD5-DLBCL), which comprises isolating a chromosomal DNA from the respective patients with lymphoma, and determining that, in the chromosomal DNA,

    (1) the prognosis of the CD5+DLBCL patient with amplification of chromosome 13 q21.1-q31.3 (13q21.1-q31.3) region is poor;
    (2) the prognosis of the CD5+DLBCL patient with deletion of chromosome 1 p36.21-p36.13 (1p36.21-p36.13) region is poor; and
    (3) the prognosis of the CD5-DLBCL patient with amplification of chromosome 5 p15.33-p14.2 (5p15.33-p14.2) region is good.

2.  The method according to claim 1, wherein the amplification or the deletion of the chromosomal region is measured by hybridization of a plurality of DNA probes containing the chromosomal region with a chromosomal DNA from the patient.

3.  The method according to claim 2, wherein the DNA probes are BAC/PAC DNA clones.

4.  The method according to claim 2 or 3, wherein the hybridization is carried out on a solid phase carrier.

5.  A DNA array used for the method according to claim 4, in which the plurality of DNA probes containing the chromosomal region are immobilized on the solid phase carrier.

6.  The DNA array of claim 5, wherein the DNA probes are BAC/PAC DNA clones.

7.  A method for determining the prognosis of a CD5+DLBCL patient and a CD5-DLBCL patient, which comprises isolating a biological sample from the patient with lymphoma, and determining that, in the biological sample,

(1) the prognosis of the CD5+DLBCL patient with an increased gene expression in 13q21.1-q31.3 region is poor;
(2) the prognosis of the CD5+DLBCL patient with a decreased gene expression in 1p36.21-p36.13 region is poor; and
(3) the prognosis of the CD5-DLBCL patient with an increased gene expression in 5p15.33-p14.2 region is good.

8. The method according to claim 7, wherein the gene in 13q21.1-q31.3 region is C13orf25 gene having the following characteristics of:

(a) encoding a protein containing the amino acid sequences of SEQ ID NO: 4 and SEQ ID NO: 5; and/or
(b) transcribing the following precursor micro RNAs:

miR91-precursor-13 micro RNA, miR18-precursor-1, 3 micro RNA,
miR19a-precursor-13 micro RNA, miR19b-precursor-13 micro RNA
and miR92-precursor-13 micro RNA, and

the following mature micro RNAs:

miR-17, miR-91, miR-18, miR-19a, miR-20, miR-19b and miR-92.

9. The method according to claim 7, wherein the increased gene expression or the decreased gene expression is etermined by measuring a gene transcript.

10. The method according to claim 9, wherein the gene transcript is a mRNA.

11. The method according to claim 10, wherein the increased gene expression or the decreased gene expression is measured by hybridization of a DNA probe which is a full length or a part of the gene with a gene mRNA or cDNA.

12. The method according to claim 11, wherein the hybridization is carried out on a solid phase carrier.

13. A DNA array used for the method according to claim 12, in which the DNA probes are immobilized on the solid phase carrier.

14. The method according to claim 9, wherein the gene transcript is a protein.

15. The method according to claim 14, wherein the increase or decrease of the gene transcript is determined by using an antibody which specifically binds to the protein.

16. An antibody used for the method according to claim 15.

17. The antibody of claim 16, which recognizes the amino acid sequences of SEQ ID NO: 4 and SEQ ID NO: 5.

18. A purified polynucleotide of C13orf25 gene, which has the following characteristics of:

(a) encoding a protein containing the amino acid sequences of SEQ ID NO: 4 and SEQ ID NO: 5; and/or
(b) transcribing the following precursor micro RNAs:

miR91-precursor-13 micro RNA, miR18-precursor-1, 3 micro RNA,
miR19a-precursor-13 micro RNA, miR19b-precursor-13 micro RNA
and miR92-precursor-13 micro RNA, and

the following mature micro RNAs:

miR-17, miR-91, miR-18, miR-19a, miR-20, miR-19b and miR-92.

19. An oligonucleotide probe which comprises a partial continuous sequence of the polynucleotide of claim 18 and is hybridized to C13orf25 gene under a stringent condition.

**20.** A DNA array, which comprises the oligonucleotide probe of claim 19.

**21.** An oligonucleotide primer for PCR amplification of C13orf25 gene.

Fig. 1

EP 1 676 914 A1

## Fig. 2

Fig. 3

Fig. 4

## Fig. 5

A    Chr. 1q

B    Chr. 8

Fig. 6

EP 1 676 914 A1

46

Fig. 7

# Fig. 8

A

Normal male versus normal female

B

# Fig. 9

A

OCI-Ly7      D778

A

Ch 13

Karpas 1718 (SLVL)

B

Ch 13

Rec1 (MCL)

C

Ch 13

OCI-LY4 (DLBCL)

D

Ch 13

Jurkat (T-ALL)

Fig. 10

Fig. 1 1

Fig. 12

Fig. 1 3

Fig. 14

Fig. 15

# Fig. 1 6

A — cen RP11-282D2 ... RP11-121J7 tell; Exon 1 2 3 4; 5' 3' BC040320; 5' 3' AF339828

B — ATG TAA; 5' 3' Transcript-A; 140 bp 73 bp 73 bp 645 bp; ATG TAA; 5' 3' bA121J7.2 (Vega_gene ID); 78 bp 73 bp 648 bp; ATG TAA; 5' 3' Transcript-B; 140 bp 4918 bp

C  Transcript-A ORF (32-AA)  MFCHVDVKISSKRQTCLTTSQTWVFSCSLKTH

Transcript-B ORF (70-AA)  MFCHVDVKISSKRYTWTKLPLNVPKLVLIYLQSHFVL
FFFSMCQSIWERPAIGRATTSSASWMVGYDCLL

**EP 1 676 914 A1**

<table>
<tr><td colspan="2" align="center">**INTERNATIONAL SEARCH REPORT**</td><td colspan="2">International application No.<br>PCT/JP2004/014305</td></tr>
<tr><td colspan="4">A. CLASSIFICATION OF SUBJECT MATTER<br>Int.Cl⁷ C12N15/11, C07K16/18, C12Q1/68</td></tr>
<tr><td colspan="4">According to International Patent Classification (IPC) or to both national classification and IPC</td></tr>
<tr><td colspan="4">B. FIELDS SEARCHED</td></tr>
<tr><td colspan="4">Minimum documentation searched (classification system followed by classification symbols)<br>Int.Cl⁷ C12N15/11, C07K16/18, C12Q1/68</td></tr>
<tr><td colspan="4">Documentation searched other than minimum documentation to the extent that such documents are included in the fields searched</td></tr>
<tr><td colspan="4">Electronic data base consulted during the international search (name of data base and, where practicable, search terms used)<br>JSTPlus, WPI(DIALOG), BIOSIS(DIALOG), PUBMED, OMIM, GENE,<br>EMBL/DDBJ/Genebank/SwissProt/PIR/Geneseq</td></tr>
</table>

C. DOCUMENTS CONSIDERED TO BE RELEVANT

| Category* | Citation of document, with indication, where appropriate, of the relevant passages | Relevant to claim No. |
|---|---|---|
| P,X | OTA A. et al., Identification and Characterization of a Novel Gene, C13orf25, as a Target for 13q31-q32 Amplification in Malignant Lymphoma, Cancer Res. (2004-May), Vol.64, No.9, pages 3087 to 3095 | 17-21 |
| P,A | TAGAWA H. et al., Genome-wide array-based comparative genomic hybridization of diffuse Large B-cell lymphoma: comparison between CD5-positive and CD5-negative cases, Cancer Res. (2004-Sep), Vol.64, No.17, pages 5948 to 5955 | 17-21 |

☒ Further documents are listed in the continuation of Box C.     ☐ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |
| Date of the actual completion of the international search<br>14 December, 2004 (14.12.04) | Date of mailing of the international search report<br>28 December, 2004 (28.12.04) |
| Name and mailing address of the ISA/<br>Japanese Patent Office | Authorized officer |
| Facsimile No. | Telephone No. |

Form PCT/ISA/210 (second sheet) (January 2004)

**INTERNATIONAL SEARCH REPORT**

International application No.

PCT/JP2004/014305

C (Continuation).  DOCUMENTS CONSIDERED TO BE RELEVANT

| Category* | Citation of document, with indication, where appropriate, of the relevant passages | Relevant to claim No. |
|---|---|---|
| A | KOBAYASHI T. et al., Microarray reveals differences in both tumors and vascular specific gene expression in denovo CD5+ and CD5- diffuse large B-cell lymphomas, Cancer Res. (2003-Jan), Vol.63, No.1, pages 60 to 66 | 17-21 |
| A | CHRISTIAN S.L. et al., An evaluation of the assembly of an approximately 15-Mb region on human chromosome 13q32-q33 linked to bipolar disorder and schizophrenia, Genomics. (2002), Vol.79, No.5, pages 635 to 656 | 17-21 |
| A | Norihiko KAWAMATA, "Non-hodgkin lymphoma no Shindan to Chiryo 8 DNA Chip Profiling ni Motozuku Chiryo no Kanosei", Hematology frontier (2002), Vol.12, No.11, pages 1627 to 1638 | 17-21 |
| A | Motoko YAMAGUCHI et al., "CD5 Yosei Bimansei Daisaibogata Lymphoma no Byotai", Annual Review Ketsueki (2003-Jan), Vol.2003, pages 110 to 117 | 17-21 |
| A | Masuhiro SETO et al., "Akusei Lymphoma Kenkyu no Shintenkai Bimansei Daisaibogata B-saibosei Lymphoma o Chushin ni", Saibo (2002), Vol.34, No.9, pages 361 to 364 | 17-21 |
| A | Motoko YAMAGUCHI, CD5+ diffuse large B-cell lymphoma, Hematology & Oncology (2002), Vol.45, No.5, pages 427 to 434 | 17-21 |

Form PCT/ISA/210 (continuation of second sheet) (January 2004)

## INTERNATIONAL SEARCH REPORT

International application No.

PCT/JP2004/014305

**Box No. II      Observations where certain claims were found unsearchable (Continuation of item 2 of first sheet)**

This international search report has not been established in respect of certain claims under Article 17(2)(a) for the following reasons:

1. ☒ Claims Nos.: 1-4, 7-12, 14, 15
   because they relate to subject matter not required to be searched by this Authority, namely:
   The inventions as set forth in claims 1 to 4, 7 to 12, 14 and 15 are relevant to diagnostic methods or methods for treatment of the human body by therapy and thus relate to a subject matter which this International Searching Authority is not required,      (continued to extra sheet.)

2. ☒ Claims Nos.: 5, 6, 13, 16
   because they relate to parts of the international application that do not comply with the prescribed requirements to such an extent that no meaningful international search can be carried out, specifically:
   The inventions according to claims 5, 6, 13 and 16 relate to a DNA array having a DNA probe immobilized on a support or an antibody to be used in a specific method.  However, the subject to which the DNA probe to be used or the antibody binds is not specified.    (continued to extra sheet)

3. ☐ Claims Nos.:
   because they are dependent claims and are not drafted in accordance with the second and third sentences of Rule 6.4(a).

**Box No. III      Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**      ☐  The additional search fees were accompanied by the applicant's protest.

☐  No protest accompanied the payment of additional search fees.

Form PCT/ISA/210 (continuation of first sheet (2)) (January 2004)

**INTERNATIONAL SEARCH REPORT**

International application No.

PCT/JP2004/014305

Continuation of Box No.II-1 of continuation of first sheet(2)

under the provisions of Article 17(2)(a)(i) of the PCT and Rule 39.1(iv) of the Regulations under the PCT, to search.

Continuation of Box No.II-2 of continuation of first sheet(2)

Since the subject employed never specifies the constitution of the DNA array or the antibody, the inventions according to these claims do not comply with the prescribed requirements to such an extent that no meaningful search can be carried out.

Form PCT/ISA/210 (extra sheet) (January 2004)

SEQUENCE LISTING

<110> Aichi Prefecture

<110> NGK Insulators, Ltd.

<120> Disease Type of Lymphoma and Method of Assessing Prognosis

<130> KAB/FP6262612

<140> EP 04773479.3
<141> 2004-09-22

<150> PCT/JP2004/014305
<151> 2004-09-22

<150> US 60/504,208
<151> 2003-09-22

<150> US 60/557,390
<151> 2004-03-30

<160> 5

<170> PatentIn version 3.1

<210> 1
<211> 22
<212> DNA
<213> Artificial Sequence

<220>
<223> Description of artificial sequence: Synthetic oligonucleotide

<220>
<221> misc-feature
<222> (11)..(16)
<223> n is a, t, g or c

<400> 1
ccgactcgag nnnnnnatgt gg                                            22


<210> 2
<211> 24
<212> DNA
<213> Artificial Sequence

<220>
<223>   Description of artificial sequence: Synthetic oligonucleotide

<400>   2
tccggtcgta gtaaagcgca ggcg                                                    24


<210>   3
<211>   20
<212>   DNA
<213>   Artificial Sequence

<220>
<223>   Description of artificial sequence: Synthetic oligonucleotide

<400>   3
ctgaagtctc aagtgggcat                                                         20


<210>   4
<211>   32
<212>   PRT
<213>   Homo sapiens

<220>
<223>   Transcript-A of C13orf24

<400>   4
Met Phe Cys His Val Asp Val Lys Ile Ser Ser Lys Arg Gln Thr Cys
1               5                   10                  15


Leu Thr Thr Ser Gln Thr Trp Val Phe Ser Cys Ser Leu Lys Thr His
            20                  25                  30


<210>   5
<211>
<212>   PRT
<213>   Homo sapiens

<220>
<223>   Transcript-A of C13orf24

<400>   5
Met Phe Cys His Val Asp Val Lys Ile Ser Ser Lys Arg Tyr Thr Trp
1               5                   10                  15

```
Thr Lys Leu Pro Leu Asn Val Pro Lys Leu Val Leu Ile Tyr Leu Gln
            20                  25                  30


Ser His Phe Val Leu Phe Phe Phe Ser Met Cys Gln Ser Ile Trp Glu
        35                  40                  45


Arg Pro Ala Ile Gly Arg Ala Thr Thr Ser Ser Ala Ser Trp Asn Val
        50                  55                  60


Gly Tyr Asp Cys Leu Leu
65                  70
```